# EUROPEAN PATENT SPECIFICATION

(11) **EP 1 501 508 B1**
(45) Date of publication and mention of the grant of the patent: **21.02.2007**
(21) Application number: 03747499.6
(22) Date of filing: 17.04.2003
(51) Int. Cl.: A61K 31/44, A61K 31/495, A61K 31/505, A61P 17/06, A61P 19/02, A61P 19/08, A61P 29/00, A61P 35/00, C07D 239/48, C07D 241/26, C07D 277/56, C07D 401/04

(54) **INHIBITORS OF HISTONE DEACETYLASE**
HISTONDEACETYLASE-HEMMER
INHIBITEURS D'HISTONE DEACETYLASE

(30) Priority: 27.04.2002 GB 0209715
(43) Date of publication of application: 02.02.2005
(73) Proprietor: AstraZeneca AB, 151 85 (SE)
(72) Inventor: STOKES, Elaine Sophie Elizabeth, Macclesfield, Cheshire SK10 4TG (GB); WARING, Michael James, Macclesfield, Cheshire SK10 4TG (GB); GIBSON, Keith Hopkinson, Macclesfield, Cheshire SK10 4TG (GB)
(74) Representative: Bryant, Tracey
(86) International application number: PCT/GB2003/001703
(87) International publication number: WO 2003/092686

(56) References cited:
- WO-A-01/16106
- WO-A-01/38322
- WO-A-03/024448
- MARKS P A ET AL: "HISTONE DEACETYLASE INHIBITORS AS NEW CANCER DRUGS" CURRENT OPINION IN ONCOLOGY, CURRENT SCIENCE LTD, US, vol. 13, no. 6, 2001, pages 477-483, XP009010050 ISSN: 1040-8746

## Description

This invention relates to benzamide derivatives, or pharmaceutically acceptable salts or in *vivo* hydrolysable esters or amides thereof. These benzamide derivatives possess histone deacetylase (HDAC) inhibitory activity and accordingly have value in the treatment of disease states associated with cancer (Marks *et al., Nature Reviews*, 1, 194-202, (2001)), cystic fibrosis (Li, S. *et al, J. Biol. Chem*., 274, 7803-7815, (1999)), Huntingdons chorea (Steffan, J. *S. et. al., Nature*, 413, 739-743, (2001)) and sickle cell anaemia (Gabbianelli, M. *et al., Blood,* 95, 3555-3561, (2000)), and accordingly are useful in methods of treatment of a warm-blooded animal, such as man. The invention also relates to processes for the manufacture of said benzamide derivatives, to pharmaceutical compositions containing them and to their use in the manufacture of medicaments to inhibit HDAC in a warm-blooded animal, such as man.

In the eukaryotic cell, DNA is compacted to prevent transcription factor accessibility. When the cell is activated this compact DNA is made available to DNA-binding proteins, thereby allowing the induction of gene transcription (Beato, M., *J*. *Med. Chem*., 74, 711-724 (1996); Wolffe, A. P., *Nature*, 387, 16-17 (1997)). Nuclear DNA associates with histones to form a complex known as chromatin. The core histones, termed H2A, H2B, H3 and H4 surrounded by 146 base pairs of DNA form the fundamental unit of chromatin, the nucleosome. The N-terminal tails of the core histones contain lysines that are sites for post-transcriptional acetylation. Acetylation neutralizes the potential of the side chain to form a positive charge on the lysine side chain, and is thought to impact chromatin structure.

Histone Deacetylases (HDACs) are zinc-containing enzymes which catalyse the removal of acetyl groups from the ε-amino termini of lysine residues clustered near the amino terminus of nucleosomal histones. HDACs may be divided into two classes, the first (HDAC 1, 2, 3 and 8) represented by yeast Rpd3-like proteins, and the second (HDAC 4, 5, 6, 7, 9 and 10) represented by yeast Hdal-like proteins. The reversible process of acetylation is important in transcriptional regulation and cell-cycle progression. HDAC deregulation has been associated with several cancers and HDAC inhibitors, such as Trichostatin A (a natural product isolated from *Streptomyces hygroscopicus*), have been shown to exhibit significant anti-tumour effects and inhibition of cell-growth (Meinke, P. T., *Current Medicinal Chemistry*, 8, 211-235 (2001)). Yoshida *et al, Exper. Cell Res.,* 177, 122-131 (1988) teaches that Trichostatin A causes arrest of rat fibroblasts at the G1 and G2 phases of the cell cycle, thereby implicating HDAC in cell cycle regulation. Furthermore, Trichostatin A has been shown to induce terminal differentiation, inhibit cell growth, and prevent the formation of tumours in mice (Finnin *et al., Nature*, 401, 188-193 (1999)).

International patent publication number WO 03/024448 discloses a range of HDAC inhibitory compounds, including some benzamide derivatives.

To date only a few inhibitors of HDAC are known in the art. There is thus a need to identify additional HDAC inhibitors.

Accordingly, the present invention provides a compound of the formula (I): 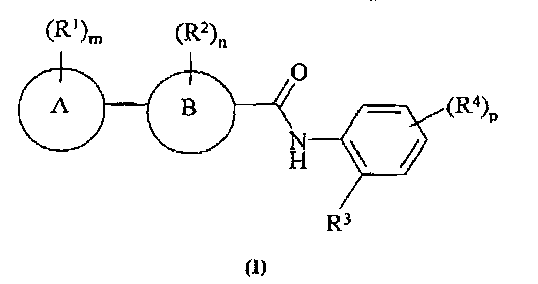 wherein:
Ring A is a pyridyl, quinolyl, indolyl, pyrimidinyl, morpholinyl, piperidinyl, piperazinyl, pyridazinyl, pyrazinyl, thiazolyl, thienyl, thienopyrimidinyl, thienopyridinyl, purinyl, 1',2',3',6'-tetrahydropyridinyl, triazinyl, oxazolyl, pyrazolyl, or furanyl; wherein if Ring A contains an -NH- moiety that nitrogen may be optionally substituted by a group selected from G;
Ring B is thienyl, thiadiazolyl, thiazolyl, pyrimidyl, pyrazinyl, pyridazinyl or pyridyl;
R¹ is a substituent on carbon and is selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl, aryl, aryloxy, arylC₁₋₆alkyl, heterocyclic group, (heterocyclic group)C₁₋₆alkyl or a group (D-E-); wherein R¹, including group (D-E-), may be optionally substituted on carbon by one or more V; and wherein, if said heterocyclic group contains an -NH- moiety that nitrogen may be optionally substituted by a group selected from J; C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl or a group (D'-E'-); wherein V, including group (D'-E'-), may be optionally substituted on carbon by one or more W;
W and Z are independently selected from halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl or N,N-(C₁₋₆alkyl)₂sulphamoyl;
G, J and K are independently selected from C₁₋₈alkyl, C₂₋₈alkenyl, C₂₋₈alkynyl, C₁₋₈alkanoyl, C₁₋₈alkylsulphonyl, C₁₋₈alkoxycarbonyl, carbamoyl, N-(C₁₋₈alkyl)carbamoyl, N,N-(C₁₋₈alkyl)carbamoyl, benzyloxycarbonyl, benzoyl and phenylsulphonyl, aryl, arylC₁₋₆alkyl or (heterocyclic group)C₁₋₆alkyl; wherein G, J and K may be optionally substituted on carbon by one or more Q; and wherein if said heterocyclic group contains an - NH- moiety that nitrogen may be optionally substituted by a group selected from hydrogen or C₁₋₆alkyl;
Q is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, C₁₋₆alkoxycarbonylamino, N-(C₁₋₆alkyl)sulphamoyl, N,N-(C₁₋₆alkyl)₂sulphamoyl, aryl, aryloxy, arylC₁₋₆alkyl, arylC₁₋₆alkoxy, heterocyclic group, (heterocyclic group)C₁₋₆alkyl, (heterocyclic group)C₁₋₆alkoxy, or a group (D"-E"-); wherein Q, including group (D"-E"-), may be optionally substituted on carbon by one or more Z;
D, D' and D" are independently selected from C₁₋₆alkyl, C₂₋₆alkenyl, C₂₋₆alkynyl, C₃₋₈cycloalkyl, C₃₋₈cycloalkylC₁₋₆alkyl, aryl, arylC₁₋₆alkyl, heterocyclic group, (heterocyclic group)C₁₋₆alkyl; wherein D, D' and D" may be optionally substituted on carbon by one or more F'; and wherein if said heterocyclic group contains an -NH- moiety that nitrogen may be optionally substituted by a group selected from K;
E, E' and E'' are independently selected from -N(R^{a})-, -O-, -C(O)O-, -OC(O)-, -C(O)-, -N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})-, -N(R^{a})SO₂-; wherein R^{a} and R^{b} are independently selected from hydrogen or C₁₋₆alkyl optionally substituted by one or more F and r is 0-2;
F and F' are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₙ wherein a is 0 to 2, C₁₋₆alkoxycarbonyl, N-(C₁₋₆alkyl)sulphamoyl and N,N-(C₁₋₆alkyl)₂sulphamoyl;
m is 0, 1, 2, 3 or 4; wherein the values of R¹ may be the same or different;
R² is fluoro or chloro;
n is 0, 1 or 2, wherein the values of R² may be the same or different;
R³ is amino or hydroxy;
R⁴ is halo, nitro, cyano, hydroxy, trifluoromethyl, trifluoromethoxy, amino, carboxy or carbamoyl; and
p is 0, 1 or 2, wherein the values of R⁴ may be the same or different;
and wherein:
an aryl group is a group selected from phenyl, indenyl, indanyl, naphthyl, tetrahydronaphthyl and fluorenyl,
and a heterocyclic group is a saturated, partially saturated or unsaturated, mono or bicyclic ring containing 3-12 atoms of which at least one atom is chosen from nitrogen, sulphur or oxygen, and which may, unless otherwise specified, be carbon or nitrogen linked, and wherein a CH₂ group can optionally be replaced by a C(O), and wherein a ring sulphur atom may be optionally oxidised to form the S-oxide(s);
or a pharmaceutically acceptable salt or in vivo hydrolysable ester or amide thereof

In this specification the term "alkyl" includes both straight and branched chain alkyl groups. For example, "C₁₋₈alkyl"and "C₁₋₆alkyl" includes methyl, ethyl, propyl, isopropyl, pentyl, hexyl, heptyl and *t*-butyl. However, references to individual alkyl groups such as 'propyl' arc specific for the straight-chained version only and references to individual branched chain alkyl groups such as 'isopropyl' are specific for the branched chain version only. The term "halo" refers to fluoro, chloro, bromo and iodo.

Where optional substituents are chosen from "one or more" groups it is to be understood that this definition includes all substituents being chosen from one of the specified groups or the substituents being chosen from two or more of the specified groups.

A "heterocyclyl" is a saturated, partially saturated or unsaturated, mono or bicyclic ring containing 3-12 atoms of which at least one atom is chosen from nitrogen, sulphur or oxygen, which may, unless otherwise specified, be carbon or nitrogen linked, wherein a ring sulphur atom may be optionally oxidised to form the S-oxide(s). Preferably a "heterocyclyl" is a saturated, partially saturated or unsaturated, monocyclic ring containing 5 or 6 atoms of which at least one atom is chosen from nitrogen, sulphur or oxygen which may, unless otherwise specified, be carbon or nitrogen linked, wherein a ring sulphur atom may be optionally oxidised to form S-oxide(s). Examples and suitable values of the term "heterocyclyl" are thiazolidinyl, pyrrolidinyl, 1,3-benzodioxolyl, 1,2,4-oxadiazolyl, 2-azabicyclo[2.2.1]heptyl, morpholinoyl, tetrahydrofuranyl, furanyl, tetrahydropyranyl, piperidinyl, piperazinyl, thiomorpholinyl, 1,3-dioxolanyl, homopiperazinyl, thienyl, pyrrolyl, pyrazolyl, oxadiazolyl, tetrazolyl, oxazolyl, thienopyrimidinyl, thienopylidinyl, thieno[3,2d]pyrimidinyl, 1,3,5-triazinyl, purinyl, 1,2,3,4-tetrahydroquinolinyl, 1',2',3',6'-tetrahydropyridinyl, tetrahydropyridinyl, benzimidazolyl, benzthiazolyl, benzoxazolyl, benzothienyl, benzofuranyl, indazolyl, quinazolinyl, cinnolinyl, phthalazinyl, quinoxalinyl, napthyridinyl, benzotriazolyl, pyrrolothienyl, imidazothienyl, isoxazolyl, imidazolyl, thiadiazolyl, isothiazolyl, 1,2,3-triazolyl, 1,2,4-triazolyl, pyranyl, indolyl, pyrimidinyl, thiazolyl, pyrazinyl, pyridazinyl, pyridyl, quinolyl, quinazolinyl, and 1-isoquinolonyl.

A "heterocyclic group" is a saturated, partially saturated or unsaturated, mono or bicyclic ring containing 3-12 atoms of which at least one atom is chosen from nitrogen, sulphur or oxygen, which may, unless otherwise specified, be carbon or nitrogen linked, wherein a CH₂ group can optionally be replaced by a C(O), and wherein a ring sulphur atom may be optionally oxidised to form the S-oxide(s). Preferably a "heterocyclic group" is a saturated, partially saturated or unsaturated, monocyclic ring containing 5 or 6 atoms of which at least one atom is chosen from nitrogen, sulphur or oxygen or a 9 or 10 membered bicyclic ring which may, unless otherwise specified, be carbon or nitrogen linked, wherein a CH₂ group can optionally be replaced by a C(O), and wherein a ring sulphur atom may be optionally oxidised to form S-oxide(s). Examples and suitable values of the term "heterocyclic group" are pyrrolidinyl, 2-pyrrolidonyl 2,5-dioxopyrrolidinyl, 2,4-dioxoimidazolidinyl, 2-oxo-1,3,4-triazolinyl, oxazolidinyl, 2-oxazolidonyl, 5,6-dihydro-uracilyl, 1,3-benzodioxolyl, 1,2,4-oxadiazolyl, 2-azabicyclo[2.2.1]heptyl, morpholinyl, 2-oxotetrahydrofuranyl, tetrahydrofuranyl, furanyl, tetrahydropyranyl, piperidinyl, piperazinyl, thiomorpholinyl, 1,1-dioxothiomorpholinyl, 1,3-dioxolanyl, homopiperazinyl, thiophenyl, thienopyridinyl, thienopyrimidinyl, thieno[3,2d]pyrimidinyl, 1,3,5-triazinyl, purinyl, quinolinyl, isoquinolinyl, 1,2,3,4-tetrahydroquinolinyl, 1',2',3',6'-tetrahydropyridinyl, tetrahydropyridinyl, tetrahydroisoquinolinyl, imidazolyl, benzimidazolyl, benzothiazolyl, benzoxazolyl, benzothiophenyl, benzofuranyl, indazolyl, quinazolinyl, cinnolinyl, phthalazinyl, quinoxalinyl, napthyridinyl, oxazolyl, isoxazolyl, pyrrolyl, tetrazolyl, thiadiazolyl, isothiazolyl, 1,2,3-triazolyl, 1,2,4-triazolyl, pyranyl, indolyl, phthalamido, isoindolyl, pyrimidinyl, thiazolyl, pyrazolyl, 3-pyrrolinyl, pyrazinyl, pyridazinyl, pyridinyl, pyridonyl, quinolonyl, pyrimidonyl and 1-isoquinolinyl.

An "aryl" group is, for example, phenyl, indenyl, indanyl, naphthyl, tetrahydronaphthyl or fluorenyl, preferably phenyl.

An example of "C₁₋₆alkanoyloxy" is acetoxy. Examples of of "C₁₋₈alkoxycarbonyl", "C₁₋₆alkoxycarbonyl" and C₁₋₄alkoxycarbonyl include methoxycarbonyl, ethoxycarbonyl, n-and *t*-butoxycarbonyl. Examples of C₂₋₆alkynyl are ethynyl and 2-propynyl. Examples of "C₁₋₆alkoxy" include methoxy, ethoxy, propoxy and *t*-butoxy. Examples of "C₁₋₆alkanoylamino" and C₁₋₃alkanoylamino include formamido, acetamido and propionylamino. Examples of "C₁₋₆alkylS(O)ₐ wherein a is 0 to 2" include C₁₋₄alkylsulphonyl, C₁₋₃alkylS(O)ₐ, methylthio, ethylthio, methylsulphinyl, ethylsulphinyl, mesyl and ethylsulphonyl. Examples of "C₁₋₆alkanoyl", "C₁₋₆alkanoyl" and C₁₋₄alkanoyl include C₁₋₃alkanoyl, propionyl and acetyl. Examples of "*N*-(C₁₋₆alkyl)amino" and *N*-(C₁₋₃alkyl)amino include methylamino, ethylamino, propylamino and butylamino. Examples of "*N,N*-(C₁₋₆alkyl)₂amino" and *N,N*-(C₁₋₂alkyl)₂amino include di-*N*-methylamino, di-(*N*-ethyl)amino, di-(*N*-butyl)amino and *N*-ethyl-*N*-methylamino. Examples of "C₂₋₈alkenyl"and "C₂₋₆alkenyl" are C₂₋₃alkenyl and include vinyl, allyl and 1-propenyl. Examples of "*N*-(C₁₋₆alkyl)sulphamoyl" are *N*-(C₁₋₃alkyl)sulphamoyl, *N*-(methyl)sulphamoyl and *N*-(ethyl)sulphamoyl. Examples of "*N*-(C₁₋₈alkyl)₂sulphamoyl" and "*N*-(C₁₋₆alkyl)₂sulphamoyl" are *N,N*-(C₁₋₃alkyl)₂sulphamoyl, *N,N*-(dimethyl)sulphamoyl and *N*-(methyl)-*N*-(ethyl)sulphamoyl. Examples of "*N*-(C₁₋₈alkyl)carbamoyl" and "*N*-(C₁₋₆alkyl)carbamoyl" are *N*-(C₁₋₄alkyl)carbamoyl, *N*-(C₁₋₃alkyl)carbamoyl, methylaminocarbonyl and ethylaminocarbonyl. Examples of "*N,N*-(C₁₋₆alkyl)₂carbamoyl" are *N,N*-(C₁₋₄alkyl)carbamoyl, *N,N*-(C₁₋₂alkyl)₂carbamoyl, dimethylaminocarbonyl and methylethylaminocarbonyl. Examples of "(heterocyclic group)C₁₋₆alkyl" include piperidin-1-ylmethyl, piperidin-1-ylethyl, piperdin-1-ylpropyl, pyridylmethyl, 3-morpholinopropyl, 2-morpholinoethyl and 2-pyrimid-2-ylethyl. Examples of "(heterocyclic group)C₁₋₆alkoxy" include (heterocyclic group)methoxy, (heterocyclic group)ethoxy and (heterocyclic group)propoxy. Examples of "arylC₁₋₆alkyl" include benzyl, 2-phenylethyl, 2-phenylpropyl and 3-phenylpropyl Examples of "aryloxy" include phenoxy and naphthyloxy. Examples of "C₃₋₈cycloalkyl" include cyclopropyl and cyclohexyl. Examples of "C₃₋₈cycloalkylC₁₋₆alkyl" include cyclopropylmethyl and 2-cyclohexylpropyl. Examples of "C₁₋₆alkoxycarbonylamino" include methoxycarbonylamino and *t*-butoxycarbonylamino.

Within this specification composite terms are used to describe groups comprising more that one functionality such as arylC₁₋₆alkyl. Such terms are to be interpretted as is understood by a person skilled in the art. For example arylC₁₋₆alkyl comprises C₁₋₆alkyl substituted by aryl and such a group includes benzyl, 2-phenylethyl, 2-phenylpropyl and 3-phenylpropyl.

A suitable pharmaceutically acceptable salt of a compound of the invention is, for example, an acid-addition salt of a compound of the invention which is sufficiently basic, for example, an acid-addition salt with, for example, an inorganic or organic acid, for example acetic, hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

The compounds of the formula (**I**) may be administered in the form of an *in vivo* hydrolysable ester or *in vivo* hydrolysable amide of a compound of the formula (**I**).

An *in vivo* hydrolysable ester of a compound of the formula (**I**) containing carboxy or hydroxy group is, for example, a pharmaceutically acceptable ester which is hydrolysed in the human or animal body to produce the parent acid or alcohol. Suitable pharmaceutically acceptable esters for carboxy include C₁₋₆alkoxymethyl esters for example methoxymethyl, C₁₋₆alkanoyloxymethyl esters for example pivaloyloxymethyl, phthalidyl esters, C₃₋₈cycloalkoxycarbonyloxyC₁₋₆alkyl esters for example 1-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyloxyethyl and may be formed at any carboxy group in the compounds of this invention.

An *in vivo* hydrolysable ester of a compound of the formula (I) containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and *N*-(*N,N-*dialkylaminoethyl)-N-alkylcarbamoyl (to give carbamates), *N,N*-dialkylaminoacetyl and carboxyacetyl. Examples of substituents on benzoyl include morpholino and piperazino linked from a ring nitrogen atom via a methylene group to the 3- or 4- position of the benzoyl ring.

A suitable value for an *in vivo* hydrolysable amide of a compound of the formula **(I)** containing a carboxy group is, for example, a *N*-C₁₋₆alkyl or *N,N*-di-C₁₋₆alkyl amide such as *N*-methyl, *N*-ethyl, *N*-propyl, *N,N*-dimethyl, *N*-ethyl-*N*-methyl or *N,N*-diethyl amide.

Some compounds of the formula **(I)** may have chiral centres and/or geometric isomeric centres (E- and Z- isomers), and it is to be understood that the invention encompasses all such optical, diastereoisomers and geometric isomers that possess HDAC inhibitory activity.

The invention relates to any and all tautomeric forms of the compounds of the formula (**I**) that possess HDAC inhibitory activity.

Further values of Ring A, Ring B, R¹, R², R³, R⁴, m, n and p are as follows. Such values may be used where appropriate with any of the definitions, claims or embodiments defined hereinbefore or hereinafter.

Ring A is a pyridyl, quinolyl, indolyl, pyrimidinyl, morpholinyl, piperidinyl, piperazinyl, pyridazinyl, pyrazinyl, thiazolyl, thienyl, thienopyrimidinyl, thienopyridinyl, purinyl, 1',2',3',6'-tetrahydropyridinyl, triazinyl, oxazolyl, pyrazolyl, or furanyl; wherein if Ring A contains an -NH- moiety that nitrogen may be optionally substituted by a group selected from G.

Ring A is pyridin-4-yl, pyridin-3-yl, pyridin-2-yl, quinolin-8-yl, pyrimidin-6-yl, pyrimidin-5-yl, pyrimidin-4-yl, morpholin-4-yl, piperidin-4-yl, piperidin-3-yl, piperdin-2-yl, piperazin-4-yl, pyridazin-5-yl, pyrazin-6-yl, thiazol-2-yl, thien-2-yl, thieno[3,2d]pyrimidinyl, thieno[3,2b]pyrimidinyl, thieno[3,2b]pyridinyl, purin-6-yl, 1',2',3',6'-tetrahydropyridin-4-yl or triazin-6-yl; wherein if Ring A contains an -NH- moiety that nitrogen may be optionally substituted by a group selected from G.

Ring A is pyridin-4-yl, pyridin-3-yl, pyridin-2-yl, morpholin-4-yl, piperidin-4-yl, piperidin-3-yl, piperdin-2-yl, piperazin-4-yl, thiazol-2-yl, thien-2-yl, furan-3-yl, pyrrolidin-1-yl, piperidin-1-yl, triazol-1-yl or 1',2',3',6'-tetrahydropyridin-4-yl wherein if Ring A contains an -NH- moiety that nitrogen may be optionally substituted by a group selected from G.

Ring A is a pyridyl, pyrimidyl, morpholinyl, piperidinyl, piperazinyl, pyridazinyl, thienyl, pyrazinyl, thiazolyl, 1,2,4-triazolyl or furanyl.

Ring A is pyridin-4-yl, pyridin-3-yl, pyridin-2-yl or 1,2,4-triazolyl.

Ring B is thienyl, thiadiazolyl, thiazolyl, pyrimidyl, pyrazinyl, pyridazinyl or pyridyl.

Ring B is thienyl, thiazolyl, pyrimidyl, pyrazinyl, pyridazinyl or pyridyl.

Ring B is thienyl or pyridyl.

Ring B is thienyl or pyridyl wherein both the thienyl and the pyridyl are attached to Ring A in the 2-position of the thienyl or pyridyl ring and to the amide group of formula (I) in the 5-position of the thienyl or pyridyl ring.

R¹ is halo, amino, C₁₋₆alkyl, C₁₋₆alkoxy, C₁₋₃alkanoyloxy, *N*-(C₁₋₃alkyl)amino, *N,N*-(C₁₋₃alkyl)₂amino, C₁₋₃alkanoylamino, *N*-(C₁₋₃alkyl)carbamoyl, *N,N*-(C₁₋₃alkyl)₂carbamoyl.

R¹ is halo, amino, C₁₋₆alkyl or C₁₋₆alkoxy.

R¹ is halo, amino, methyl or methoxy.

R¹ is a substituent on carbon and is selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, aryl, aryloxy, arylC₁₋₆alkyl, heterocyclic group, (heterocyclic group)C₁₋₆alkyl or a group (D-E-); wherein R¹, including group (D-E-), may be optionally substituted on carbon by one or more V; and wherein, if said heterocyclic group contains an -NH- moiety that nitrogen may be optionally substituted by a group selected from J;

V is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl or a group (D'-E'-); wherein V, including group (D'-E'-), may be optionally substituted on carbon by one or more W;

W and Z are independently selected from halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl or *N,N*-(C₁₋₆alkyl)₂sulphamoyl;

G, J and K are independently selected from C₁₋₈alkyl, C₂₋₈alkenyl, C₂₋₈alkynyl, C₁₋₈alkanoyl, C₁₋₈alkylsulphonyl, C₁₋₈alkoxycarbonyl, carbamoyl, *N*-(C₁₋₈alkyl)carbamoyl, *N,N*-(C₁₋₈alkyl)carbamoyl, benzyloxycarbonyl, benzoyl and phenylsulphonyl, aryl, arylC₁₋₆alkyl or (heterocyclic group)C₁₋₆alkyl; wherein G, J and K may be optionally substituted on carbon by one or more Q; and wherein if said heterocyclic group contains an - NH- moiety that nitrogen may be optionally substituted by a group selected from hydrogen or C₁₋₆alkyl;

Q is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, C₁₋₆alkoxycarbonylamino, *N*-(C₁₋₆alkyl)sulphamoyl, *N*,*N*-(C₁₋₆alkyl)₂sulphamoyl, aryl, aryloxy, arylC₁₋₆alkyl, arylC₁₋₆alkoxy, heterocyclic group, (heterocyclic group)C₁₋₆alkyl, (heterocyclic group)C₁₋₆alkoxy, or a group (D"-E"-); wherein Q, including group (D"-E"-), may be optionally substituted on carbon by one or more Z;

D, D' and D" are independently selected from C₁₋₆alkyl, C₂₋₆alkenyl, C₂₋₆alkynyl, C₃₋₈cycloalkyl, C₃₋₈cycloalkylC₁₋₆alkyl, aryl, arylC₁₋₆alkyl, heterocyclic group, (heterocyclic group)C₁₋₆alkyl; wherein D, D' and D" may be optionally substituted on carbon by one or more F'; and wherein if said heterocyclic group contains an -NH- moiety that nitrogen may be optionally substituted by a group selected from K;

E, E' and E" are independently selected from -N(R^{a})-, -O-, -C(O)O-, -OC(O)-, -C(O)-, -N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})-, -N(R^{a})SO₂-; wherein R^{a} and R^{b} are independently selected from hydrogen or C₁₋₆alkyl optionally substituted by one or more F and r is 0-2; and

F and F' are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl and *N,N*-(C₁₋₆alkyl)₂sulphamoyl.

R¹ is a substituent on carbon and is selected from cyano, hydroxy, C₁₋₆alkyl or a group (D-E-); wherein R¹, including group (D-E-), may be optionally substituted on carbon by one or more V;

V is cyano, hydroxy or a group (D'-E'-); wherein V, including group (D'-E'-), may be optionally substituted on carbon by one or more W;

W and Z are independently selected from cyano, C₁₋₆alkyl or C₁₋₆alkoxy;

G and K are independently selected from C₁₋₈alkyl, C₂₋₈alkenyl, C₂₋₈alkynyl, arylC₁₋₆alkyl or (heterocyclic group)C₁₋₆alkyl; wherein G and K may be optionally substituted on carbon by one or more Q;

Q is cyano, hydroxy, oxo, C₁₋₆alkyl, C₂₋₆alkenyl, C₁₋₆alkoxy, C₁₋₆alkanoyl, C₁₋₆alkanoyloxy, *N*-(C₁₋₆alkyl)carbamoyl, *N,N*-(C₁₋₆alkyl)₂carbamoyl, C₁₋₆alkoxycarbonyl, C₁₋₆alkoxycarbonylamino, aryl, aryloxy or a group (D"-E"-); wherein Q, including group (D"-E"-), may be optionally substituted on carbon by one or more Z;

D, D' and D" are independently selected from aryl, arylC₁₋₆alkyl or heterocyclic group; wherein D, D' and D" may be optionally substituted on carbon by one or more F'; and wherein if said heterocyclic group contains an -NH- moiety that nitrogen may be optionally substituted by a group selected from K;

E, E' and E" are independently selected from -O-, -C(O)O-, -OC(O)-, -C(O)-, -N(R^{a})C(O)-, -C(O)N(R^{a})-, -S(O)ᵣ-; wherein R^{a} is selected from hydrogen or C₁₋₆alkyl optionally substituted by one or more F and r is 0-2; and

F and F' are independently selected from nitro, hydroxy, C₁₋₆alkyl, C₁₋₆alkoxy, C₁₋₆alkanoyl, *N*-(C₁₋₆alkyl)amino, *N,N*-(C₁₋₆alkyl)₂amino, C₁₋₆alkanoylamino or C₁₋₆alkoxycarbonyl.

m is 0, 1, 2, 3 or 4; wherein the values of R¹ may be the same or different.

m is 0, 1, or 2; wherein the values of R¹ may be the same or different.

m is 0 or 1.

m is 0.

m is 1.

R² is halo.

R² is fluoro or chloro.

R² is fluoro.

n is 0, 1 or 2, wherein the values of R² may be the same or different.

n is 0 or 1.

n is 0.

n is 1.

R³ is amino or hydroxy.

R³ is amino.

R³ is hydroxy.

R⁴ is halo, nitro, cyano, hydroxy, trifluoromethyl, trifluoromethoxy, amino, carboxy or carbamoyl.

R⁴ is halo, cyano, trifluoromethyl or trifluoromethoxy.

R⁴ is halo.

p is 0, 1 or 2, wherein the values of R⁴ may be the same or different.

p is 0 or 1.

p is 0.

pis 1.

Therefore in an additional aspect of the invention there is provided a compound of formula (I) (as depicted above) wherein:
Ring A is a pyridyl, indolyl, pyrimidyl, morpholinyl, piperidinyl, piperazinyl, pyridazinyl, thienyl, pyrazinyl, thiazolyl, oxazolyl, 1,2,4-triazolyl, isoxazolyl, isothiazolyl, pyrazolyl, or furanyl;
Ring B is thienyl, thiadiazolyl, thiazolyl, pyrimidyl, pyrazinyl, pyridazinyl or pyridyl;
R¹ is halo, amino, C₁₋₆alkyl, C₁₋₆alkoxy, C₁₋₃alkanoyloxy, *N*-(C₁₋₃alkyl)amino, *N*,*N*-(C₁₋₃alkyl)₂amino, C₁₋₃alkanoylamino, *N*-(C₁₋₃alkyl)carbamoyl, *N*,*N*-(C₁₋₃alkyl)₂carbamoyl;
m is 0, 1, 2, 3 or 4; wherein the values of R¹ may be the same or different;
R² is fluoro or chloro;
n is 0, 1 or 2, wherein the values of R² may be the same or different;
R³ is amino or hydroxy;
R⁴ is halo, nitro, cyano, hydroxy, trifluoromethyl, trifluoromethoxy, amino, carboxy or carbamoyl; and
p is 0, 1 or 2, wherein the values of R⁴ may be the same or different;
or a pharmaceutically acceptable salt or *in vivo* hydrolysable ester or amide thereof.

Therefore in an additional aspect of the invention there is provided a compound of formula (I) (as depicted above) wherein:
Ring A is pyridin-4-yl, pyridin-3-yl, pyridin-2-yl or 1,2,4-triazolyl;
Ring B is thienyl or pyridyl;
R¹ is halo, amino, methyl or methoxy;
m is 0, 1, or 2; wherein the values of R¹ may be the same or different;
R² is fluoro;
n is 0 or 1;
R³ is amino;
R⁴is halo; and
p is 0, 1 or 2, wherein the values of R⁴ may be the same or different;
or a pharmaceutically acceptable salt or *in vivo* hydrolysable ester or amide thereof.

Therefore in an additional aspect of the invention there is provided a compound of formula **(I)** (as depicted above) wherein:
Ring A is pyridin-4-yl, pyridin-3-yl, pyridin-2-yl, morpholin-4-yl, piperidin-4-yl, piperidin-3-yl, piperdin-2-yl, piperazin-4-yl, thiazol-2-yl, thien-2-yl, furan-3-yl, pyrrolidin-1-yl, piperidin-1-yl, triazol-1-yl or 1',2',3',6'-tetrahydropyridin-4-yl wherein if Ring A contains an -NH- moiety that nitrogen may be optionally substituted by a group selected from G;
Ring B is thienyl, thiazolyl, pyrimidyl, pyrazinyl, pyridazinyl or pyridyl;
R¹ is a substituent on carbon and is selected from cyano, hydroxy, C₁₋₆alkyl or a group (D-E-); wherein R¹, including group (D-E-), may be optionally substituted on carbon by one or more V;
V is cyano, hydroxy or a group (D'-E'-); wherein V, including group (D'-E'-), may be optionally substituted on carbon by one or more W;
W and Z are independently selected from cyano, C₁₋₆alkyl or C₁₋₆alkoxy;
G and K are independently selected from C₁₋₈alkyl, C₂₋₈alkenyl, C₂₋₈alkynyl, arylC₁₋₆alkyl or (heterocyclic group)C₁₋₆alkyl; wherein G and K may be optionally substituted on carbon by one or more Q;
Q is cyano, hydroxy, oxo, C₁₋₆alkyl, C₂₋₆alkenyl, C₁₋₆alkoxy, C₁₋₆alkanoyl, C₁₋₆alkanoyloxy, *N*-(C₁₋₆alkyl)carbamoyl, *N,N*-(C₁₋₆alkyl)₂carbamoyl, C₁₋₆alkoxycarbonyl, C₁₋₆alkoxycarbonylamino, aryl, aryloxy or a group (D"-E"-); wherein Q, including group (D"-E"-), may be optionally substituted on carbon by one or more Z;
D, D' and D" are independently selected from aryl, arylC₁₋₆alkyl or heterocyclic group; wherein D, D' and D" may be optionally substituted on carbon by one or more F'; and wherein if said heterocyclic group contains an -NH- moiety that nitrogen may be optionally substituted by a group selected from K;
E, E' and E" are independently selected from -O-, -C(O)O-, -OC(O)-, -C(O)-, -N(R^{a})C(O)-, -C(O)N(R^{a})-, -S(O)ᵣ-; wherein R^{a} is selected from hydrogen or C₁₋₆alkyl optionally substituted by one or more F and r is 0-2;
F and F' are independently selected from nitro, hydroxy, C₁₋₆alkyl, C₁₋₆alkoxy, C₁₋₆alkanoyl, *N*-(C₁₋₆alkyl)amino, *N,N*-(C₁₋₆alkyl)₂amino, C₁₋₆alkanoylamino or C₁₋₆alkoxycarbonyl;
m is 0, 1, or 2; wherein the values of R¹ may be the same or different;
R² is fluoro;
n is 0 or 1;
R³ is amino;
R⁴ is halo; and
p is 0, 1 or 2, wherein the values of R⁴ may be the same or different;
or a pharmaceutically acceptable salt or *in vivo* hydrolysable ester or amide thereof.

In another aspect of the invention, preferred compounds of the invention are any one of the Examples or a pharmaceutically acceptable salt or an *in vivo* hydrolysable ester or amide thereof.

In another aspect of the invention there is provided compound of formula (**I**) or a pharmaceutically acceptable salt thereof (wherein Ring A, Ring B, R¹, R², R³, R⁴, m, n and p are, unless otherwise specified, as defined in formula (**I**)).

In a further aspect of the invention, preferred compounds of the invention are any one of the Examples or a pharmaceutically acceptable salt thereof.

Another aspect of the present invention provides a process for preparing a compound of formula (**I**) or a pharmaceutically acceptable salt or an *in vivo* hydrolysable ester thereof which process (wherein Ring A, Ring B, R¹, R², R³, R⁴, m, n and p are, unless otherwise specified, as defined in formula (**I**)) comprises of:
(a) The reaction of a compound of the formula (**II**) 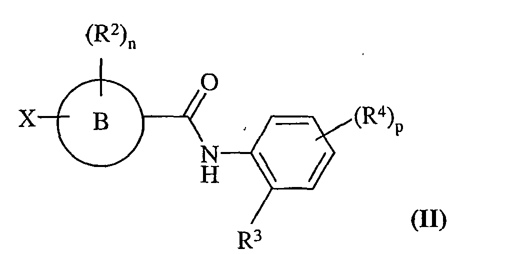 wherein X is a reactive group, with a compound of the formula **(III)** 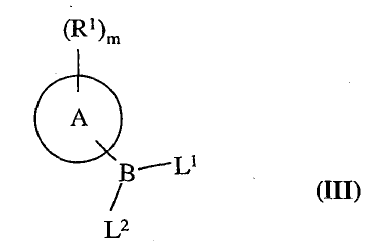 wherein L¹ and L² are ligands;
(b) The reaction of a compound of the formula **(IV)** 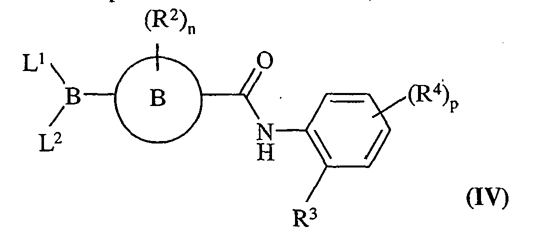 wherein L¹ and L² are ligands, with a compound of the formula (**V**) 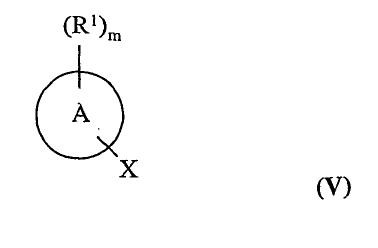 wherein X is a reactive group; or
(c) The reaction, in the presence of 4-(4,6-dimethoxy-1,3,5-triazinyl-2-yl)-4-methylmorpholinium chloride, of a compound of the formula **(VI)** 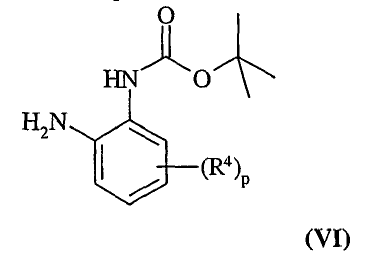 with a compound of the formula (VII) 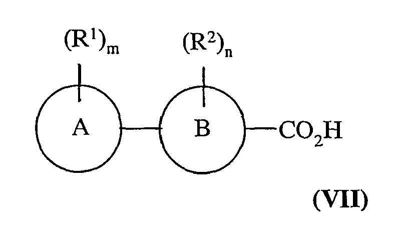
and thereafter if necessary:
i) converting a compound of the formula **(I)** into another compound of the formula **(I);** and/or
ii) removing any protecting groups;

Processes (a), (b) or (c) may be carried out in the presence of a base. A suitable base for process (a), (b) or (c) is, for example, an organic amine base such as, for example, pyridine, 2,6-lutidine, collidine, 4-dimethylaminopyridine, triethylamine, morpholine, N-methylmorpholine or diazabicyclo[5.4.0]undec-7-ene, or, for example, an alkali or alkaline earth metal carbonate or hydroxide, for example sodium carbonate, potassium carbonate, calcium carbonate, sodium hydroxide or potassium hydroxide, or, for example, an alkali metal hydride, for example sodium hydride, or a metal alkoxide such as sodium ethoxide;

A suitable reactive group X is, for example, a halogeno, alkoxy, aryloxy or sulphonyloxy group, for example a chloro, bromo, methoxy, phenoxy, methanesulphonyloxy, trifluromethanesulphonyloxy or toluene-4-sulphonyloxy group. The reactions are conveniently carried out in the presence of a suitable inert solvent or diluent, for example an alkanol or ester such as methanol, ethanol, isopropanol or ethyl acetate, a halogenated solvent such as methylene chloride, chloroform or carbon tetrachloride, an ether such as tetrahydrofuran, 1,2-dimethoxyethane or 1,4-dioxan, an aromatic solvent such as toluene, or a dipolar aprotic solvent such as N,N-dimethylformamide, N,N-dimethylacetamide, N-methylpyrrolidin-2-one or dimethylsulphoxide. The reactions are conveniently carried out at a temperature in the range, for example, 10 to 250°C, preferably in the range 40 to 80°C;

A suitable value for the ligands L¹ and L² which are present on the boron atom include, for example, a hydroxy, C₁₋₄alkoxy or C₁₋₆alkyl ligand, for example a hydroxy, methoxy, ethoxy, propoxy, isopropoxy, butoxy, methyl, ethyl, propyl, isopropyl or butyl ligand. Alternatively the ligands L¹ and L² may be linked such that, together with the boron atom to which they are attached, they form a ring. For example, L¹ and L² together may define an oxy-(C₂₋₄)alkylene-oxy group, for example an oxyethyleneoxy or oxytrimethyleneoxy group such that, together with the boron atom to which they are attached, they form a cyclic boronic acid ester group;

Processes (a) or (b) may be carried out in the presence of a catalyst. A suitable catalyst for process (a) or (b) includes, for example, a metallic catalyst such as a palladium(0), palladium(II), nickel(0) or nickel(II) catalyst, for example tetrakis(triphenylphosphine)palladium(0), palladium(II) chloride, palladium(II) bromide, bis(triphenylphosphine)palladium(II) chloride, tetrakis(triphenylphosphine)nickel(0), nickel(II) chloride, nickel(II) bromide or bis(triphenylphosphine)nickel(II) chloride. In addition a free radical initiator may conveniently be added, for example an azo compound such as azo(bisisobutyronitrile);

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

### Biological Assays

The following assays can be used to measure the effects of the compounds of the present invention as HDAC inhibitors, as inhibitors *in vitro* of pooled histone deacetylases from nuclear extracts prepared from the human cervical cancer cell line HeLa, as inhibitors *in vitro* of recombinant human HDAC1 produced in Hi5 insect cells, and as inducers *in vitro* of Histone H3 acetylation in whole cells

### (a) In Vitro Enzyme Assay of Pooled Histone Deacetylases

HDAC inhibitors were screened against pooled histone deacetylases from nuclear extracts prepared from the human cervical cancer cell line HeLa.

The deacetylase assays were carried out in a 40 µl reaction. 2.5 µg of nuclear extract diluted in 15 µl of reaction buffer (25 mM TrisHCl (pH 8), 137 mM NaCl, 2.7 mM KCl,1 mM MgCl₂) was mixed with either buffer alone (5 µl) or buffer containing compound (5 µl) for 30 minutes at ambient temperature. 25 µM fluor-de-lys substrate (Biomol) diluted in 20 µl of buffer was then added to the reaction and incubated for one hour at ambient temperature. The reaction was stopped by addition of an equal volume (40 µl) fluor de lys developer (Biomol) containing Trichostatin A at 2 µM. The reaction was allowed to develop for 30 minutes at ambient temperature and then fluorescence measured at an excitation wavelength of 360 nM and an emission wavelength of 465 nM. IC₅₀ values for HDAC enzyme inhibitors were determined by performing dose response curves with individual compounds and determining the concentration of inhibitor producing fifty percent decrease in the maximal signal (no inhibitor control).

### (b) In Vitro Enzyme Assay of recombinant HDAC1

HDAC inhibitors were screened against recombinant human HDAC1 produced in Hi5 insect cells. The enzyme was cloned with a FLAG tag at the C-terminal of the gene and affinity purified using Anti-FLAG M2 agarose from SIGMA (A2220).

The deacetylase assays were carried out in a 50 µl reaction. 75ng of enzyme diluted in 15 µl of reaction buffer (25 mM TrisHCl (pH 8), 137 mM NaCl, 2.7 mM KCl,1 mM MgCl₂) was mixed with either buffer alone (5 µl) or buffer containing compound (10 µl) for 30 minutes at ambient temperature. 50 µM fluor-de-lys substrate (Biomol) diluted in 25 µl of buffer was then added to the reaction and incubated for one hour at ambient temperature. The reaction was stopped by addition of an equal volume (50 µl) fluor de lys developer (Biomol) containing Trichostatin A at 2 µM. The reaction was allowed to develop for 30 minutes at ambient temperature and then fluorescence measured at an excitation wavelength of 360 nM and an emission wavelength of 465 nM. IC₅₀ values for HDAC enzyme inhibitors were determined by performing dose response curves with individual compounds and determining the concentration of inhibitor producing fifty percent decrease in the maximal signal (no inhibitor control).

### (c) In Vitro Enzyme Assay of Histone Deacetylase activity in whole cells

Histone H3 acetylation in whole cells using immunohistochemistry and analysis using the Cellomics arrayscan. A549 cells were seeded in 96 well plates at 1x10⁴ cells/well, and allowed to adhere overnight. They were treated with inhibitors for 24 hours and then fixed in 1.8% formaldehyde in tris buffered saline (TBS) for one hour. Cells were permeabilized with ice-cold methanol for 5 minutes, rinsed in TBS and then blocked in TBS 3% low-fat dried milk for 90 minutes. Cells were then incubated with polyclonal antibodies specific for the acetylated histone H3 (Upstate #06-599) diluted 1 in 500 in TBS 3% milk for one hour. Cells were rinsed three times in TBS and then incubated with fluorescein conjugated secondary antibodies (Molecular Probes #A11008) & Hoechst 333542 (1 µg/ml) (Molecular Probes #H3570) in TBS 1% Bovine serum albumin (Sigma #B6917) for one hour. Unbound antibody was removed by three rinses with TBS and after the final rinse 100 µl of TBS was added to the cells and the plates sealed and analysed using the Cellomics arrayscan.

EC₅₀ values for HDAC inhibitors were determined by performing dose response curves with individual compounds and then determining the concentration of inhibitor producing fifty percent of the maximal signal (reference compound control - Trichostatin A (Sigma)).

Although the pharmacological properties of the compounds of the formula **(I)** vary with structural change as expected, in general activity possessed by compounds of the formula **(I),** may be demonstrated at the following concentrations or doses in one or more of the above tests (a), (b and (c):-

| | |
|---|---|
| Test (a):- | IC₅₀ in the range, for example, < 50.0 µM; |
| Test (b):- | IC₅₀ in the range, for example, < 2.5 µM; |
| Test (c):- | EC₅₀ in the range, for example, < 9.0 µM; |

According to a further aspect of the invention there is provided a pharmaceutical composition which comprises a compound of the formula **(I),** or a pharmaceutically acceptable salt or *in vivo* hydrolysable ester or amide thereof, as defined hereinbefore in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula **(I)** will normally be administered to a warm-blooded animal at a unit dose within the range 5-5000 mg per square meter body area of the animal, i.e. approximately 0.1-100 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

According to a further aspect of the present invention there is provided a compound of the formula **(I),** or a pharmaceutically acceptable salt or *in vivo* hydrolysable ester or amide thereof, as defined hereinbefore for use in a method of treatment of the human or animal body by therapy.

We have found that the compounds defined in the present invention, or a pharmaceutically acceptable salt or *in vivo* hydrolysable ester or amide thereof, are effective cell cycle inhibitors (anti-cell proliferation agents), which property is believed to arise from their HDAC inhibitory properties. We also believe that the compounds of the present invention may be involved in the inhibition of angiogenesis, activation of apoptosis and differentiation. Accordingly the compounds of the present invention are expected to be useful in the treatment of diseases or medical conditions mediated alone or in part by HDAC enzymes, i.e. the compounds may be used to produce a HDAC inhibitory effect in a warm-blooded animal in need of such treatment. Thus the compounds of the present invention provide a method for treating the proliferation of malignant cells characterised by inhibition of HDAC enzymes, i.e. the compounds may be used to produce an anti-proliferative effect mediated alone or in part by the inhibition of HDACs.

According to one aspect of the present invention there is provided a compound of the formula **(I),** or a pharmaceutically acceptable salt or *in vivo* hydrolysable ester or amide thereof, as defined hereinbefore for use in a method of treatment of the human or animal body by therapy.

Thus according to a further aspect of the invention there is provided a compound of the formula (**I**), or a pharmaceutically acceptable salt or *in vivo* hydrolysable ester or amide thereof, as defined hereinbefore for use as a medicament.

According to a further aspect of the invention there is provided the use of a compound of the formula **(I),** or a pharmaceutically acceptable salt or *in vivo* hydrolysable ester or amide thereof, as defined hereinbefore in the manufacture of a medicament for use in the production of a HDAC inhibitory effect in a warm-blooded animal such as man.

According to a further feature of this aspect of the invention there is provided a method for producing a HDAC inhibitory effect in a warm-blooded animal, such as man, in need of such treatment which comprises administering to said animal an effective amount of a compound of the formula **(I),** or a pharmaceutically acceptable salt or *in vivo* hydrolysable ester or amide thereof, as defined hereinbefore.

According to a further aspect of the invention there is provided the use of a compound of the formula **(I),** or a pharmaceutically acceptable salt or *in vivo* hydrolysable ester or amide thereof, as defined hereinbefore in the manufacture of a medicament for use in the production of a cell cycle inhibitory (anti-cell-proliferation) effect in a warm-blooded animal such as man.

According to a further feature of this aspect of the invention there is provided a method for producing a cell cycle inhibitory (anti-cell-proliferation) effect in a warm-blooded animal, such as man, in need of such treatment which comprises administering to said animal an effective amount of a compound of the formula **(I),** or a pharmaceutically acceptable salt or *in vivo* hydrolysable ester or amide thereof, as defined hereinbefore.

According to an additional feature of this aspect of the invention there is provided a method of treating cancer in a warm-blooded animal, such as man, in need of such treatment which comprises administering to said animal an effective amount of a compound of the formula **(I),** or a pharmaceutically acceptable salt or *in vivo* hydrolysable ester or amide thereof, as defined hereinbefore.

According to a further feature of the invention there is a compound of the formula **(I),** or a pharmaceutically acceptable salt or *in* vivo hydrolysable ester or amide thereof, as defined hereinbefore in the manufacture of a medicament for use in the treatment of cancer.

According to an additional feature of this aspect of the invention there is provided a compound of the formula **(I),** or a pharmaceutically acceptable salt or *in vivo* hydrolysable ester or amide thereof, as defined hereinbefore, for use in the treatment of cancer.

In a further aspect of the present invention there is provided the use of a compound of the formula **(I),** or a pharmaceutically acceptable salt or *in vivo* hydrolysable ester or amide thereof, as defined hereinbefore, in the manufacture of a medicament for use in lung cancer, colorectal cancer, breast cancer, prostate cancer, lymphoma and leukaemia.

In a further aspect of the present invention the is provided a method of treating lung cancer, colorectal cancer, breast cancer, prostate cancer, lymphoma or leukaemia, in a warm-blooded animal, such as man, in need of such treatment which comprises administering to said animal an effective amount of a compound of the formula **(I),** or a pharmaceutically acceptable salt or *in vivo* hydrolysable ester or amide thereof, as defined hereinbefore.

Cancers that are amenable to treatment with the present invention include oesophageal cancer, myeloma, hepatocellular, pancreatic and cervical cancer, Ewings tumour, neuroblastoma, kaposis sarcoma, ovarian cancer, breast cancer, colorectal cancer, prostate cancer, bladder cancer, melanoma, lung cancer [including non small cell lung cancer (NSCLC) and small cell lung cancer (SCLC)], gastric cancer, head and neck cancer, brain cancer, renal cancer, lymphoma and leukaemia.

It is further expected that a compound of the present invention will possess activity against other cell-proliferation diseases in a wide range of other disease states including leukaemias, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

There is further provided is a compound of the formula **(I),** or a pharmaceutically acceptable salt or *in vivo* hydrolysable ester or amide thereof, as defined hereinbefore, for use in a method of treating inflammatory diseases, autoimmune diseases and allergic/atopic diseases.

In particular a compound of the formula **(I),** or a pharmaceutically acceptable salt or *in vivo* hydrolysable ester or amide thereof, as defined hereinbefore, is provided for use in a method of treating inflammation of the joint (especially rheumatoid arthritis, osteoarthritis and gout), inflammation of the gastro-intestinal tract (especially inflammatory bowel disease, ulcerative colitis and gastritis), inflammation of the skin (especially psoriasis, eczema, dermatitis), multiple sclerosis, atherosclerosis, spondyloarthropathies (ankylosing spondylitis, psoriatic arthritis, arthritis connected to ulcerative colitis), AIDS-related neuropathies, systemic lupus erythematosus, asthma, chronic obstructive lung diseases, bronchitis, pleuritis, adult respiratory distress syndrome, sepsis, and acute and chronic hepatitis (either viral, bacterial or toxic).

Further provided is a compound of the formula **(I),** or a pharmaceutically acceptable salt or *in vivo* hydrolysable ester or amide thereof, as defined hereinbefore, for use as a medicament in the treatment of inflammatory diseases, autoimmune diseases and allergic/atopic diseases in a warm-blooded animal such as man.

In particular a compound of the formula **(I),** or a pharmaceutically acceptable salt or *in vivo* hydrolysable ester or amide thereof, as defined hereinbefore, is provided for use as a medicament in the treatment of inflammation of the joint (especially rheumatoid arthritis, osteoarthritis and gout), inflammation of the gastro-intestinal tract (especially inflammatory bowel disease, ulcerative colitis and gastritis), inflammation of the skin (especially psoriasis, eczema, dermatitis), multiple sclerosis, atherosclerosis, spondyloarthropathies (ankylosing spondylitis, psoriatic arthritis, arthritis connected to ulcerative colitis), AIDS-related neuropathies, systemic lupus erythematosus, asthma, chronic obstructive lung diseases, bronchitis, pleuritis, adult respiratory distress syndrome, sepsis, and acute and chronic hepatitis (either viral, bacterial or toxic).

Further provided is the use of a compound of the formula **(I),** or a pharmaceutically acceptable salt or *in vivo* hydrolysable ester or amide thereof, as defined hereinbefore, in the manufacture of a medicament for use in the treatment of inflammatory diseases, autoimmune diseases and allergic/atopic diseases in a warm-blooded animal such as man.

As stated above the size of the dose required for the therapeutic or prophylactic treatment of a particular cell-proliferation disease will necessarily be varied depending on the host treated, the route of administration and the severity of the illness being treated. A unit dose in the range, for example, 1-100 mg/kg, preferably 1-50 mg/kg is envisaged.

The HDAC inhibitory activity defined hereinbefore may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may include one or more of the following categories of anti-tumour agents:
(i) other cell cycle inhibitory agents that work by the same or different mechanisms from those defined hereinbefore, for example cyclin dependent kinase (CDK) inhibitors, in particular CDK2 inhibitors;
(ii) cytostatic agents such as antioestrogens (for example tamoxifen, toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone 5α-dihydroreductase (for example finasteride), anti-invasion agents (for example metalloproteinase inhibitors like marimastat and inhibitors of urokinase plasminogen activator receptor function) and inhibitors of growth factor function, (such growth factors include for example vascular endothelial growth factor, epithelial growth factor, platelet derived growth factor and hepatocyte growth factor such inhibitors include growth factor antibodies, growth factor receptor antibodies, tyrosine kinase inhibitors and serine/threonine kinase inhibitors);
(iii) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincrisitine and taxoids like taxol, taxotere); topoisomerase inhibitors (for example epipodophyllotoxins like etoposide and teniposide, amsacrine, topotecan);
(iv) antiangiogenic agents that work by different mechanisms from those defined hereinbefore (for example receptor tyrosine kinases like Tie-2, inhibitors of integrin αvβ3 function, angiostatin, razoxin, thalidomide), and including vascular targeting agents; and
(v) differentiation agents (for example retinoic acid and vitamin D).

According to this aspect of the invention there is provided a pharmaceutical product comprising a compound of the formula **(I)** as defined hereinbefore and an additional anti-tumour substance as defined hereinbefore for the conjoint treatment of cancer.

In addition to their use in therapeutic medicine, the compounds of formula (I) and their pharmaceutically acceptable salts or *in vivo* hydrolysable esters or amides thereof, are also useful as pharmacological tools in the development and standardisation of in vitro and *in vivo* test systems for the evaluation of the effects of inhibitors of cell cycle activity in laboratory animals such as cats, dogs, rabbits, monkeys, rats and mice, as part of the search for new therapeutic agents.

The invention will now be illustrated in the following Examples in which, generally:
(i) operations were carried out at ambient temperature, *i.e*. in the range 17 to 25°C and under an atmosphere of an inert gas such as argon unless otherwise stated;
(ii) evaporations were carried out by rotary evaporation *in vacuo* and work-up procedures were carried out after removal of residual solids by filtration;
(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Merck, Darmstadt, Germany or high pressure liquid chromatography (HPLC) was performed on C18 reverse phase silica, for example on a Dynamax C-18 60Å preparative reversed-phase column;
(iv) yields, where present, are not necessarily the maximum attainable;
(v) in general, the structures of the end-products of the Formula (I) were confirmed by nuclear magnetic resonance (NMR) and/or mass spectral techniques; fast-atom bombardment (FAB) mass spectral data were obtained using a Platform spectrometer and, where appropriate, either positive ion data or negative ion data were collected; NMR chemical shift values were measured on the delta scale [proton magnetic resonance spectra were determined using a Jeol JNM EX 400 spectrometer operating at a field strength of 400 MHz, Varian Gemini 2000 spectrometer operating at a field strength of 300MHz or a Bruker AM300 spectrometer operating at a field strength of 300MHz]; the following abbreviations have been used: s, singlet; d, doublet; t, triplet; q, quartet; m, multiplet; br, broad;
(vi) intermediates were not generally fully characterised and purity was assessed by thin layer chromatographic, HPLC, infra-red (IR) and/or NMR analysis;
(vii) melting points are uncorrected and were determined using a Mettler SP62 automatic melting point apparatus or an oil-bath apparatus; melting points for the end-products of the formula (I) were determined after crystallisation from a conventional organic solvent such as ethanol, methanol, acetone, ether or hexane, alone or in admixture;
(viii) the following abbreviations have been used:-
   - DMF: *N,N*-dimethylformamide
   - DMSO: dimethylsulphoxide
   - THF: tetrahydrofuran

### Example 1

### N2-(2-Aminophenyl)-5-(pyridin-3-yl)thiophene-2-carboxamide

N2-(2-t-Butoxycarbonylaminophenyl)-5-(pyridin-3-yl)thiophene-2-carboxamide (Method 1; 70 mg, 0.177 mmol), 1,4-dioxane (0.67 ml) and a 4M solution of hydrochloric acid in dioxane (0.67 ml) were stirred at ambient temperature for 18 hours. The resultant precipitate was collected by filtration and washed with diethyl ether and dried *in vacuo* to give the title compound dihydrochloride (40 mg, 62%); NMR Spectrum; (DMSO-d₆) 7.36 (m, 2H); 7.49 (d, 1H), 7.63 (d, 1H), 7.84 (m, 1H), 7.91 (d, 1H), 8.42 (d, 1H), 8.56 (d, 1H), 8.73 (d, 1H), 9.20 (s, 1H), 10.85 (s, 1H); Mass Spectrum: M+H⁺ 296.

### Example 2

### N2-(2-Aminophenyl)-5-(pyridin-4-yl)thiophene-2-carboxamide

N2-(2-t-Butoxycarbonylaminophenyl)-5-(pyridin-4-yl)thiophene-2-carboxamide (Method 2; 118 mg, 0.298 mmol), 1,4-dioxane (1.1 ml) and a 4M solution of hydrochloric acid in dioxane (1.1 ml) were stirred at ambient temperature for 18 hours. The resultant precipitate was collected by filtration and washed with diethyl ether and dried *in vacuo* to give the title compound dihydrochloride (82mg, 75%); NMR Spectrum: (DMSO-d₆) 7.32 (m, 2H), 7.45 (m, 1H), 7.60 (m, 1H), 8.25 (d, 1H), 8.30 (d, 2H), 8.50 (d, 1H), 8.87 (d, 2H), 10.92 (s, 1H); Mass Spectrum: M+H⁺ 296.

### Example 3

### N3-(2-Aminophenyl)-6-(pyridin-4-yl)nicotinamide

N3-(2-t-Butoxycarbonylaminophenyl)-6-(pyridin-4-yl)nicotinamide (Method 4; 65 mg, 0.17 mmol), 1,4-dioxane (0.63 ml) and a 4M solution of hydrochloric acid in dioxane (0.63 ml) were stirred at ambient temperature for 1 hour. The resultant precipitate was collected by filtration and washed with iso-hexane. The resulting solid was dissolved in aqueous sodium hydroxide solution (2M, 10 ml) and the solution was extracted with ethyl acetate. The combined organic extracts were dried over magnesium sulfate and evaporated to afford the title compound (25 mg, 51%). NMR Spectrum: (CDCl₃) 3.92 (br s, 2H), 6.86 (m, 1H), 7.13 (m, 1H), 7.45 (m, 1H), 7.65 (m, 1H), 7.81 (m, 3H), 8.17 (s, 1H), 8.38 (m, 1H), 8.78 (d, 2H), 9.24 (s, 1H); Mass Spectrum: M+H⁺ 291.

### Example 4

### N3-(2-Aminophenyl)-6-(1H-1,2,4-triazol-1-yl)nicotinamide

A solution of 1-(N-t-butoxycarbonylamino)-2-aminobenzene (Method 6; 104 mg, 0.5 mmol) in DMF (1.6 ml)) was added to 6-(1H-1,2,4-triazol-1-yl)nicotinic acid, (CAS 281232-20-0) (95 mg, 0.5 mmol) followed by 4-(4,6-dimethoxy-1,3,5-triazinyl-2-yl)-4-methylmorpholinium chloride (Method 7, 138 mg, 0.5 mmol) and the reaction mixture stirred at ambient temperature for 48 hours. The solvent was removed *in vacuo* and the resultant residue partitioned between ethyl acetate and water. The organic phase was separated, then washed with water, brine and dried over sodium sulfate. The organic extracts were concentrated by half and a 4M solution of hydrochloric acid in 1,4-dioxane (1 ml) added. The reaction mixture was stirred at ambient temperature for a further 64 hours. The product was purified by preparative HPLC, eluting with an increasing gradient of acetonitrile in water (containing 0.1% (v/v) trifluoroacetic acid) to afford the title compound as its trifluoroacetate (38 mg, 24%); NMR Spectrum (DMSO-d₆) 6.71 (t, 1H), 6.87 (d, 1H), 7.06 (t, 1H), 7.23 (d, 1H), 8.02 (d, 1H), 8.38 (s, 1H), 8.61 (dd, 1H), 9.11 (s, 1H), 9.49 (s, 1H), 9.99 (s, 1H); Mass Spectrum: M+H⁺ 281.

### Example 5

### N2-(2-Aminophenyl)-5-(pyridin-2-yl)thiophene-2-carboxamide

N2-(2-t-Butoxycarbonylaminophenyl)-5-(pyridin-2-yl)thiophene-2-carboxamide (Method 8, 186 mg, 0.47 mmol) was deprotected as Example 1 to afford the title compound (150 mg, 96%); NMR Spectrum (DMSO-d₆): 7.39 (m, 4H), 7.52 (d, 1H), 7.64 (d, 1H), 7.90 (m, 2H), 8.03 (d, 1H), 8.34 (d, 1H), 8.58 (d, 1H), 10.77 (s, 1H); Mass Spectrum: M+H⁺ 296.

### Example 6

### N-(2-Aminophenyl)-3,3'-bipyridine-6-carboxamide

N-(2-t-Butoxycarbonylaminophenyl)-3,3'-bipyridine-6-carboxamide (Method 9; 40 mg, 0.10 mmol), 1,4-dioxane (1 ml) and a 4M solution of hydrochloric acid in 1,4-dioxane (1 ml) were stirred at ambient temperature for 18 hours. The resultant precipitate was collected by filtration and washed with diethyl ether and dried *in vacuo* to give the title compound *tris*hydrochloride (16 mg, 40%); NMR Spectrum (DMSO-d₆): 7.36 (m, 2H), 7.47 (d, 1H), 7.66 (d, 1H), 7.96 (t, 1H), 8.31 (d, 1H), 8.54 (d, 1H), 8.73 (d, 1H), 8.88 (d, 1H), 9.21 (s, 1H), 9.32 (s, 1H), 10.75 (s, 1H); Mass Spectrum: M+H⁺ 291.

### Example 7

### N-(2-Aminophenyl)-2,3'-bipyridine-5-carboxamide

N-(2-t-Butoxycarbonylaminophenyl)-2,3'-bipyridine-5-carboxamide (Method 10; 145 mg, 0.37 mmol), 1,4-dioxane (3 ml) and a 4M solution of hydrochloric acid in 1,4-dioxane (3 ml) were stirred at ambient temperature for 18 hours. The resultant precipitate was collected by filtration and washed with diethyl ether. The solid was then dissolved in water and basified to pH 10 using 28% aqueous ammonium hydroxide. The resulting precipitate was filtered, washed with water and dried *in vacuo* to give the title compound (33 mg, 31%); NMR Spectrum: (DMSO-d₆): 5.00 (s, 2H), 6.61 (t, 1H), 6.81 (d, 1H), 7.00 (t, 1H), 7.22 (d, 1H), 7.58 (m, 1H), 8.24 (d, 1H), 8.45 (d, 1H), 8.52 (d, 1H), 8.69 (d, 1H), 9.26 (s, 1H), 9.36 (s, 1H), 9.85 (s, 1H); Mass Spectrum: M+H⁺ 291.

### Example 8

### N-(2-Aminophenyl)-6-(3-furyl)nicotinamide

N-(2-t-Butoxycarbonylaminophenyl)-6-(3-furyl)nicotinamide (Method 12; 200 mg, 0.5 mmol), 1,4-dioxane (4 ml) and a 4M solution of hydrochloric acid in 1,4-dioxane (4 ml) were stirred at ambient temperature for 18 hours. The resultant precipitate was collected by filtration and washed with diethyl ether. The solid was then dissolved in water and basified to pH 10 using 28% aqueous ammonium hydroxide. The resulting precipitate was filtered off, washed with water and dried *in vacuo* to give the title compound (87 mg, 62%); NMR Spectrum: (DMSO-d₆): 6.85 (t, 1H), 7.01 (d, 1H), 7.15 (m, 2H), 7.32 (d, 1H), 7.83 (s, 1H), 7.90 (d, 1H), 8.39 (d, 1H), 8.48 (s, 1H), 9.16 (s, 1H), 10.08 (s, 1H); Mass Spectrum: M+H⁺ 280.

### Example 9

### N-(2-Aminophenyl)-6-(morpholin-4-yl)nicotinamide

To a solution of 6-(4-morpholino)nicotinic acid (250 mg, 1.2 mmol) and 1-(N-t-butyloxycarbonylamino)-2-aminobenzene (Method 6; 250 mg, 1.2 mmol) in N,N-dimethylformamide (5 ml) was added N-methylmorpholine (0.15 ml, 1.4 mmol) and 2,4-dimethoxy-6-chlorotriazine (246 mg, 1.4 mmol). The resulting mixture was stirred at ambient temperature for 70 hours. The mixture was poured into water and extracted three times with ethyl acetate. The combined organic extracts were dried over magnesium sulfate and evaporated. The residue was purified by flash chromatography eluting with methanol/dichloromethane (0-5%). The product obtained was dissolved in 1,4-dioxane (2.5 ml) and the solution was treated with a 4M solution of hydrochloric acid in 1,4-dioxane (2.4 ml) and the mixture stirred for 2 hours. The solid was collected by filtration, washed with diethyl ether and redissolved in 2M sodium hydroxide solution. The solution was extracted three times with ethyl acetate and the combined organics were dried over magnesium sulfate and evaporated to afford the crude product. This was purified by flash chromatography eluting with methanol/dichloromethane (0-20%) to give the title compound (118 mg, 33%); NMR Spectrum: (DMSO-d₆) 3.59 (m, 4H), 3.70 (m, 4H), 4.87 (s, 2H), 6.60 (t, 1H), 6.78 (d, 1H), 6.91 (d, 1H), 6.96 (t, 1H), 7.16 (d, 1H), 8.11 (d, 1H), 8.76 (s, 1H), 9.45 (s, 1H); Mass Spectrum: M+H⁺ 299.

### Example 10

### N-(2-Aminophenyl)-1',2',3',6'-tetrahydro-2,4'-bipyridine-5-carboxamide

t-Butyl 5-[({2-[(t-butoxycarbonyl)amino]phenyl}amino)carbonyl]-3',6'-dihydro-2,4'-bipyridine-1'(2*'H*)-carboxylate (Method 13; 1.98 g, 4 mmol), 1,4-dioxane (40 ml) and a 4M solution of hydrochloric acid in 1,4-dioxane (40 ml) were stirred at ambient temperature for 18 hours. The resultant precipitate was collected by filtration and washed with diethyl ether and dried *in vacuo* to give the title compound as its *tris*hydrochloride (1.30 g, 81%); NMR Spectrum: (DMSO-d₆) 2.85 (m, 2H), 3.32 (m, 2H), 3.82 (m, 2H), 6.92 (s, 1H), 7.37 (t, 1H), 7.42 (t, 1H), 7.52 (d, 1H), 7.64 (d, 1H), 7.83 (d, 1H), 8.54 (d, 1H), 9.25 (s, 1H), 9.40 (s, 2H), 10.80 (s, 1H); Mass Spectrum: M+H⁺ 295.

### Example 11

### N-(2-Aminophenyl)-1'-benzyl-1',2',3',6'-tetrahydro-2,4'-bipyridine-5-carboxamide

To a suspension of N-(2-aminophenyl)-1',2',3',6'-tetrahydro-2,4'-bipyridine-5-carboxamide *tris*hydrochloride salt (Example 10; 100 mg, 0.25 mmol) in N,N-dimethylformamide (5 ml), triethylamine (0.34 ml, 2.5 mmol) was added and the solution stirred at room temperature for 10 minutes. Benzyl bromide (0.03 ml, 0.25 mmol) and potassium iodide (83 mg, 0.5 mmol) were added and the resulting solution was stirred at 50°C for 18 hours. The cooled solution was purified using an SCX-2 column, eluting with a 2M solution of ammonia in methanol. The resultant residue was further purified by flash chromatography eluting with methanol/dichloromethane (0-20%) to give the title compound (21 mg, 22%); NMR Spectrum: (DMSO-d₆) 2.60 (m, 2H), 2.65 (m, 2H), 3.12 (s, 2H), 3.60 (s, 2H), 4.94 (s, 2H), 6.58 (t, 1H), 6.73 (d, 1H), 6.81 (s, 1H), 6.96 (t, 1H), 7.14 (d, 1H), 7.25 (m, 1H), 7.33 (m, 4H), 7.63 (d, 1H), 8.24 (d, 1H), 9.03 (s, 1H), 9.69 (s, 1H); Mass Spectrum: M+H⁺ 385.

### Example 12

Using a method analogous to that described in Example 11, N-(2-Aminophenyl)-1',2',3',6'-tetrahydro-2,4'-bipyridine-5-carboxamide *tris*hydrochloride salt (Example 10) was reacted with the appropriate starting material to give the compounds described in **Table 1:**

**Table 1**

| | | | | |
|---|---|---|---|---|
| 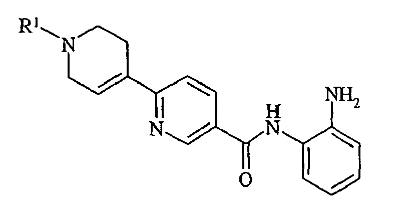 | | | | |

| Note | R¹ | NMR Spectrum | Mass Spectrum | Starting Material |
|---|---|---|---|---|
| 1 | 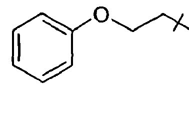 | (DMSO-d₆): 2.52 (m, 2H), 2.60 (s, 2H), 2.77 (t, 2H), 2.85 (t, 2H), 4.14 (t, 2H), 4.93 (s, 2H), 6.58 (t, 1H), 6.75 (d, 1H), 6.84 (s, 1H), 6.96 (m, 4H), 7.16 (d, 1H), 7.26 (t, 2H), 7.66 (d, 1H), 8.25 (d, 1H), 9.05 (s, 1H), 9.70 (s, 1H) | M+H⁺ 415 | |
| 2 | 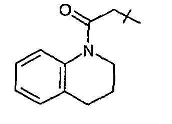 | (DMSO-d₆): 1.90 (t, 2H), 2.60 (m, 2H), 2.70 (m, 2H), 2.79 (m, 2H), 3.30 (m, 2H), 3.49 (s, 2H), 3.77 (m, 2H), 4.95 (s, 2H), 6.60 (t, 1H), 6.79 (d, 1H), 6.83 (s, 1H), 6.99 (t, 1H), 7.10 (d, 1H), 7.20 (m, 3H), 7.62 (m, 1H), 7.68 (d, 1H), 8.28 (d, 1H), 9.08 (s, 1H), 9.73 (s, 1H) | M+H⁺ 468 | Method 39 |
| 3 | 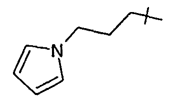 | | M+H⁺ 402.5 | |
| 4 | 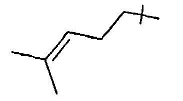 | | M+H⁺ 377.5 | |
| 5 | 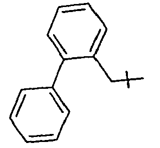 | | M+H⁺ 461.5 | |
| 6 | 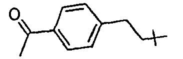 | | M+H⁺ 441.5 | |
| 7 | 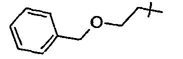 | | M+H⁺ 429.5 | |
| 8 | 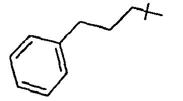 | | M+H⁺ 413.4 | |
| 9 | 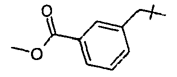 | | M+H⁺ 443.5 | |
| 10 | 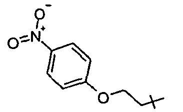 | | M+H⁺ 460.5 | CAS 13288-06-7 |
| 11 | 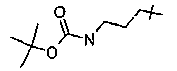 | | M+H⁺ 452.5 | |
| 12 | 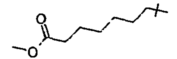 | | M+H⁺ 437.5 | |
| 13 | 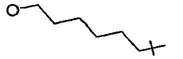 | | M+H⁺ 395.3 | |
| 14 | 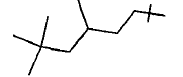 | | M+H⁺ 421.6 | |
| 15 | 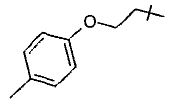 | | M+H⁺ 429.5 | |
| 16 | 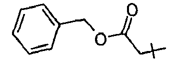 | | M+H⁺ 443.5 | |
| 17 | 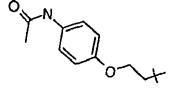 | | M+H⁺ 472.5 | |
| 18 | 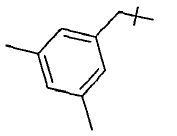 | | M+H⁺ 413.5 | |
| 19 | 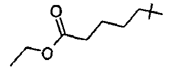 | | M+H⁺ 423.4 | |
| 20 | 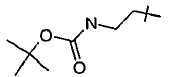 | | M+H⁺ 43 8.5 | |
| 21 | 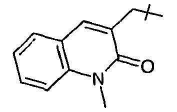 | | M+H⁺ 466.5 | CAS 114561-16-9 |
| 22 | 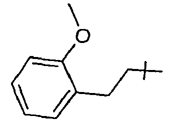 | | M+H⁺ 429.5 | |
| 23 | 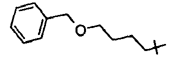 | | M+H⁺ 457.5 | |
| 24 | 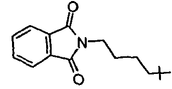 | | M+H⁺ 496.5 | |
| 25 | 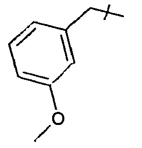 | | M+H⁺ 415.5 | |
| 26 | 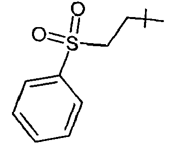 | | M+H⁺ 463.4 | CAS 38798-68-4 |
| 27 | 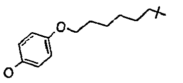 | | M+H⁺ 487.5 | CAS 105589-66-0 |
| 28 | 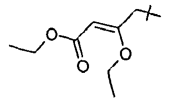 | | M+H⁺ 451.5 | |
| 29 | 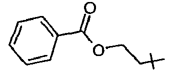 | | M+H⁺ 443.5 | |
| 30 | 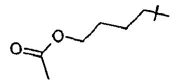 | | M+H⁺ 409.5 | |
| 31 | 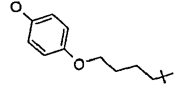 | | M+H⁺ 459.5 | CAS 66619-92-9 |
| 32 | 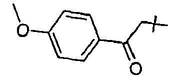 | | M+H⁺ 443.4 | |
| 33 | 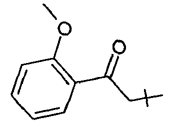 | | M+H⁺ 443.3 | |
| 34 | 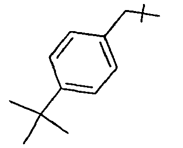 | | M+H⁺ 441.5 | |
| 35 | 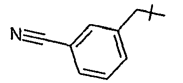 | | M+H⁺ 410.5 | |
| 36 | 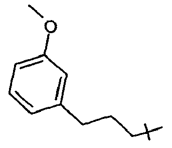 | | M+H⁺ 443.5 | |
| 37 | 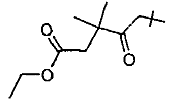 | | M+H⁺ 465.5 | |
| 38 | 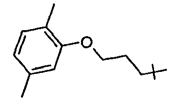 | | M+H⁺ 457.5 | CAS 3245-55-4 |
| 39 | 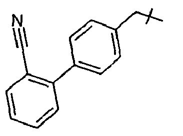 | | M+H⁺ 486.5 | |
| 40 | 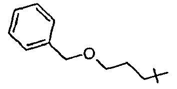 | | M+H⁺ 443.5 | |
| 41 | 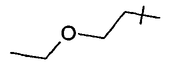 | | M+H⁺ 367.3 | |
| 42 | 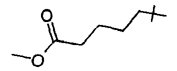 | | M+H⁺ 409.5 | |
| 43 | 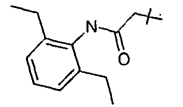 | | M+H⁺ 484.5 | |
| 44 | 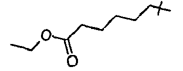 | | M+H⁺ 437.4 | |
| 45 | 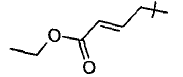 | | M+H⁺ 407.5 | |
| 46 | 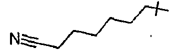 | | M+H⁺ 404.5 | |
| 47 | 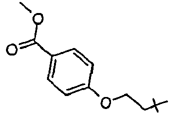 | | M+H⁺ 473.5 | CAS 56850-91-0 |
| 48 | 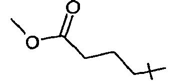 | | M+H⁺ 395.5 | |
| 49 | 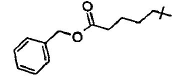 | | M+H⁺ 485.5 | |
| 50 | 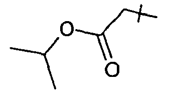 | | M+H⁺ 395.5 | |
| 51 | 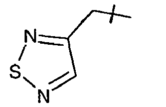 | | M+H⁺ 393.4 | |
| 52 | 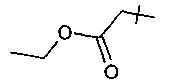 | | M+H⁺ 381.3 | |
| 53 | 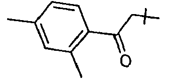 | | M+H⁺ 441.5 | |
| 54 | 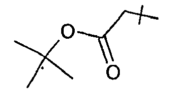 | | M+H⁺ 409.3 | |

### Example 13

### N-(2-Aminophenyl)-6-(4-benzylpiperazin-1-yl)nicotinamide

N-(2-t-Butoxycarbonylaminophenyl)-6-(4-benzylpiperazin-1-yl)nicotinamide (Method 41; 70 mg, 0.14 mmol) was stirred with a 2M solution of hydrochloric acid in 1,4-dioxane (4 ml) for 24 hours. The resulting precipitate was filtered off, washed with ether and dissolved in water. The solution was basified to pH 10 using 28% aqueous ammonium hydroxide. The resulting precipitate was filtered off and washed with water to give the title compound (30 mg, 55%); NMR Spectrum: (DMSO-d₆) 2.43 (m, 4H), 3.52 (s, 2H), 3.62 (s, 4H), 4.84 (s, 2H), 6.60 (t, 1H), 6.76 (d, 1H), 6.87 (d, 1H), 6.95 (t, 1H), 7.12 (d, 1H), 7.31 (m, 4H), 7.42 (m, 1H), 8.09 (d, 1H), 8.72 (s, 1H), 9.40 (s, 1H); Mass Spectrum: M+H⁺ 388.

### Example 14

### N-(2-Aminophenyl)-6-(piperazin-1-yl)nicotinamide

N-(2-t-Butoxycarbonylaminophenyl)-6-(piperazin-1-yl)nicotinamide (Method 42; 226 mg, 0.45 mmol) was stirred with a 2M solution of hydrochloric acid in 1,4-dioxane (8 ml) for 2 hours. The resulting precipitate was filtered off, washed with ether and basified using a 2M solution of sodium hydroxide in water. The product was extracted into ethyl acetate and the organics were washed with brine, dried over magnesium sulfate and evaporated to give the title compound (66 mg, 49%); NMR Spectrum: (DMSO-d₆) 2.79 (t, 4H), 3.55 (t, 4H), 4.88 (s, 2H), 6.58 (t, 1H), 6.78 (d, 1H), 6.85 (d, 1H), 6.95 (t, 1H), 7.16 (d, 1H), 8.05 (d, 1H), 8.71 (s, 1H), 9.40 (s, 1H); Mass Spectrum: M+H⁺-Boc 298.

### Example 15

### N-(2-Aminophenyl)-5-(piperazin-1-yl)pyrazine-2-carboxamide

To a suspension of t-Butyl-4-{5-[({2-[(t-butoxycarbonyl)amino]phenyl}amino)carbonyl] pyrazin-2-yl}piperazine-1-carboxylate (Method 15; 195 mg, 0.39 mmol) in 1,4-dioxane (3 ml) was added a 4M solution of hydrochloric acid in 1,4-dioxane (3 ml). The reaction mixture was allowed to stir at ambient temperature for 16 hours before being diluted with diethyl ether and the solid collected by suction filtration. This solid was dissolved in water and basified by addition of a 28% aqueous solution of ammonium hydroxide. The resultant suspension was extracted with ethyl acetate. The combined organic extracts were washed with brine, dried over magnesium sulfate, filtered and evaporated to give the title compound (87 mg, 75%); NMR Spectrum: (DMSO-d₆) 2.82 (m, 4H), 3.63 (m, 4H), 4.81 (s, 2H), 6.63 (t, 1H), 6.81 (d, 1H), 6.92 (t, 1H), 7.46 (d, 1H), 8.32 (s, 1H), 8.70 (s, 1H), 9.58 (s, 1H); Mass Spectrum: M+H⁺ 299.

### Example 16

### N-(2-Aminophenyl)-5-(4-benzylpiperazin-1-yl)pyrazine-2-carboxamide

To a solution of t-butyl [2-({[5-(4-benzylpiperazin-1-yl)pyrazin-2-yl]carbonyl}amino)phenyl] carbamate (Method 16; 78 mg, 0.15 mmol) in 1,4-dioxane (2 ml) was added a 4M solution of hydrochloric acid in 1,4-dioxane (2 ml). The reaction mixture was allowed to stir at ambient temperature for 6 hours before being diluted with diethyl ether and the solid collected by suction filtration and dried under a stream of air. This solid was dissolved in water (2 ml) and basified to pH 10 by the addition of 28% aqueous ammonium hydroxide solution (5 drops). The resultant precipitate was collected by filtration and dried under vacuum, at 60°C, for 2 hours to afford the title compound as its free base (42 mg, 72%); NMR Spectrum: (DMSO-d₆) 2.52 (m, 4H), 3.55 (s, 2H), 3.74 (m, 4H), 4.83 (s, 2H), 6.64 (t, 1H), 6.82 (d, 1H), 6.94 (t, 1H), 7.27 (m, 1H), 7.35 (m, 4H), 7.48 (d, 1H), 8.34 (s, 1H), 8.71 (s, 1H), 9.58 (s, 1H); Mass Spectrum: M+H⁺ 389.

### Example 17

### N-(2-Aminophenyl)-2-piperazin-1-ylpyrimidine-5-carboxamide

To a solution of t-Butyl-4-{5-[({2-[(t-butoxycarbonyl)amino]phenyl}amino)carbonyl] pyrimidin-2-yl}piperazine-1-carboxylate (Method 18, 290 mg, 0.58 mmol) in 1,4-dioxane (2 ml) was added a 4M solution of hydrochloric acid in 1,4-dioxane (2 ml). The reaction mixture was allowed to stir at ambient temperature for 6 hours before being diluted with diethyl ether and the solid collected by suction filtration and dried under a steam of air. This solid was dissolved in water (2 ml) and basified to pH 10 by the addition of 28% aqueous ammonium hydroxide solution (5 drops). The resultant precipitate was collected by filtration and dried under vacuum at 60°C for 2 hours to afford the title compound as its free base (105 mg, 61%); NMR Spectrum: (DMSO-d₆) 2.75 (m, 4H), 3.77 (m, 4H), 4.91 (s, 2H), 6.58 (t, 1H), 6.77 (d, 1H), 6.96 (t, 1H), 7.14 (d, 1H), 8.88 (s, 2H), 9.45 (s, 1H); Mass Spectrum: M-H 297.

### Example 18

### N-(2-Aminophenyl)-5-piperazin-1-ylpyrazine-2-carboxamide trishydrochloride

To a suspension of t-Butyl-4-{5-[({2-[(t-butoxycarbonyl)amino]phenyl}amino)carbonyl] pyrazin-2-yl}piperazine-1-carboxylate (Method 15, 1.21 g, 2.43 mmol) in 1,4-dioxane (30 ml) was added a 4M solution of hydrochloric acid in 1,4-dioxane (30 ml). The reaction mixture was allowed to stir at ambient temperature for 3 hours before being diluted with diethyl ether and the solid collected by suction filtration and dried *in vacuo* at 60°C to give the title compound (1.02g, 87%); NMR Spectrum: (DMSO-d₆) 3.24 (m, 4H), 4.01 (m, 4H), 7.24 (m, 2H), 7.32 (m, 1H), 7,58 (d, 1H), 8.45 (s, 1H), 8.79 (s, 1H), 9.40 (s, 1H), 10.24 (s, 1H).

### Example 19

### N-(2-Aminophenyl)-2-[4-(3-anilino-3-oxopropyl)piperazin-1-yl]pyrazine-5-carboxamide

A mixture of N-(2-Aminophenyl)-5-piperazin-1-ylpyrazine-2-carboxamide (Example 18; 53 mg, 0.18 mmol), 3-chloro-N-phenylpropanamide (34 mg, 0.19 mmol), potassium iodide (55 mg, 0.33 mmol) and triethylamine (250 µl, 1.79 mmol) in N,N-dimethylformamide (3 ml) was heated to 80°C for 16 hours. The reaction mixture was then allowed to cool before being poured into water. The precipitate was collected by suction filtration to give the title compound as its DMF adduct (10 mg, 11%); NMR Spectrum: (DMSO-d₆) 2.52 (m, 2H), 2.56 (m, 4H), 3.29 (m, 2H), 3.90 (m, 4H), 4.83 (s, 2H), 6.64 (t, 1H), 6.82 (d, 1H), 6.94 (t, 1H), 7.03 (t, 1H), 7.29 (t, 2H), 7.47 (d, 1H), 7.59 (d, 2H), 8.37 (s, 1H), 8.71 (s, 1H), 9.59 (s, 1H), 10.00 (s, 1H); Mass Spectrum: M+H⁺ 446.

### Example 20

Using an analogous procedure to that described for Example 19, N-(2-aminophenyl)-5-piperazin-1-ylpyrazine-2-carboxamide *tris*hydrochloride (Example 18) was reacted with the appropriate alkylating agent at the temperature and for the duration indicated to give the compounds described in **Table 2**:

**Table 2**

| | | | | | |
|---|---|---|---|---|---|
| 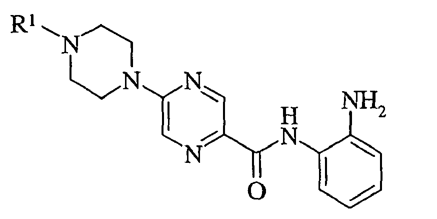 | | | | | |

| Note | R¹ | NMR Spectrum | Mass Spectrum | Reaction conditions | Starting material |
|---|---|---|---|---|---|
| 1 | 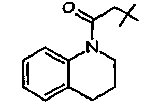 | (DMSO-d₆) 1.91 (qn, 2H), 2.59 (m, 4H), 2.73 (t, 2H), 3.40 (s, 2H), 3.69 (m, 4H), 3.74 (t, 2H), 4.83 (s, 2H), 6.64 (t, 1H), 6.82 (d, 1H), 6.94 (t, 1H), 7.09 (t, 1H), 7.18 (m, 2H), 7.48 (d, 1H), 7.58 (m, 1H), 8.34 (s, 1H), 8.71 (s, 1H), 9.59 (s, 1H) | M+H⁺ 472 | 80°C, 16 hours | Method 39 |
| 2 | 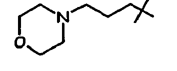 | (DMSO-d₆) 1.62 (qn, 2H), 2.33 (m, 8H), 2.52 (m, 4H), 3.57 (m, 4H), 3.72 (m,4H), 4.83 (s, 2H), 6.64 (t, 1H), 6.82 (d, 1H), 6.94 (t, 1H), 7.48 (d, 1H), 8.35 (s, 1H), 8.71 (s, 1H), 9.59 (s, 1H) | M+H⁺ 426 | 80°C, 16 hours | |
| 3 | 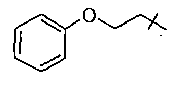 | (DMSO-d₆) 2.64 (m, 4H), 2.78 (t, 2H), 3.75 (m, 4H), 4.13 (t, 2H), 4.83 (s, 2H), 6.64 (t, 1H), 6.82 (d, 1H), 6.94 (m, 4H), 7.29 (t, 2H), 7.48 (d, 1H), 8.37 (s, 1H), 8.71 (s, 1H), 9.60 (s, 1H) | M+H⁺ 419 | 80°C, 16 hours | |

### Example 21

### N-(2-Aminophenyl)-2-[4-(2-phenoxyethyl)piperazin-1-yl]pyrimidine-5-carboxamide

To a solution of N-(2-aminophenyl)-2-piperazin-1-ylpyrimidine-5-carboxamide (Example 17; 45 mg, 0.15 mmol) in N,N-dimethylformamide (2 ml) was added β-bromophenetole ([CAS 589-10-6], 34 mg, 0.17 mmol), triethylamine (210 µl, 1.51 mmol) and potassium iodide (48 mg, 0.29 mmol). The reaction mixture was heated to 80°C and stirred for 16 hours before being allowed to cool and poured onto water (20 ml). The resultant precipitate was collected by suction filtration, washed with water and diethyl ether and dried to give the title compound (36 mg, 57%); NMR Spectrum: (DMSO-d₆) 2.58 (m, 4H), 2.77 (t, 2H), 3.86 (m, 4H), 4.13 (t, 2H), 4.92 (s, 2H), 6.58 (t, 1H), 6.77 (d, 1H), 6.95 (m, 4H), 7.14 (d, 1H), 7.29 (t, 2H), 8.89 (s, 2H), 9.47 (s, 1H); Mass Spectrum: M+H⁺ 419.

### Example 22

### N-(2-Aminophenyl)-5-(pyrrolidin-1-yl)thiophene-2-carboxamide

N-(2-t-Butoxycarbonylaminophenyl)-5-(pyrrolidin-1-yl)thiophene-2-carboxamide (Method 21; 62 mg, 0.160 mmol), 1,4-dioxane (0.6 ml) and a 4M solution of hydrochloric acid in 1,4-dioxane (0.6 ml) were stirred at ambient temperature for18 hours. The resultant precipitate was collected by filtration and washed with diethyl ether. A solution of the resulting solid in water (10 ml) was basified to pH 14 with 2M aqueous sodium hydroxide solution, and the product was extracted with ethyl acetate. The combined organic extracts were washed with brine, dried over sodium sulfate, filtered and evaporated to afford the title compound (32 mg, 70%); NMR Spectrum: (CDCl₃) 2.08 (m, 4H), 3.34 (m, 4H), 3.92 (s, 2H), 5.72 (d, 1H), 6.82 (m, 2H), 7.05 (m, 1H), 7.29 (m, 2H), 7.36 (d, 1H); Mass Spectrum: M+H⁺ 288.

### Example 23

### N-(2-Aminophenyl)-5-(piperidin-1-yl)thiophene-2-carboxamide

N-(2-t-Butoxycarbonylaminophenyl)-5-(piperidin-l-yl)thiophene-2-carboxamide (Method 24, 103 mg, 0.257 mmol), 1,4-dioxane (2.0 ml) and a 4M solution of hydrochloric acid in 1,4-dioxane (2.0 ml) were stirred at ambient temperature for 70 hours. The resultant precipitate was collected by filtration and washed with diethyl ether. A suspension of the resulting solid in water (20 ml) was basified to pH 14 with 2M aqueous sodium hydroxide solution and the product was extracted with ethyl acetate. The combined organic extracts were washed with brine, dried over sodium sulfate, filtered and evaporated. The resultant residue was purified by flash chromatography eluting with 2% methanol/dichloromethane to afford the title compound (31 mg, 40%); NMR Spectrum: (CDCl₃) 1.62 (m, 2H), 1.73 (m, 4H), 3.26 (m, 4H), 3.91 (s, 2H), 6.00 (d, 1H), 6.82 (m, 2H), 7.06 (m, 1H), 7.29 (m, 1H), 7.36 (m, 2H); Mass Spectrum: M+H⁺ 302

### Example 24

### N-(2-Aminophenyl)-5-piperidin-4-ylthiophene-2-carboxamide

t-Butyl 4-(5- {[(2-t-butoxycarbonylaminophenyl)amino]carbonyl}-2-thienyl)piperidine-1-carboxylate (Method 27, 130 mg, 0.26 mmol) was stirred and dissolved in 1,4-dioxane (1.0 ml) and a 4M solution of hydrochloric acid in 1,4-dioxane (1.0 ml, 4 mmol) added. The reaction mixture was stirred at ambient temperature for 24 hours. The resultant precipitate was collected by filtration to give the hydrochloride. This was dissolved in water and extracted with ethyl acetate. The aqueous solution was basified with 28% aqueous ammonia solution, extracted with ethyl acetate, the organic layer washed with brine, dried over magnesium sulfate, filtered and evaporated to give the title compound (20 mg, 26%); NMR Spectrum: (DMSO-d₆) 1.51 (m, 2H), 1.87 (m, 2H), 2.59 (m, 2H), 2.96 (m, 3H), 4.87 (s, 2H), 6.59 (m, 1H), 6.78 (d, 1H), 6.96 (m, 2H), 7.12 (d, 1H), 7.81 (d, 1H), 9.56 (s, 1H); Mass Spectrum: M+H⁺ 302.

### Example 25

### N-(2-Aminophenyl)-5-(1,2,3,6-tetrahydropyridin-4-yl)thiophene-2-carboxamide

t-Butyl 4-(5-{[(2-t-butoxycarbonylaminophenyl)amino]carbonyl }-2-thienyl)-3,6-dihydropyridine-1(2*H*)-carboxylate (Method 28, 1.0 g, 2 mmol) was stirred and dissolved in 1,4-dioxane (7.5 ml) and 4M solution of hydrochloric acid in 1,4-dioxane (7.5 ml, 30 mmol) added. The reaction mixture was stirred at ambient temperature for 24 hours. The resultant precipitate was collected by filtration, dissolved in water and extracted with ethyl acetate. The aqueous solution was basified with 28% ammonia solution then extracted with ethyl acetate. The organic extracts were washed with brine, dried over magnesium sulfate, filtered and evaporated to give the title compound (88 mg, 15%); NMR Spectrum: (DMSO-d₆) 2.34 (d, 2H), 2.90 (t, 2H), 3.36 (d, 2H), 4.88 (s, 2H), 6.31 (s, 1H), 6.60 (t, 1H), 6.78 (d, 1H), 6.98 (t, 1H), 7.13 (d, 2H), 7.86 (d, 1H), 9.61 (s, 1H); Mass Spectrum: M+H⁺300.

### Example 26

### N-(2-Aminophenyl)-5-(1-benzylpiperidin-4-yl)thiophene-2-carboxamide

To a suspension of N-(2-Aminophenyl)-5-piperidin-4-ylthiophene-2-carboxamide (Example 24; 50 mg, 0.14 mmol) in N,N-dimethylformamide (3 ml) was added triethylamine (0.19 ml, 1.4 mmol) and the solution stirred at room temperature for 10 minutes. Benzyl bromide (27 mg, 0.16 mmol) and potassium iodide (23 mg, 0.14 mmol) were added and the resulting solution was stirred at ambient temperature for 18 hours. The solution was evaporated. The resultant residue was purified by flash chromatography eluting with methanol/dichloromethane (0-20%) to give a solid residue. This residue was dissolved in dichloromethane, washed with water and evaporated to give the title compound (28 mg, 50%); NMR Spectrum: (DMSO-d₆) 1.82 (m, 2H), 2.20 (m, 2H), 3.12 (m, 2H), 3.50 (m, 3H), 4.35 (s, 2H), 4.91 (s, 2H) 6.21 (t, 1H), 6.80 (d, 1H), 6.98 (m, 2H), 7.14 (d, 1H), 7.50 (m, 5H), 7.86 (s, 1H), 9.61 (s, 1H); Mass Spectrum: M+H⁺ 392

### Example 27

### t-Butyl {3-[4-(5-{[(2-aminophenyl)amino]carbonyl}-2-thienyl)-3,6-dihydropyridin-1(2H)-yl]propyl}carbamate

To a solution of N-(2-Aminophenyl)-5-(1,2,3,6-tetrahydropyridin-4-yl)thiophene-2-carboxamide (Example 25; 168 mg, 0.56 mmol) in N,N-dimethylacetamide (5 ml) was added 3-(t-butoxycarbonylamino)propyl bromide (147 mg, 0.62 mmol) and triethylamine (1 ml, 7.2 mmol) and the resulting solution stirred at 50°C for 4 hours. The cooled solution was evaporated and the residue purified by flash chromatography eluting with methanol/dichloromethane (0-25%) to give the title compound (201 mg, 79%); NMR Spectrum: (MeOH-d₄) 1.34 (s, 9H), 1.69 (t, 2H), 2.59 (s, 4H), 2.86 (s, 2H), 3.02 (t, 2H), 3.28 (s, 2H), 6.18 (s, 1H), 6.65 (t, 1H), 6.79 (d, 1H), 6.97 (t, 1H), 7.05 (m, 3H), 7.65 (s, 1H); Mass Spectrum: M+H⁺ 457.

### Example 28

Using a method analogous to that described inEexample 26, N-(2-aminophenyl)-5-(piperidin-4-yl)thiophene-2-carboxamide (Example 24) was reacted with the appropriate alkylating agent at the appropriate temperature. The crude products were purified using an SCX-2 column, eluting with a 2M solution of ammonia in methanol. The resultant residue was further purified by flash chromatography eluting with methanol/dichloromethane (0-20%) to give the compounds described in **Table 3**:

**Table 3**

| | | | | | |
|---|---|---|---|---|---|
| 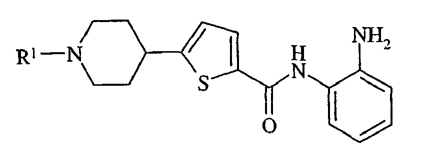 | | | | | |

| Note | R¹ | NMR Spectrum | Mass Spectrum | Starting Material | Temperature |
|---|---|---|---|---|---|
| 1 | 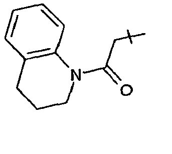 | (DMSO-d₆): 1.15 (m, 2H), 1.55 (m, 2H), 1.91 (m, 4H), 2.20 (m, 2H), 2.73 (m, 2H), 2.85 (m, 2H), 3.40 (m, 1H), 3.72 (m, 2H), 4.85 (s, 2H), 6.58 (t, 1H), 6.76 (d, 1H), 6.96 (m, 2H), 7.06 (m, 2H), 7.14 (m, 2H), 7.57 (m, 1H), 7.78 (d, 1H), 9.55 (s, 1H). | M+H⁺ 475 | Method 39 | Ambient |
| 2 | 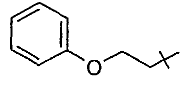 | (DMSO-d₆): 1.65 (m, 2H), 1.94 (m, 2H), 2.21 (m, 2H), 2.75 (m, 3H), 3.02 (m, 2H), 4.08 (s, 2H), 4.86 (s, 2H), 6.56 (t, 1H), 6.76 (d, 1H), 6.96 (m, 5H), 7.10 (d, 1H), 7.25 (m, 2H), 7.78 (s, 1H), | M+H⁺ 422 | | Ambient |
| | | 9.54 (s, 1H). | | | |
| 3 | 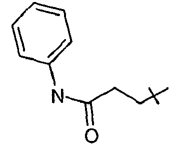 | (DMSO-d₆): 1.68 (m, 2H), 2.02 (m, 2H), 2.18 (m, 2H), 2.72 (m, 2H), 2.78 (s, 1H), 2.90 (m, 2H), 3.05 (m, 2H), 4.94 (s, 2H), 6.65 (t, 1H), 6.82 (d, 1H), 7.05 (m, 3H), 7.15 (d, 1H), 7.35 (m, 2H), 7.64 (m, 2H), 7.90 (s, 1H), 9.64 (s, 1H), 10.10 (s, 1H). | M+H⁺ 449 | | 80°C |
| 4 | 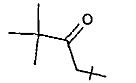 | | M+H⁺ 399.6 | | 40 °C |
| 5 | 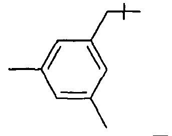 | | M+H⁺ 419.6 | | 40 °C |
| 6 | 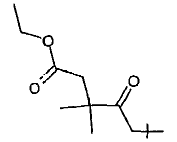 | | M+H⁺ 471.6 | | 40 °C |
| 7 | 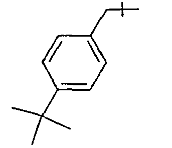 | | M+H⁺ 447.7 | | 40 °C |
| 8 | 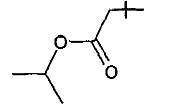 | | M+H⁺ 401.5 | | 40 °C |
| 9 | 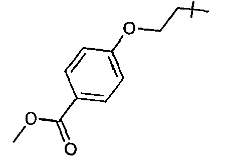 | | M+H⁺ 479.6 | CAS 56850-91-0 | 40 °C |
| 10 | 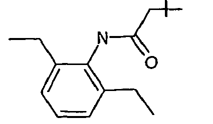 | | M+H⁺ 490.7 | | 40 °C |
| 11 | 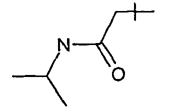 | | M+H⁺ 400.6 | CAS 75726-96-4 | 40 °C |
| 12 | 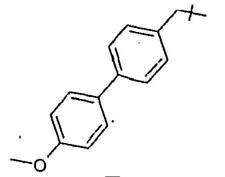 | | M+H⁺ 497.7 | CAS 20854-61-9 | 40°C |
| 13 | 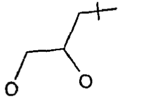 | | M+H⁺ 375.5 | | 40°C |
| 14 | 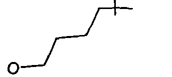 | | M+H⁺ 373.5 | | 40 °C |
| 15 | 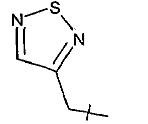 | | M+H⁺ 399.5 | | 40 °C |
| 16 | 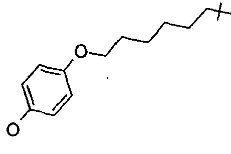 | | M+H⁺ 493.7 | CAS 105589-66-0 | 40°C |
| 17 | 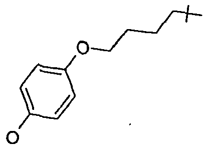 | | M+H⁺ 465.6 | CAS 66619-92-9 | 40°C |
| 18 | 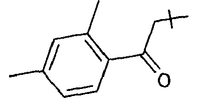 | | M+H⁺ 447.6 | | 40°C |
| 19 | 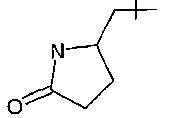 | | M+H⁺ 398.5 | | 40°C |
| 20 | 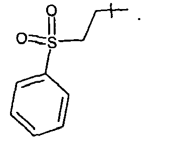 | | M+H⁺ 469.6 | CAS 38798-68-4 | 40°C |
| 21 | 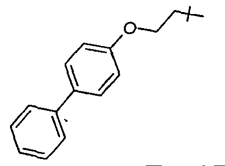 | | M+H⁺ 497.7 | CAS 3351-60-8 | 40°C |

### Example 29

### N-(2-Aminophenyl)-5-piperazin-1-ylthiophene-2-carboxamide

t-Butyl 4-{5-[({2-[(t-butoxycarbonyl)amino]pheny}amino)carbonyl]thien-2-yl}piperazine-1-carboxylate (Method 29; 150 mg, 0.30 mmol), 1,4-dioxane (0.45 ml) and a 4M solution of hydrochloric acid in 1,4-dioxane (0.45 ml) were stirred at ambient temperature for 18 hours. The resultant precipitate was collected by filtration and washed with diethyl ether and dried *in vacuo* to give the title compound dihydrochloride. This solid was suspended in dichloromethane (5 ml) and the resulting suspension was treated with 1,1,3,3-tetramethylguanidine (55 µl) to give a clear solution. This solution was purified by flash chromatography eluting with methanol/dichloromethane (5 - 20%) to give the title compound (83 mg, 92%); NMR Spectrum: (DMSO-d₆) 3.03 (m, 4H), 3.27 (m, 4H), 4.85 (s, 2H), 6.23 (d, 1H), 6.57 (t, 1H), 6.77 (d, 1H), 6.93 (t, 1H), 7.10 (d, 1H), 7.75 (d, 1H), 9.39 (s, 1H); Mass Spectrum: M+H⁺ 303.

### Example 30

### N-(2-Aminophenyl)-5-(4-benzylpiperazin-1-yl)thiophene-2-carboxamide

A mixture of N-(2-aminophenyl)-5-(piperazin-1-yl)thiophene-2-carboxamide (Example 29, 80.0 mg, 0.26 mmol), benzyl bromide (45.3 mg, 0.26 mmol), triethylamine (50.5 mg, 0.52 mmol) and N,N-dimethylformamide (2.0 ml) were allowed to stand at ambient temperature for 18 hours. The mixture was evaporated and the residue was purified by flash chromatography eluting with methanol/dichloromethane (0.5-5%) to give the title compound (29 mg, 28%); NMR Spectrum: (DMSO-d₆) 2.49 (m, 4H), 3.19 (m, 4H), 3.53 (s, 2H), 4.81 (s, 2H), 6.17 (d, 1H), 6.56 (t, 1H), 6.74 (d, 1H), 6.92 (t, 1H), 7.10 (d, 1H), 7.26 (m, 1H), 7.33 (m, 4H), 7.68 (d, 1H), 9.28 (s, 1H); Mass Spectrum: M+H⁺ 393.

### Example 31

### N-(2-aminophenyl)-2-(4-methylpiperazin-1-yl)-1,3-thiazole-5-carboxamide

Nickel (II) acetate (70 mg, 0.28 mmol) was added to a stirred suspension of N-(2-nitrophenyl)-2-(4-methylpiperazin-1-yl)-1,3-thiazole-5-carboxamide (Method 32; 50 mg, 0.14 mmol) in methanol (4 ml) at 0°C. Sodium borohydride (53 mg, 1.4 mmol) was added over 15 minutes and the resulting mixture stirred at ambient temperature for 4 hours. The mixture was filtered, the solvent evaporated and the residue purified by flash chromatography eluting with methanol/dichloromethane (0-25%) to give the title compound (25 mg, 57%); NMR Spectrum: (DMSO-d₆) 2.24 (s, 3H), 2.43 (s, 4H), 3.49 (t, 4H), 4.76 (s, 2H), 6.56 (t, 1H), 6.75 (d, 1H), 6.96 (t, 1H), 7.11 (d, 1H), 8.01 (s, 1H), 9.43 (s, 1H); Mass Spectrum: M+H⁺ 318.5

### Example 32

### N-(2-Aminophenyl)-2-(4-benzylpiperazin-1-yl)-1,3-thiazole-5-carboxamide

To a solution of N-(2-aminophenyl)-2-piperazin-1-yl-1,3-thiazole-5-carboxamide (Method 34; 100 mg, 0.33 mmol) in N,N-dimethylformamide (5 ml) was added potassium iodide (82 mg, 0.49 mmol), benzyl bromide (62 mg, 0.36 mmol) and triethylamine (0.46 ml, 3.3 mmol) and the resulting solution stirred at ambient temperature for 48 hours. The product was isolated using an SCX-2 column, eluting with a 2M solution of ammonia in methanol. The resultant residue was purified by reverse phase preparative. HPLC eluting with CH₃CN/0.1%NH₃ (5-95%) in water to give the title compound (63 mg, 49%); NMR Spectrum: (DMSO-d₆): 2.50 (s, 4H), 3.50 (s, 4H), 3.55 (s, 2H), 4.85 (s, 2H), 6.58 (t, 1H), 6.76 (d, 1H), 6.95 (t, 1H), 7.10 (d, 1H), 7.28 (m, 1H), 7.34 (s, 4H), 8.01 (s, 1H), 9.42 (s, 1H); Mass Spectrum: M+H⁺ 394.

### Example 33

Using a method analogous to that described in Example 32, N-(2-aminophenyl)-2-piperazin-1-yl-1,3-thiazole-5-carboxamide (Method 34) was reacted with the appropriate alkylating agent, at the temperature and for the duration indicated to give the compounds described in **Table 4:**

**Table 4**

| | | | | | |
|---|---|---|---|---|---|
| 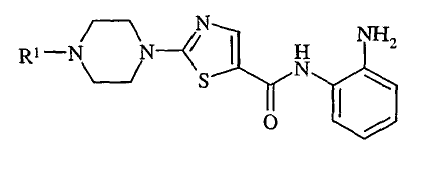 | | | | | |

| Note | R¹ | NMR Spectrum | Mass Spectrum | Reaction conditions | Starting Material |
|---|---|---|---|---|---|
| 1 | 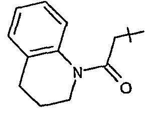 | (DMSO-d₆): 1.90 (m, 2H), 2.60 (s, 4H), 2.72 (t, 2H), 3.41 (s, 2H), 3.45 (s, 4H), 3.73 (t, 2H), 4.86 (s, 2H), 6.58 (t, 1H), 6.76 (d, 1H), 6.95 (t, 1H), 7.09 (m, | M+H⁺ 477 | 1 hour at Ambient temperature | Method 39 |
| | | 2H), 7.17 (m, 2H), 7.57 (m, 1H), 8.01 (s, 1H), 9.42 (s, 1H). | | | |
| 2 | 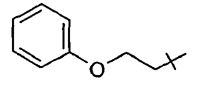 | (DMSO-d₆): 2.63 (s, 4H), 2.78 (t, 2H), 3.49 (s, 4H), 4.10 (t, 2H), 4.84 (s, 2H), 6.56 (t, 1H), 6.75 (d, 1H), 6.93 (m, 4H), 7.08 (d, 1H), 7.27 (t, 2H), 8.00 (s, 1H), 9.40 (s, 1H). | M+H⁺ 424 | 48 hours at Ambient temperature, then 1 hour at 80°C | |
| 3 | 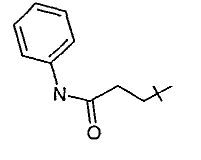 | (CDCl₃): 2.55 (t, 2H), 2.69 (s, 4H), 2.78 (t, 2H), 3.64 (s, 4H), 3.89 (s, 2H), 6.79 (d, 2H), 7.07 (m, 2H), 7.27 (m, 3H), 7.49 (d, 2H), 7.73 (s, 2H), 10.12 (s, 1H). | M+H⁺ 451 | 48 hours ambient temperature, then 18 hours at 80°C | |
| 4 | 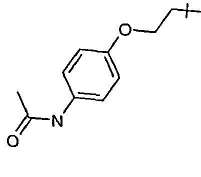 | | M+H⁺ 481.5 | 24 hours at 40°C | CAS 57011-90-2 |
| 5 | 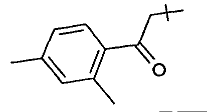 | | M+H⁺ 450.5 | 24 hours at 40°C | |
| 6 | 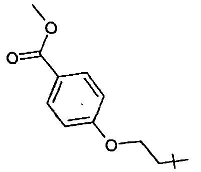 | | M+H⁺ 482.5 | 24 hours at 40°C | CAS 56850-91-0 |
| 7 | 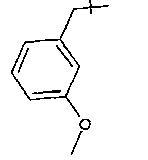 | | M+H⁺ 424.4 | 24 hours at 40°C | |
| 8 | 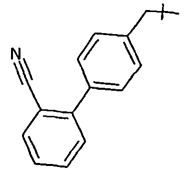 | | M+H⁺ 495.5 | 24 hours at 40°C | |
| 9 | 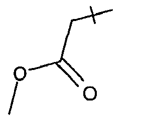 | | M+H⁺ 376.4 | 24 hours at 40°C | |
| 10 | 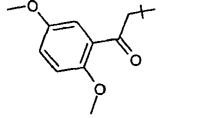 | | M+H⁺ 482.3 | 24 hours at 40°C | |
| 11 | 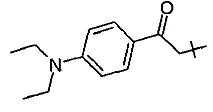 | | M+H⁺ 493.5 | 24 hours at 40°C | |
| 12 | 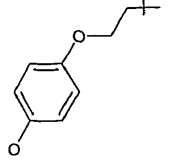 | | M+H⁺ 440.4 | 24 hours at 40°C | |
| 13 | 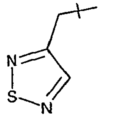 | | M+H⁺ 402.4 | 24 hours at 40°C | |
| 14 | 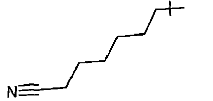 | | M+H⁺ 413.4 | 24 hours at 40°C | |
| 15 | 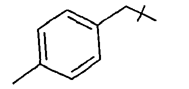 | | M+H⁺ 408.5 | 24 hours at 40°C | |
| 16 | 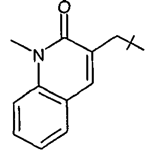 | | M+H⁺ 475.5 | 24 hours at 40°C | CAS 114561-16-9 |
| 17 | 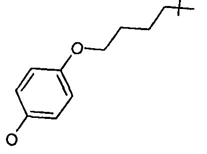 | | M+H⁺ 468.5 | 24 hours at 40°C | CAS 66619-92-9 |
| 18 | 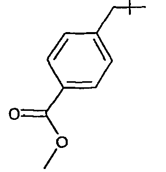 | | M+H⁺ 452.4 | 24 hours at 40°C | |
| 19 | 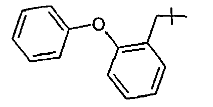 | | M+H⁺ 486.5 | 24 hours at 40°C | |
| 20 | 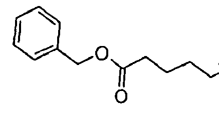 | | M+H⁺ 494.5 | 24 hours at 40°C | |
| 21 | 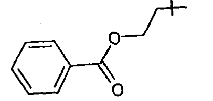 | | M+H⁺ 452.4 | 24 hours at 40°C | |
| 22 | 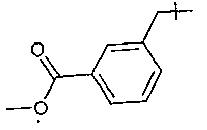 | | M+H⁺ 452.4 | 24 hours at 40°C | |
| 23 | 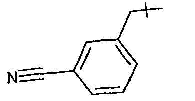 | | M+H⁺ 419.4 | 24 hours at 40°C | |
| 24 | 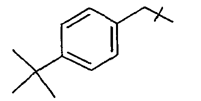 | | M+H⁺ 450.5 | 24 hours at 40°C | |
| 25 | 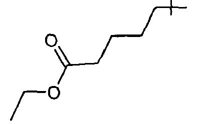 | | M+H⁺ 432.4 | 24 hours at 40°C | |
| 26 | 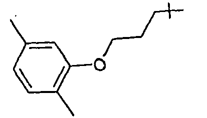 | | M+H⁺ 466.5 | 24 hours at 40°C | CAS 3245-55-4 |
| 27 | 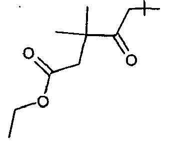 | | M+H⁺ 474.5 | 24 hours at 40°C | |
| 28 | 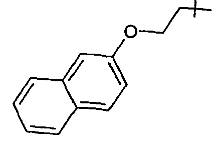 | | M+H⁺ 474.5 | 24 hours at 40°C | |
| 29 | 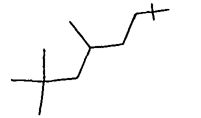 | | M+H⁺ 430.5 | 24 hours at 40°C | |
| 30 | 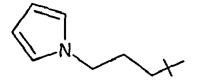 | | M+H⁺ 411.5 | 24 hours at 40°C | |
| 31 | 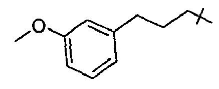 | | M+H⁺ 452.4 | 24 hours at 40°C | |
| 32 | 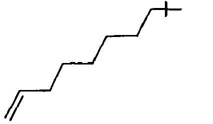 | | M+H⁺ 414.5 | 24 hours at 40°C | |
| 33 | 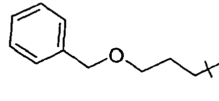 | | M+H⁺ 452.5 | 24 hours at 40°C | |
| 34 | 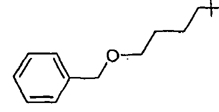 | | M+H⁺ 466.5 | 24 hours at 40°C | |
| 35 | 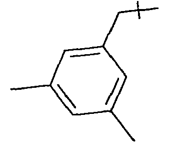 | | M+H⁺ 422.4 | 24 hours at 40°C | |
| 36 | 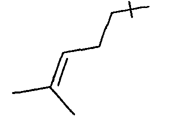 | | M+H⁺ 386.5 | 24 hours at 40°C | |
| 37 | 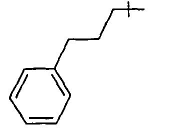 | | M+H⁺ 422.4 | 24 hours at 40°C | |
| 38 | 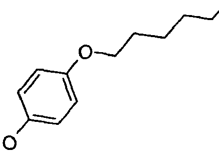 | | M+H⁺ 496.5 | 24 hours at 40°C | CAS 105589-66-0 |
| 39 | 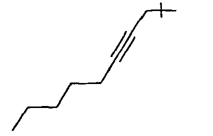 | | M+H⁺ 412.5 | 24 hours at 40°C | |

### Preparation of the Starting Materials

The starting materials for the above examples are either commercially available or are readily prepared by standard methods from known materials. For example the following reactions are illustrations but not limitations of the preparation of some of the starting materials used in the above reactions.

### Method 1

### N2-(2-t-Butoxycarbonylaminophenyl)-5-(pyridin-3-yl)thiophene-2-carboxamide

N2-(2-t-Butoxycarboylaminophenyl)-5-bromothiophene-2-carboxamide (Method 3; 200 mg, 0.50 mmol), pyridine-3-boronic acid (74 mg, 0.60 mmol), *tetrakis*(triphenylphosphine) palladium (5 mg, 0.005 mmol), 1,2-dimethoxyethane (3 ml) and a saturated aqueous solution of sodium hydrogen carbonate (3 ml) were stirred at 80°C under an atmosphere of argon for 72 hours. The cooled mixture was partitioned between ethyl acetate and water. The organics were washed with brine, dried over magnesium sulfate and evaporated. The resultant residue was purified by flash chromatography eluting with methanol/dichloromethane (2%) to give the title compound (90 mg, 46%) ; NMR Spectrum: (DMSO-d₆) 1.43 (s, 9H), 7.16 (m, 2H), 7.55 (m, 3H), 7.73 (d, 1H), 7.92 (d, 1H), 8.13 (m, 1H), 8.55 (d, 1H), 8.63 (br, 1H), 8.95 (s, 1H), 9.86 (s, 1H); Mass Spectrum: M+H⁺ 396.

### Method 2

### N2-(2-t-Butoxycarbonylaminophenyl)-5-(pyridin-4-yl)-thiophene-2-carboxamide

N2-(2-t-Butoxycarboylaminophenyl)-5-bromothiophene-2-carboxamide (Method 3; 200 mg, 0.50 mmol), pyridine-4-boronic acid (74 mg, 0.60 mmol), *tetrakis*(triphenylphosphine) palladium (5 mg, 0.005 mmol), 1,2-dimethoxyethane (3 ml) and a saturated aqueous solution of sodium hydrogen carbonate (3 ml) were stirred at 80°C under an atmosphere of argon for 72 hours. The cooled mixture was partitioned between ethyl acetate and water. The organics were washed with brine dried over magnesium sulfate and evaporated. The resultant residue was purified by flash chromatography eluting with methanol/dichloromethane (2 %) to give the title compound (136 mg, 69 %); NMR Spectrum: (DMSO-d₆) 1.42 (s, 9H), 7.16 (m, 2H), 7.52 (m, 2H), 7.70 (d, 2H), 7.87 (d, 1H), 7.94 (d, 1H), 8.60 (d, 2H), 8.65 (br, 1H), 9.90 (br, 1H); Mass Spectrum: M+H⁺ 396.

### Method 3

### N2-(2-t-Butoxycarboylaminophenyl)-5-bromothiophene-2-carboxamide

5-Bromothiophene-2-carboxylic acid (1.50 g, 7.25 mmol) and 1-(N-t-butoxycarbonylamino)-2-aminobenzene (Method 6; 1.66 g, 7.97 mmol) were stirred together in *N,N-*dimethylacetamide (50 ml) for 10 minutes, 4-(4,6-dimethoxy-1,3,5-triazinyl-2-yl)-4-methylmorpholinium chloride (Method 7; 2.41 g, 8.70 mmol) was added and the mixture stirred at ambient temperature for 24 hours. The solvent was evaporated and the residue partitioned between ethyl acetate and water. The organic extracts were dried over magnesium sulfate and evaporated then the resultant residue purified by flash chromatography eluting with dichloromethane to give the title compound (2.67 g, 93 %); NMR Spectrum: (DMSO-d₆), 1.44 (s, 9H), 7.16 (m, 2H), 7.35 (d, 1H), 7.42 (d, 1H), 7.55 (d, 1H), 7.72 (d, 1H), 8.60 (br, 1H), 9.81 (br, 1H); Mass Spectrum:( M+H⁺ -Boc) 299.

### Method 4

### N3-(2-t-Butoxycarbonylaminophenyl)-6-(pyridin-4-yl)nicotinamide

N3-(2-t-Butoxycarbonylaminophenyl)-6-bromonicotinamide (Method 5; 100 mg, 0.26 mmol), pyridine-4-boronic acid (38 mg, 0.31 mmol), *tetrakis*(triphenylphosphine) palladium (10 mg), saturated sodium carbonate solution (2 ml) and dimethoxyethane (2 ml) was stirred at 80°C for 48 hours under an atmosphere of argon. The mixture was poured into brine (20 ml) and extracted with ethyl acetate. The combined organic extracts were dried over magnesium sulfate and evaporated. The resultant residue was purified by flash column chromatography, eluting with methanol/ethyl acetate (0-10%), to give the title compound (65 mg, 64 %). NMR Spectrum: (CDCl₃) 1.52 (s, 9H), 7.19 (m, 2H), 7.43 (m, 1H), 7.61 (m, 1H), 7.83 (m, 2H), 7.94 (d, 2H), 8.37 (dd, 1H), 8.75 (d, 2H), 9.31 (d, 1H), 9.79 (brs, 1H); Mass Spectrum: M+H⁺ 391.

### Method 5

### N3-(2-t-Butoxycarbonylaminophenyl)-6-bromonicotinamide

6-Bromonicotinic acid (1.0 g, 5.0 mmol) and 1-(N-t-butoxycarbonylamino)-2-aminobenzene (Method 6; 1.0 g, 5.0 mmol) were dissolved in DMF (10 ml), 4-(4,6-dimethoxy-1,3,5-triazinyl-2-yl)-4-methylmorpholinium chloride (Method 7; 1.7 g, 6.0 mmol) was added and the resulting solution was stirred for 18 hours at ambient temperature. The solution was poured into water (50 ml) and extracted with ethyl acetate (3 x 50 ml). The combined organic extracts were dried over magnesium sulfate and evaporated. The residue was purified by trituration with diethyl ether (20 ml) and the resulting solid was collected by filtration. The solid was washed with diethyl ether (20 ml) and dried *in vacuo* to afford the title compound (1.5 g, 77 %); NMR Spectrum: (CDCl₃) 1.45 (s, 9H), 7.12 (t, 1H), 7.20 (t, 1H), 7.47 (d, 1H), 7.61 (d, 1H), 7.83 (d, 1H), 8.21 (d, 1H), 8.63 (s, 1H), 8.91 (s, 1H), 9.97 (s, 1H); Mass Spectrum: M-H 390 and 392.

### Method 6

### 1-(N-t-Butoxycarbonylamino)-2-aminobenzene

The title compound was prepared according to the literature method described in Seto, C, T.; Mathias, J. P.; Whitesides, G. M.; *J. Am. Chem. Soc*., **1993,** *115*, 1321-1329.

### Method 7

### 4-(4,6-Dimethoxy-1,3,5-triazinyl-2-yl)-4-methylmorpholinium chloride

4-(4,6-Dimethoxy-1,3,5-triazinyl-2-yl)-4-methylmorpholinium chloride was prepared according to the literature procedure described in Kunishima, M., Kawachi, C., Morita, J., Terao, K., Iwasaki, F., Tani, S., *Tetrahedron*, **1999,** *55*, 13159-13170.

### Method 8

### N2-(2-t-Butoxycarbonylaminophenyl)-5-(pyridin-2-yl)thiophene-2-carboxamide

5-(Pyridin-2-yl)thiophene-2-carboxylic acid (205 mg, 1.0 mmol) and 1-(N-t-butoxycarbonylamino)-2-aminobenzene (Method 6; 229 mg, 1.1 mmol) were stirred together in *N*,*N*-dimethylacetamide (5ml) for 10 minutes, 4-(4,6-dimethoxy-1,3,5-triazinyl-2-yl)-4-methylmorpholinium chloride (Method 7; 332 mg, 1.2 mmol) was added and the mixture stirred at ambient temperature for 24 hours. The solvent was evaporated and the residue partitioned between ethyl acetate and water. The organic extracts were dried over magnesium sulfate , filtered and evaporated and the resultant residue purified by flash chromatography eluting with 0-5% methanol/dichloromethane to give the title compound (193 mg, 49 %); NMR Spectrum: (DMSO-d₆) 1.44 (s, 9H), 7.16 (m, 2H), 7.34 (m, 1H), 7.52 (m, 2H), 7.88 (m, 3H), 8.00 (d, 1H), 8.57 (d, 1H), 8.65 (s, 1H), 9.83 (s, 1H); Mass Spectrum:( M+H⁺-Boc ) 296.

### Method 9

### N-(2-t-Butoxycarbonylaminophenyl)-3,3'-bipyridine-6-carboxamide

2-(N-t-Butoxycarbonylamino)phenyl-5-bromopicolinamide (Method 40; 76 mg, 0.19 mmol), 3-pyridine boronic acid (29 mg, 0.23 mmol), *tetrakis*(triphenylphosphine) palladium (40 mg, 0.03 mmol), sodium carbonate (20 mg, 0019 mmol) and ethanol (3 ml) were heated to 150°C for 10 minutes in the microwave. The cooled mixture was concentrated and partitioned between ethyl acetate and water. The organics were washed with brine, dried over magnesium sulfate and evaporated. The resultant residue was purified by flash chromatography eluting with ethyl acetate/iso-hexane (25-75%) to give the title compound (45 mg, 60%); Mass Spectrum: M+H⁺ 391.

### Method 10

### N-(2-t-Butoxycarbonylaminophenyl)-2,3'-bipyridine-5-carboxamide

N-(2-t-Butoxycarbonylaminophenyl)-6-chloronicotinamide (Method 14; 123 mg, 0.35 mmol), 3-pyridine boronic acid trimer (Method 11; 37 mg, 0.12 mmol), *tetrakis*(triphenylphosphine) palladium (74 mg, 0.06 mmol), 1,2-dimethoxyethane (2 ml) and a saturated aqueous solution of sodium hydrogen carbonate (2 ml) were stirred at 80°C under an atmosphere of argon for 2.5 hours. The cooled mixture was diluted with ethyl acetate and water, then the aqueous layer was removed by passage through a diatomaceous earth column. The organics were evaporated and the resultant residue was purified by flash chromatography eluting with methanol/dichloromethane (0-10%) to give the title compound (145 mg, 106%); Mass Spectrum: M+H⁺ 391.

### Method 11

### 3-Pyridine Boronic acid trimer

3-Pyridine Boronic acid trimer was prepared according to the method of W. Li *et al., J. Org. Chem*. **2002,** *67,* 5394.

### Method 12

### N-(2-t-Butoxycarbonylaminophenyl)-6-(3-furyl)nicotinamide

N-(2-t-Butoxycarbonylaminophenyl)-6-chloronicotinamide (Method 14; 174 mg, 0.5 mmol), 3-furan boronic acid (56 mg, 0.5 mmol), *tetrakis*(triphenylphosphine) palladium (104 mg, 0.09 mmol), 1,2-dimethoxyethane (2.5 ml) and a saturated aqueous solution of sodium hydrogen carbonate (2.5 ml) were stirred at 80°C, under an atmosphere of argon, for 1.5 hours. The cooled mixture was concentrated and stirred vigorously with ethyl acetate and water, then the aqueous layer was removed by passage through a diatomaceous earth column. The organics were evaporated to give the title compound which was used without purification; Mass Spectrum: M+H⁺ 380.

### Method 13

### t-Butyl 5-[({2-[(t-butoxycarbonyl)amino]phenyl}amino)carbonyl]-3',6'-dihydro-2,4'-bipyridine-1'(2'H)-carboxylate

N-(2-t-Butoxycarbonylaminophenyl)-6-chloronicotinamide (Method 14; 174 mg, 0.5 mmol), N-(t-butoxycarbonyl-3,4-dehydropiperidinyl)-4-pinacolatoboron (Method 38; 155 mg, 0.5 mmol), *tetrakis*(triphenylphosphine) palladium (104 mg, 0.09 mmol), 1,2-dimethoxyethane (2.5 ml) and a saturated aqueous solution of sodium hydrogen carbonate (2.5 ml) were stirred at 80°C, under an atmosphere of argon, for 36 hours. The cooled mixture was stirred vigorously in ethyl acetate and water. The aqueous layer was removed by passage through a diatomaceous earth column. The organics were evaporated and the resultant residue was purified by flash chromatography eluting with ethyl acetate/iso-hexane (25-75%) to give the title compound (171 mg, 69%); NMR Spectrum: (DMSO-d₆) 1.44 (s, 18H), 2.62 (m, 2H), 3.58 (t, 2H), 4.10 (m, 2H), 6.86 (m, 1H), 7.11 (t, 1H), 7.20 (t, 1H), 7.51 (d, 1H), 7.59 (d, 1H), 7.71 (d, 1H), 8.27 (d, 1H), 8.61 (s, 1H), 9.07 (s, 1H), 9.90 (s, 1H); Mass Spectrum: M+H⁺ 495.

### Method 14

### N-(2-t-Butoxycarbonylaminophenyl)-6-chloronicotinamide

2-Chloro-4,6-dimethoxy-1,3,5-triazine (10.9 g, 62.2 mmol) was dissolved in N,N-dimethylformamide (105 ml) and cooled to 0°C. N-methylmorpholine (6.8 ml, 62.2 mmol) was added slowly as to maintain the temperature below 10°C. A solution of 6-chloronicotinic acid (7.0 g, 44.4 mmol) and 1-(N-t-butyloxycarbonylamino)-2-aminobenzene (Method 6; 9.2 g, 44.4 mmol) in N,N-dimethylformamide (105 ml) was added *via* a cannula, maintaining the temperature below 10°C. The mixture was stirred at 0-5°C for 2 hours. The mixture was then concentrated and the residue triturated with ether and filtered. The resultant solution was concentrated to give the title compound (16.93g, 100%); NMR Spectrum: (DMSO-d₆) 1.45 (s, 9H), 7.12 (t, 1H), 7.21 (t, 1H), 7.50 (d, 1H), 7.62 (d, 1H), 7.72 (d, 1H), 8.33 (d, 1H), 8.63 (s, 1H), 8.95 (s, 1H), 9.96 (s, 1H); Mass Spectrum: M+H⁺-t-Bu 292.

### Method 15

### t-Butyl 4-{5-[({2-[(t-butoxycarbonyl)amino]phenyl}amino)carbonyl]pyrazin-2-yl}piperazine-1-carboxylate

A solution of N-(2-t-Butoxycarbonylaminophenyl)-5-chloropyrazine-2-carboxamide (Method 17, 177 mg, 0.51 mmol) and t-butyl-1-piperazinecarboxylate (240 mg, 1.29 mmol) in N,N-dimethylacetamide (3 ml) was heated to 80°C for 2 hours. The reaction mixture was then allowed to cool before being poured onto water (60 ml). The resultant precipitate was collected by.suction filtration, washed with water and diethyl ether and dried *in vacuo* to give the title compound (206 mg, 81%); NMR Spectrum: (DMSO-d₆) 1.43 (s, 9H), 1.49 (s, 9H), 3.49 (m, 4H), 3.78 (m, 4H), 7.15 (t, 1H), 7.24 (m, 2H), 7.95 (d, 1H), 8.25 (s, 1H), 8.74 (s, 1H), 8.99 (s, 1H), 9.98 (s, 1H); Mass Spectrum: M+H⁺ -Boc 399.

### Method 16

### t-Butyl [2-({[5-(4-benzylpiperazin-1-yl)pyrazin-2-yl]carbonyl}amino)phenyl]carbamate

A solution of N-(2-t-Butoxycarbonylaminophenyl)-5-chloropyrazine-2-carboxamide (Method 17, 75 mg, 0.22 mmol) and 1-benzylpiperazine (100 µl, 0.58 mmol) in N,N-dimethylacetamide (3 ml) was heated to 80°C for 2 hours. Reaction mixture was then allowed to cool and water was then added. The resultant precipitate was collected by suction filtration, washed with water and isohexane/diethyl ether, and dried under a stream of air to give the title compound (75mg, 70%); NMR Spectrum: (DMSO-d₆) 1.48 (s, 9H), 2.52 (m, 4H), 3.55 (s, 2H), 3.75 (m, 4H), 7.13 (t, 1H), 7.22 (t, 1H), 7.27 (m, 2H), 7.35 (m, 4H), 7.96 (d, 1H), 8.24 (s, 1H), 8.72 (s, 1H), 8.98 (s, 1H), 9.96 (s, 1H); Mass Spectrum: M+H⁺ 489.

### Method 17

### N-(2-t-Butoxycarbonylaminophenyl)-5-chloropyrazine-2-carboxamide

To a suspension of 5-hydroxypyrazine-2-carboxylic acid (2.0 g, 14.3 mmol) in dichloromethane (100 ml) was added N,N-dimethylformamide (10 drops) and thionyl chloride (5.5 ml, 75.4 mmol). The reaction mixture was heated to reflux for 3.5 hours before being allowed to cool and evaporated to dryness. The residue was azeotroped with toluene and dried *in vacuo.* The resultant solid was then redissolved in dichloromethane (50 ml) and treated with di-isopropylethylamine (7.5 ml, 43.1 mmol). The reaction mixture was stirred for 5 minutes before addition of a solution of 1-(N-t-butyloxycarbonylamino)-2-aminobenzene (Method 6; 2.69 g, 12.9 mmol) in dichloromethane (50 ml) and stirring continued at ambient temperature for 16 hours. The reaction was then carefully poured onto a saturated aqueous solution of sodium bicarbonate (120 ml). The organic layer was separated, washed with water and brine, dried over magnesium sulfate and evaporated to dryness. The resultant residue was triturated with diethyl ether, the solid collected by filtration and dried under a stream of air to give the title compound (2.51g, 56%); NMR Spectrum: (DMSO-d₆) 1.49 (s, 9H), 7.23 (m, 2H), 7.34 (d, 1H), 7.86 (d, 1H), 8.86 (s, 1H), 9.10 (S, 1H), 9.13 (s, 1H), 10.32 (s, 1H); Mass Spectrum: M+H⁺ -Boc 249.

### Method 18

### t-Butyl-4-{5-[({2-[(t-butoxycarbonyl)amino]phenyl}amino)carbonyl]pyrimidin-2-yl}piperazine-1-carboxylate

A solution of N-(2-t-Butoxycarbonylaminophenyl)-2-(methylsulfonyl)pyrimidine-5-carboxamide (Method 19, 400 mg, 1.02 mmol) and *t*-butyl-1-piperazinecarboxylate (475 mg, 2.55 mmol) in *N,N*-dimethylacetamide (15 ml) was heated to 80°C for 90 minutes. The reaction mixture was then allowed to cool and the solvent removed *in vacuo*. The resultant residue was purified by flash column chromatography, on silica, eluting with ethyl acetate/isohexane (25-75%), to give the title compound (291 mg, 57%); NMR Spectrum: (DMSO-d₆) 1.44 (s, 9H), 1.45 (s, 9H), 3.44 (m, 4H), 3.85 (m, 4H), 7.12 (t, 1H), 7.20 (t, 1H), 7.48 (d, 1H), 7.61 (d, 1H), 8.54 (s, 1H), 8.90 (s, 2H), 9.70 (s, 1H); Mass Spectrum: M-O^{t}Bu 423.

### Method 19

### N-(2-t-Butoxycarbonylaminophenyl)-2-(methylsulfonyl)pyrimidine-5-carboxamide

To a cooled (0°C) solution of N-(2-t-Butoxycarbonylaminophenyl)-2-(methylthio)pyrimidine-5-carboxamide (method 20; 749 mg, 2.08 mmol) in N,N-dimethylformamide (50 ml) was added *meta*-chloroperbenzoic acid (70%, 1.11 g, 4.50 mmol). The reaction mixture was allowed to warm to room temperature and stirred, under argon, for 66 hours. A further portion of *meta*-chloroperbenzoic acid (70%, 600 mg, 2.43 mmol) was then added and stirring continued for a further 5 hours. The solvent was then removed *in vacuo* and the residue partitioned between ethyl acetate and a 0.25M aqueous solution of sodium metabisulfite. The organic phase was separated, washed with water and brine, dried over magnesium sulfate, filtered and evaporated to dryness. The residue was taken up in dichloromethane and all insoluble material removed by filtration. The filtrate was evaporated, redissolved in an ethyl acetate/methanol mixture and washed. The solution was washed with a saturated aqueous solution of sodium bicarbonate, water and brine, before drying over magnesium sulfate, filtered and evaporated to give the title compound (505 mg, 62%); NMR Spectrum: (DMSO-d₆) 1.46 (s, 9H), 3.49 (s, 3H), 7.14 (t, 1H), 7.25 (t, 1H), 7.52 (d, 1H), 7.72 (d, 1H), 8.71 (s, 1H), 9.50 (s, 2H), 10.24 (s, 1H); Mass Spectrum: M+H⁺-t-Bu 337.

### Method 20

### N-(2-t-Butoxycarbonylaminophenyl)-2-(methylthio)pyrimidine-5-carboxamide

A mixture of 2-methylsulfanylpyrimidine-5-carboxylic acid (1.0 g, 5.88 mmol), 1-(N-t-butoxycarbonylamino)-2-aminobenzene (Method 6; 1.23 g, 5.91 mmol) and 4-(4,6-dimethoxy-1,3,5-triazinyl-2-yl)-4-methylmorpholinium chloride (Method 7; 2.2 g, 7.95 mmol) in *N*,*N*-dimethylformamide (30 ml) was allowed to stir at room temperature for 4 hours before being partitioned between ethyl acetate and water. The organic layer was separated, washed with water and brine, dried over magnesium sulfate, filtered and evaporated to dryness. The residue was purified by flash column chromatography, on silica, eluting with ethyl acetate in isohexane (25-75%), to give the title compound (1.69 g, 80%); NMR Spectrum: (DMSO-d₆) 1.45 (s, 9H), 2.60 (s, 3H), 7.13 (t, 1H), 7.22 (t, 1H), 7.50 (d, 1H), 7.66 (d, 1H), 8.64 (s, 1H), 9.10 (s, 2H), 9.96 (s, 1H); Mass Spectrum: M+H⁺ 361.

### Method 21

### N-(2-t-Butoxycarbonylaminophenyl)-5-(pyrrolidin-1-yl)thiophene-2-carboxamide

4-(4,6-Dimethoxy-1,3,5-triazinyl-2-yl)-4-methylmorpholinium chloride (Method 7; 382 mg, 1.38 mmol) was added to a solution of 5-pyrrolidin-1-ylthiophene-2-carboxylic acid (Method 22; 227 mg, 1.15 mmol) and 1-(N-t-butoxycarbonylamino)-2-aminobenzene (Method 6; 263 mg, 1.27 mmol) in N,N-dimethylacetamide (8 ml). The reaction mixture was stirred at ambient temperature under nitrogen for 20 hours. The solvent was evaporated and the residue partitioned between ethyl acetate and water. The organic extracts were dried over sodium sulfate, filtered and evaporated. The resultant residue was purified by flash chromatography eluting with ethyl acetate/isohexane (20-30%) followed by ethyl acetate/dichloromethane (5%) to give the title compound (65 mg, 15%); NMR Spectrum: (CDCl₃) 1.53 (s, 9H), 2.07 (m, 4H), 3.38 (m, 4H), 5.72 (d, 1H), 6.92 (s, 1H), 7.15 (m, 2H), 7.34 (m, 1H), 7.40 (d, 1H), 7.64 (m, 1H), 8.33 (m, 1H); Mass Spectrum: M+H⁺ 388.

### Method 22

### 5-(pyrrolidin-1-yl)thiophene-2-carboxylic acid

0.5M Aqueous lithium hydroxide (5.6 ml, 2.80 mmol) was added to a solution of ethyl 5-pyrrolidin-1-ylthiophene-2-carboxylate (Method 23; 450 mg, 2.00 mmol) in methanol (16 ml) and the reaction stirred at 80°C under nitrogen for 24 hours. The methanol was evaporated and the aqueous solution was washed with ether. The aqueous solution was acidified to pH 5 with 2M hydrochloric acid and extracted with ethyl acetate containing a small quantity of methanol. The ethyl acetate extracts were dried over sodium sulfate and evaporated to give the title compound (262 mg, 66%); NMR Spectrum: (DMSO-d₆) 1.99 (m, 4H), 3.26 (m, 4H), 5.78 (d, 1H), 7.41 (d, 1H); Mass Spectrum: M+H⁺ 198.

### Method 23

### Ethyl-5-(pyrrolidin-1-yl)thiophene-2-carboxylate

Cesium carbonate (1.94 g, 5.95 mmol), *tris*(dibenzylideneacetone)dipalladium(0) (194 mg, 0.212 mmol) and racemic-2,2'-bis(diphenylphosphino)-1,1-binaphthyl (396 mg, 0.636 mmol) were added to ethyl 5-bromothiophene-2-carboxylate (1.00 g, 4.25 mmol) followed by toluene (43 ml) and pyrrolidine (0.43ml, 5.10 mmol). The reaction mixture was degassed, and stirred under nitrogen at 80°C for 28 hours. The cooled mixture was partitioned between ethyl acetate and water. The organics were washed with brine, dried over sodium sulfate and evaporated. The resultant residue was purified by flash chromatography eluting with dichloromethane to give the title compound (880 mg, 92%); NMR Spectrum: (CDCl₃) 1.34 (t, 3H), 2.07 (m, 4H), 3.32 (m, 4H), 4.28 (q, 2H), 5.73 (d, 1H), 7.58 (d, 1H); Mass Spectrum: M+H⁺ 226.

### Method 24

### N-(2-t-Butoxycarbonylaminophenyl)-5-(piperidin-1-yl)thiophene-2-carboxamide

Using a procedure analogous to that described in Method 21, 5-piperidin-1-ylthiophene-2-carboxylic acid (Method 25) was reacted with 1-(N-t-butyloxycarbonylamino)-2-aminobenzene to give the title compound (26%); NMR Spectrum: (CDCl₃) 1.53 (s, 9H), 1.63 (m, 2H), 1.73 (m, 4H), 3.26 (m, 4H), 6.00 (d, 1H), 6.88 (s, 1H), 7.15 (m, 2H), 7.32 (m 1H), 7.40 (d, 1H), 7.66 (m, 1H), 8.46 (s, 1H); Mass Spectrum: M+H⁺ 402.

### Method 25

### 5-(piperidin-1-yl)thiophene-2-carboxylic acid

Using a procedure analogous to that described in Method 22, ethyl-5-(piperidin-1-yl)thiophene-2-carboxylate (Method 26) was reacted with lithium hydroxide to give the title compound (73%); NMR Spectrum: (DMSO-d₆) 1.58 (m, 6H), 3.20 (m, 4H), 6.12 (d, 1H), 7.40 (d, 1H); Mass Spectrum: M+H⁺ 212.

### Method 26

### Ethyl-5-(piperidin-1-yl)thiophene-2-carboxylate

Cesium carbonate (1.94 g, 5.95 mmol), *tris*(dibenzylideneacetone)dipalladium(0) (194 mg, 0.212 mmol) and racemic-2,2'-bis(diphenylphosphino)-1,1-binaphthyl (396 mg, 0.636 mmol) were added to ethyl 5-bromothiophene-2-carboxylate (1.00 g, 4.25 mmol) followed by toluene (43 ml) and piperidine (0.51 ml, 5.1 mmol). The reaction mixture was degassed, and stirred under nitrogen at 80°C for 28 hours. The cooled mixture was partitioned between ethyl acetate and water. The organics were washed with brine, dried over sodium sulfate and evaporated. The resultant residue was purified by flash chromatography eluting with ethyl acetate/isohexane (10-15%) followed by dichloromethane to give the title compound (488 mg, 48%); NMR Spectrum: (CDCl₃) 1.34 (t, 3H), 1.63 (m, 2H), 1.72 (m, 4H), 3.26 (m, 4H), 4.28 (q, 2H), 5.99 (d, 1H), 7.54 (d, 1H); Mass Spectrum: M+H⁺ 240.

### Method 27

### t-Butyl 4-(5-{[(2-t-butoxycarbonylaminophenyl)amino]carbonyl}-2-thienyl)piperidine-1-carboxylate

To a solution of t-butyl 4-(5-{[(2-t-butoxycarbonylaminophenyl)amino]carbonyl}-2-thienyl)-3,6-dihydropyridine-1(2*H*)-carboxylate (Method 28; 133 mg, 0.27 mmol) in absolute ethanol (25 ml) was added palladium on charcoal (10%, 133 mg) and the mixture was stirred under an atmosphere of hydrogen for 24 hours. The mixture was filtered and evaporated to afford the title compound (130 mg, 96%); NMR Spectrum: (CDCl₃). 1.48 (s, 9H), 1.53 (s, 9H), 1.66 (m, 2H), 2.00 (m, 2H), 2.84 (m, 2H), 2.98 (m, 1H), 4.20 (m, 2H), 6.74 (s, 1H), 6.82 (d, 1H), 7.20 (m, 2H), 7.56 (m, 3H), 8.99 (s, 1H); Mass Spectrum: M+H⁺ - BOC 402.

### Method 28

### t-Butyl 4-(5-{[(2-t-butoxycarbonylaminophenyl)amino]carbonyl}-2-thienyl)-3,6-dihydropyridine-1(2H)-carboxylate

A saturated solution of sodium hydrogencarbonate (3 ml) was added to a stirred solution of N-(2-t-butoxycarbonylaminophenyl)-5-bromothiophene-2-carboxamide (Method 3,123 mg, 0.31 mmol) in 1,2-dimethoxyethane (3 ml). N-(t-Butoxycarbonyl-3,4-dehydropiperidinyl)-4-pinacolatoboron (Method 38; 96 mg, 0.31 mmol) was added followed by *tetrakis*triphenylphosphine palladium (48 mg, 0.04 mmol) and the mixture stirred at 80°C for 21 hours. The cooled mixture was partitioned between ethyl acetate and water. The organic phase was separated, washed with water, dried over magnesium sulfate, filtered and evaporated. The resultant residue was purified by flash column chromatography, eluting with methanol/dichloromethane (0 -10%) to give the title compound (133mg, 86%); NMR Spectrum: (CDCl₃) 1.49 (s, 9H), 1.53 (s, 9H), 2.53 (s, 2H), 3.64 (t, 2H), 4.08 (s, 2H), 6.19 (s, 1H), 6.84 (s, 1H), 6.97 (d, 1H), 7.17 (m, 2H), 7.54 (m, 3H), 9.18 (s, 1H); Mass Spectrum: M+H⁺-Boc 400.

### Method 29

### t-Butyl-4-{5-[({2-[(t-butoxycarbonyl)amino]phenyl}amino)carbonyl]thien-2-yl}piperazine-1-carboxylate

A stirred solution of 5-[4-(t-butoxycarbonyl)piperazin-1-yl]thiophene-2-carboxylic acid (Method 30; 312 mg, 1.00 mmol) in dichloromethane (2.5 ml) containing N,N-dimethylformamide (5 µl) was treated with oxalyl chloride (87 µl, 1.0 mmol) and the mixture was stirred at ambient temperature for one hour. To this was added in one portion a solution of 1-(N-t-butyloxycarbonylamino)-2-aminobenzene (Method 6; 208 mg, 1.00 mmol) and triethylamine (0.38 ml, 2.76 mmol) in dichloromethane (2 ml). The mixture was stirred for one hour and then evaporated to dryness. The resultant dark green gum was purified by flash chromatography eluting with a gradient of ethyl acetate in dichloromethane (0-20%) to give the title compound (150 mg, 30%); Mass Spectrum: M+H⁺ 503.

### Method 30

### 5-[4-(t-Butoxycarbonyl)piperazin-1-yl]thiophene-2-carboxylic acid

A solution of t-butyl 4-(5-formylthien-2-yl)piperazine-1-carboxylate (Method 31; 2.51 g, 8.50 mmol) in ethanol (85 ml) was added in one portion to a solution of silver (I) nitrate (10.0 g, 58.8 mmol) and sodium hydroxide (4.83 g, 120.6 mmol) in water (85 ml). This mixture was stirred and heated at 65°C for 22 hours. The mixture was cooled by the addition of ice and then filtered to remove silver salts. The filtrate was carefully evaporated to remove the ethanol and the resulting aqueous solution was filtered again through a glass-fibre pad to remove tarry material. The filtrate was then diluted with water to a total volume of 400 ml and then acidified to pH 5 with acetic acid. The precipitate was filtered off, washed with water and then dried in a vacuum oven at 45°C to give the title compound (1.88 g, 71%); NMR Spectrum: (DMSO-d₆) 1.41 (s, 9H), 3.18 (m, 4H), 3.45 (m, 4H), 6.20 (d, 1H), 7.43 (d, 1H); Mass Spectrum: M+H⁺ 313.

### Method 31

### t-Butyl 4-(5-formylthien-2-yl)piperazine-1-carboxylate

A mixture of 5-bromothiophene-2-carboxaldehyde (3.82 g, 20.0 mmol), t-butyl piperazine-1-carboxylate (4.1 g, 22.0 mmol), diisopropylethylamine (7.0 ml, 40.0 mmol) and dimethylsulfoxide (5.0 ml) were stirred at 130°C under an atmosphere of nitrogen for 18 hours. The cooled mixture was partitioned between ethyl acetate and water. The organics were washed with water, brine, dried over magnesium sulfate and evaporated. The resultant solid was purified by flash chromatography eluting with dichloromethane followed by ethyl acetate/dichloromethane (15%) to give the title compound (4.3 g, 73%); NMR Spectrum: (DMSO-d₆) 1.41 (s, 9H), 3.34 (m, 4H), 3.47 (m, 4H), 6.36 (d, 1H), 7.70 (d, 1H), 9.49 (s, 1H); Mass Spectrum: M+H⁺ 297.

### Method 32

### N-(2-Nitrophenyl)-2-(4-methylpiperazin-1-yl)-1,3-thiazole-5-carboxamide

A 1.6M solution of n-butyllithium in hexane (0.7 ml, 1.1 mmol) was added to a stirred solution of 1-(5-bromo-1,3-thiazol-2-yl)-4-methylpiperazine (Method 33; 262 mg, 1.0 mmol) in diethyl ether (5 ml) at -78°C. After 15 minutes a solution of 2-nitrophenylisocyanate (164 mg, 1.0 mmol) in diethylether (5 ml) was added and the mixture warmed to ambient temperature over 4 hours. Saturated aqueous ammonium chloride solution (5 ml) was added and the solution extracted with ethyl acetate. The combined organic extracts were washed with brine, dried over magnesium sulfate, filtered and evaporated. The resultant residue was purified by flash chromatography eluting with methanol/dichloromethane (0-10%) to give the title compound (197 mg, 57%); NMR Spectrum: (CDCl₃) 2.37 (s, 3H), 2.55 (t, 4H), 3.64 (t, 4H), 7.17 (t, 1H), 7.66 (t, 1H), 7.89 (s, 1H), 8.26 (d, 1H), 8.86 (d, 1H), 11.03 (s, 1H); Mass Spectrum: M+H⁺ 348.5

### Method 33

### 1-(5-bromo-1,3-thiazol-2-yl)-4-methylpiperazine

2,5-dibromothiazole (1.0 g, 4.12 mmol), N-methylpiperazine (4.6 ml, 41.2 mmol) and 4-(N,N-dimethylamino)pyridine (49 mg, 0.4 mmol) were refluxed together in n-butanol (20 ml) for 2 hours. The cooled solution was evaporated and the residue purified by flash chromatography eluting with methanol/dichloromethane (0-10%) to give the title compound (704 mg, 65%); NMR Spectrum: (CDCl₃) 2.34 (s, 3H), 2.50 (t, 4H), 3.45 (t, 4H), 7.06 (s, 1H); Mass Spectrum: M+H⁺ 264.

### Method 34

### N-(2-Aminophenyl)-2-piperazin-1-yl-1,3-thiazole-5-carboxamide

t-Butyl 4-(5- {[(2-aminophenyl)amino]carbonyl}-1,3-thiazol-2-yl)piperazine-1-carboxylate (Method 35; 239 mg, 0.59 mmol), 1,4-dioxane (2.3 ml) and a 4M solution of hydrochloric acid in 1,4-dioxane (2.3 ml) were stirred at ambient temperature for 6 hours. The resultant precipitate was filtered and washed with diethyl ether. The solid was dissolved in water (10 ml), basified with 2N NaOH and extracted with ethyl acetate. The ethyl acetate extracts were washed with brine, dried over magnesium sulfate, filtered and evaporated to give the title compound (118 mg, 66%); NMR Spectrum: (DMSO-d₆) 2.79 (t, 4H), 3.40 (t, 4H), 4.85 (s, 2H), 6.58 (t, 1H), 6.76 (d, 1H), 6.95 (t, 1H), 7.10 (d, 1H), 8.01 (s, 1H), 9.40 (s, 1H); Mass Spectrum: M+H⁺ 304.

### Method 35

### t-Butyl 4-(5-{[(2-aminophenyl)amino]carbonyl}-1,3-thiazol-2-yl)piperazine-1-carboxylate

Nickel (II) acetate (3.04 g, 12.2 mmol) was added to a stirred suspension of t-butyl 4-(5-{[(2-nitrophenyl)amino]carbonyl}-1,3-thiazol-2-yl)piperazine-1-carboxylate (Method 36; 2.64 g, 6.1 mmol) in methanol (200 ml) at 0°C. Sodium borohydride (2.31 g, 61 mmol) was added over 15 minutes and the resulting mixture stirred at ambient temperature for 2 hours. The mixture was filtered, the solvent evaporated and the residue purified by flash chromatography eluting with methanol/dichloromethane (0-20%) to give the title compound (1.81g, 74%); NMR Spectrum: (CDCl₃) 1.49 (s, 9H), 3.56 (s, 8H), 3.85 (s, 2H), 6.82 (m, 2H), 7.09 (t, 1H), 7.28 (m, 1H), 7.35 (s, 1H), 7.68 (s, 1H); Mass Spectrum: M+H⁺,404.

### Method 36

### t-Butyl 4-(5-{[(2-nitrophenyl)amino]carbonyl}-1,3-thiazol-2-yl)piperazine-1-carboxylate

A 2.5M solution of n-butyllithium in hexane (1.7 ml, 4.25 mmol) was added to a stirred solution of t-butyl 4-(5-bromo-1,3-thiazol-2-yl)piperazine-1-carboxylate (Method 37; 1.32 g, 3.79 mmol) in diethyl ether (25 ml) at -78°C. After 15 minutes a solution of 2-nitrophenylisocyanate (0.62 g, 3.79 mmol) in diethyl ether (10 ml) was added and the mixture warmed to ambient temperature over 4 hours. Saturated aqueous ammonium chloride solution (25 ml) was added and the solution extracted with ethyl acetate. The organic extract was washed with brine, dried over magnesium sulfate, filtered and evaporated. The resultant residue was purified by flash chromatography eluting with ethyl acetate/isohexane (0-33%) to give the title compound (451 mg, 28%); NMR Spectrum: (CDCl₃) 1.49 (s, 9H), 3.60 (s, 8H), 7.17 (t, 1H), 7.66 (t, 1H), 7.88 (s, 1H), 8.26 (d, 1H), 8.86 (d, 1H), 11.04 (s, 1H); Mass Spectrum: M+H⁺ 434.

### Method 37

### t-Butyl 4-(5-bromo-1,3-thiazol-2-yl)piperazine-1-carboxylate

2,5-Dibromothiazole (1.0 g, 4.12 mmol), 1-t-butyloxycarbonylpiperazine (1.92 g, 10.3 mmol) and triethylamine (5.7 ml, 41.2 mmol) were refluxed together in n-butanol (50 ml) for 36 hours. The cooled solution was evaporated and the residue purified by flash chromatography eluting with methanol/dichloromethane (0-10%) to give the title compound (1.32 g, 92%); NMR Spectrum: (CDCl₃) 1.48 (s, 9H), 3.40 (t, 4H), 3.55 (t, 4H), 7.08 (s, 1H); Mass Spectrum: M+H⁺ 350.

### Method 38

### N-(t-Butoxycarbonyl-3,4-dehydropiperidinyl)-4-pinacolatoboron

(*Tet Lett.* **2000,** *41*, 3705)
1,1'-Bis(diphenylphosphino) ferrocenedichloropalladium (II) (1.2 g; 1.5 mmol) was added to a solution of potassium acetate (13.3 g; 136 mmol) and bis-pinacolato diboron (13.8 g; 54.3 mmol) in N,N-dimethylformamide (150 ml). (N-t-butoxycarbonyl-4-trifluoromethanesulfonyloxy-3,4-dehydropiperidinyl)-4-pinacolatoboron [CAS 138647-49-1]; 15.0 g. 45.3 mmol) in N,N-dimethylformamide (100 ml) was added slowly before the mixture was heated to 80°C for 18 hours under argon. The cooled mixture was concentrated and partitioned between ethyl acetate and water. The organics were washed with brine, dried over magnesium sulfate, filtered and evaporated. The concentrated residue was filtered through a pad of silica eluting with (50%) ethyl acetate/iso-hexane (50%) to give the crude title compound (14.6 g, 100%); NMR Spectrum: (DMSO-d₆) 1.21 (s, 12H), 1.40 (s, 9H), 2.10 (m, 2H), 3.34 (t, 2H), 3.76 (m, 2H), 6.39 (m, 1H).

### Method 39

### 1-Bromoacetyl-1,2,3,4-tetrahydroquinoline [CAS 63286-44-2]

1,2,3,4-Tetrahydroquinoline (10 g, 75 mmol) was dissolved in benzene (40 ml) and cooled to 10 °C. A solution of bromoacetyl bromide (16 g, 80 mmol) in benzene (40 ml) was added dropwise over 1 hour. The mixture was stirred for a further 15 minutes. Sodium hydroxide solution (2M, 500 ml) was added. The organic layer was separated, washed with water (100 ml), dried over magnesium sulfate, filtered then evaporated to afford the crude product. This was purified by distillation under reduced pressure followed by recrystallisation from 60 - 80 petroleum ether to afford the title compound (12.5 g, 66%). Anal. calc for C₁₁H₁₂ONBr gives C 52.0%, H 4.8%, N 5.5%, Br 31.4%; found C 51.9%, 4.8%, N 5.6%, Br 30.9%.

### Method 40

### 2-(N-t-butoxycarbonylamino)phenyl-5-bromonicotinamide

To a solution of 5-bromonicotinic acid (2.0 g, 10 mmol) and 1-(N-t-butyloxycarbonylamino)-2-aminobenzene (2.1 g, 10 mmol) in N,N-dimethylformamide (20 ml) was added 4,6-dimethoxy-1,3,5-triazin-2-yl)-4-methylmorpholinium chloride (Method 7; 85%, 3.8 g, 12 mmol) and the mixture stirred at ambient temperature for 24 hours. The mixture was concentrated and partitioned between water and ethyl acetate. The organic layer was separated, dried over magnesium sulfate and evaporated. The resulting solid residue was triturated with diethyl ether and the solid collected by filtration and dried in vacuo to give the title compound (3.0 g, 76%); Mass Spectrum: M+H⁺-t-Bu 336.

### Method 41

### N-(2-t-Butoxycarbonylaminophenyl)-6-(4-benzylpiperazin-1-yl)nicotinamide

N-(2-t-Butoxycarbonylaminophenyl)-6-chloronicotinamide (Method 14; 100 mg, 0.29 mmol) was heated to 80°C with N-benzyl piperazine (0.15 ml, 0.87 mmol) in DMA (5 ml) for 24 hours. The cooled mixture was partitioned between ethyl acetate and water. The organics were washed with brine, dried over magnesium sulfate and evaporated. The resultant residue was purified by flash chromatography eluting with ethyl acetate/iso-hexane (25-75%) to give the title compound (70 mg, 50%); Mass Spectrum: M+H⁺ 488.

### Method 42

### N-(2-t-Butoxycarbonylaminophenyl)-6-piperazin-1-ylnicotinamide

N-(2-t-Butoxycarbonylaminophenyl)-6-chloronicotinamide (Method 14; 1.8 g, 5.17 mmol) was heated to 80°C with N-boc-piperazine (2.9 g, 15.5 mmol) in DMA (100 ml) for 72 hours. The cooled mixture was partitioned between ethyl acetate and water. The organics were washed with brine, dried over magnesium sulfate, filtered and evaporated. The resultant residue was purified by flash chromatography eluting with ethyl acetate/iso-hexane (25-75%) to give the title compound (1.13 g, 44%); Mass Spectrum: M+H⁺ 442.

## Claims

1. A. compound of the formula **(I)**: 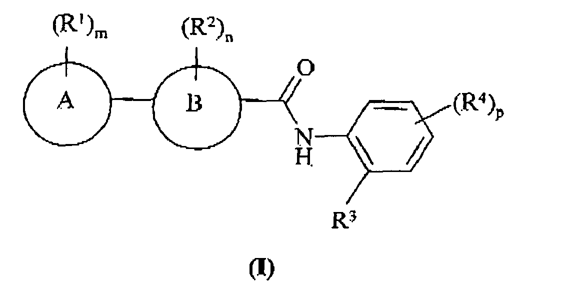 wherein:
Ring A is a pyridyl, quinolyl, indolyl, pyrimidinyl, morpholinyl, piperidinyl, piperazinyl, pyridazinyl, pyrazinyl, thiazolyl, thienyl, thienopyrimidinyl, thienopyridinyl, purinyl, 1',2',3',6'-tetrahydropyridinyl, triazinyl, oxazolyl, pyrazolyl, or furanyl; wherein if Ring A contains an -NH- moiety that nitrogen may be optionally substituted by a group selected from G;
Ring B is thienyl, thiadiazolyl, thiazolyl, pyrimidyl, pyrazinyl, pyridazinyl or pyridyl;
R¹ is a substituent on carbon and is selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, aryl, aryloxy, arylC₁₋₆alkyl, heterocyclic group, (heterocyclic group)C₁₋₆alkyl or a group (D-E-); wherein R¹, including group (D-E-), may be optionally substituted on carbon by one or more V; and wherein, if said heterocyclic group contains an -NH- moiety that nitrogen may be optionally substituted by a group selected from J;
V is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl or a group (D'-E'-); wherein V, including group (D'-E'-), may be optionally substituted on carbon by one or more W;
W and Z are independently selected from halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl or *N,N*-(C₁₋₆alkyl)₂sulphamoyl;
G, J and K are independently selected from C₁₋₈alkyl, C₂₋₈alkenyl, C₂₋₈alkynyl, C₁₋₈alkanoyl, C₁₋₈alkylsulphonyl, C₁₋₈alkoxycarbonyl, carbamoyl, *N*-(C₁₋₈alkyl)carbamoyl, *N*,*N*-(C₁₋₈alkyl)carbamoyl, benzyloxycarbonyl, benzoyl and phenylsulphonyl, aryl, arylC₁₋₆alkyl or (heterocyclic group)C₁₋₆alkyl; wherein G, J and K may be optionally substituted on carbon by one or more Q; and wherein if said heterocyclic group contains an -NH- moiety that nitrogen may be optionally substituted by a group selected from hydrogen or C₁₋₆alkyl;
Q is halo, nitro, cyano, hydroxy, oxo, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, C₁₋₆alkoxycarbonylamino, *N*-(C₁₋₆alkyl)sulphamoyl, *N,N*-(C₁₋₆alkyl)₂sulphamoyl, aryl, aryloxy, arylC₁₋₆alkyl, arylC₁₋₆alkoxy, heterocyclic group, (heterocyclic group)C₁₋₆alkyl, (heterocyclic group)C₁₋₆alkoxy, or a group (D"-E"-); wherein Q, including group (D"-E"-), may be optionally substituted on carbon by one or more Z;
D, D' and D" are independently selected from C₁₋₆alkyl, C₂₋₆alkenyl, C₂₋₆alkynyl, C₃₋₈cycloalkyl, C₃₋₈cycloalkylC₁₋₆alkyl, aryl, arylC₁₋₆alkyl, heterocyclic group, (heterocyclic group)C₁₋₆alkyl; wherein D, D' and D" may be optionally substituted on carbon by one or more F'; and wherein if said heterocyclic group contains an -NH- moiety that nitrogen may be optionally substituted by a group selected from K;
E, E' and E" are independently selected from -N(R^{a})-, -O-, -C(O)O-, -OC(O)-, -C(O)-, -N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})-, -N(R^{a})SO₂-; wherein R^{a} and R^{b} are independently selected from hydrogen or C₁₋₆alkyl optionally substituted by one or more F and r is 0-2;
F and F' are independently selected from halo, nitro, cyano, hydroxy, trifluoromethyl, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl and *N,N*-(C₁₋₆alkyl)₂sulphamoyl;
m is 0, 1, 2, 3 or 4; wherein the values of R¹ may be the same or different;
R² is fluoro or chloro;
n is 0, 1 or 2, wherein the values of R² may be the same or different;
R³ is amino or hydroxy;
R⁴ is halo, nitro, cyano, hydroxy, trifluoromethyl, trifluoromethoxy, amino, carboxy or carbamoyl; and
p is 0, 1 or 2, wherein the values of R⁴ may be the same or different;
and wherein:
an aryl group is a group selected from phenyl, indenyl, indanyl, naphthyl, tetrahydronaphthyl and fluorenyl,
and a heterocyclic group is a saturated, partially saturated or unsaturated, mono or bicyclic ring containing 3-12 atoms of which at least onc atom is chosen from nitrogen, sulphur or oxygen, and which may, unless otherwise specified, be carbon or nitrogen linked, and wherein a CH₂ group can optionally be replaced by a C(O), and wherein a ring sulphur atom may he optionally oxidised to form the S-oxide(s);
or a pharmaceutically acceptable salt or *in vivo* hydrolysable ester or amide thereof.

2. A compound of the formula **(I)** according to claim 1 wherein:
Ring A is pyzidin-4-yl, pyridin-3-yl, pyridin-2-yl, morpholin-4-yl, piperidin-4-yl, pipcridin-3-yl, piperdin-2-yl, piperazin-4-yl, thiazol-2-yl, thien-2-yl, furan-3-yl, pyrrolidin-1-yl, piperidin-1-yl, triazol-1-yl or 1',2',3',6'-tetrahydropyridin-4-yl wherein if Ring A contains an -NH- moiety that nitrogen may be optionally substituted by a group selected from G; or a pharmaceutically acceptable salt or in *vivo* hydrolysable ester or amide thereof.

3. A compound of the formula **(I)** according to claim 1 wherein:
R¹ is a substituent on carbon and is selected from cyano, hydroxy, C₁₋₆alkyl or a group (D-E-); wherein R¹, including group (D-E-), may be optionally substituted on carbon by one or more V;
V is cyano, hydroxy or a group (D'-E'-); wherein V, including group (D'-E'-), may be optionally substituted on carbon by one or more W;
W and Z are independently selected from cyano, C₁₋₆alkyl or C₁₋₆alkoxy;
G and K are independently selected from C₁₋₈alkyl, C₂₋₈alkenyl, C₂₋₈alkynyl, arylC₁₋₆alkyl or (heterocyclic group)C₁₋₆alkyl; wherein G and K may be optionally substituted on carbon by one or more Q;
Q is cyano, hydroxy, oxo, C₁₋₆alkyl, C₂₋₆alkenyl, C₁₋₆alkoxy, C₁₋₆alkanoyl, C₁₋₆alkanoyloxy, *N*-(C₁₋₆alkyl)carbamoyl, *N,N*-(C₁₋₆alkyl)₂carbamoyl, C₁₋₆alkoxycarbonyl, C₁₋₆alkoxycarbonylamino, aryl, aryloxy or a group (D"-E"-); wherein Q, including group (D"-E"-), may be optionally substituted on carbon by one or more Z;
D, D' and D" are independently selected from aryl, arylC₁₋₆alkyl or heterocyclic group; wherein D, D' and D" may be optionally substituted on carbon by one or more F'; and wherein if said heterocyclic group contains an -NH- moiety that nitrogen may be optionally substituted by a group selected from K;
E, E' and E" are independently selected from -O-, -C(O)O-, -OC(O)-, -C(O)-, -N(R^{a})C(O)-, -C(O)N(R^{a})-, -S(O)ᵣ-; wherein R^{a} is selected from hydrogen or C₁₋₆alkyl optionally substituted by one or more F and r is 0-2; and
F and F' are independently selected from nitro, hydroxy, C₁₋₆alkyl, C₁₋₆alkoxy, C₁₋₆alkanoyl, *N*-(C₁₋₆alkyl)amino, *N,N*-(C₁₋₆alkyl)₂amino, C₁₋₆alkanoylamino or C₁₋₆alkoxycarbonyl; or a pharmaceutically acceptable salt or *in vivo* hydrolysable ester or amide thereof.

4. A compound of the formula **(I)** according to claim 1 wherein m is 1.

5. A compound of the formula **(I)** according to claim 1 wherein R² is fluoro and n is 0 or 1.

6. A compound of the formula **(I)** according to claim 1 wherein R³ is amino.

7. A compound of the formula **(I)** according to claim 1 wherein p is 0.

8. A compound of the formula **(I)** according to claim 1 wherein:
Ring A is pyridin-4-yl, pyridin-3-yl, pyridin-2-yl, morpholin-4-yl, piperidin-4-yl, piperidin-3-yl, piperdin-2-yl, piperazin-4-yl, thiazol-2-yl, thien-2-yl, furan-3-yl, pyrrolidin-1-yl, piperidin-1-yl, triazol-1-yl or 1',2',3',6'-tetrahydropyridin-4-yl wherein if Ring A contains an -NH- moiety that nitrogen may be optionally substituted by a group selected from G;
Ring B is thienyl, thiazolyl, pyrimidyl, pyrazinyl, pyridazinyl or pyridyl;
R' is a substituent on carbon and is selected from cyano, hydroxy, C₁₋₆alkyl or a group (D-E-); wherein R¹, including group (D-E-), may be optionally substituted on carbon by one or more V;
V is cyano, hydroxy or a group (D'-E'-); wherein V, including group (D'-E'-), may be optionally substituted on carbon by one or more W;
W and Z are independently selected from cyano, C₁₋₆alkyl or C₁₋₆alkoxy;
G and K are independently selected from C₁₋₈alkyl, C₂₋₈alkenyl, C₂₋₈alkynyl, arylC₁₋₆alkyl or (heterocyclic group)C₁₋₆alkyl; wherein G and K may be optionally substituted on carbon by one or more Q;
Q is cyano, hydroxy, oxo, C₁₋₆alkyl, C₂₋₆alkenyl, C₁₋₆alkoxy, C₁₋₆alkanoyl, C₁₋₆alkanoyloxy, *N*-(C₁₋₆alkyl)carbamoyl, *N,N*-(C₁₋₆alkyl)₂carbamoyl, C₁₋₆alkoxycarbonyl, C₁₋₆alkoxycarbonylamino, aryl, aryloxy or a group (D"-E"-); wherein Q, including group (D"-E"-), may be optionally substituted on carbon by one or more Z;
D, D' and D" are independently selected from aryl, arylC₁₋₆alkyl or heterocyclic group; wherein D, D' and D'' may be optionally substituted on carbon by one or more F'; and wherein if said heterocyclic group contains an -NH- moiety that nitrogen may be optionally substituted by a group selected from K;
E, E' and E" are independently selected from -O-, -C(O)O-, -OC(O)-, -C(O)-, -N(R^{a})C(O)-, -C(O)N(R^{a})-, -S(O)ᵣ-; wherein R^{a} is selected from hydrogen or C₁₋₆alkyl optionally substituted by one or more F and r is 0-2;
F and F' are independently selected from nitro, hydroxy, C₁₋₆alkyl, C₁₋₆alkoxy, C₁₋₆alkanoyl, *N*-(C₁₋₆alkyl)amino, *N,N*-(C₁₋₆alkyl)₂amino, C₁₋₆alkanoylamino or C ₁₋₆alkoxycarbonyl;
m is 0, 1, or 2; wherein the values of R¹ may be the same or different;
R² is fluoro;
n is 0 or 1;
R³ is amino;
R⁴ is halo; and
p is 0, 1 or 2, wherein the values of R⁴ may be the same or different;
or a pharmaceutically acceptable salt or *in vivo* hydrolysable ester or amide thereof.

9. A process for preparing a compound of formula **(I)** or a pharmaceutically acceptable salt or an *in vivo* hydrolysable ester thereof, according to claim 1, which process comprises of:
(a) The reaction of a compound of the formula **(II)** 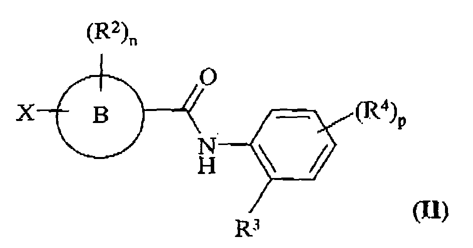 wherein X is a reactive group, with a compound of the formula **(III)** 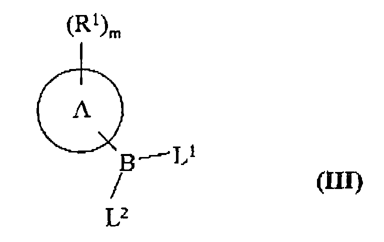 wherein L¹ and L² are ligands;
(b) The reaction of a compound of the formula **(IV)** 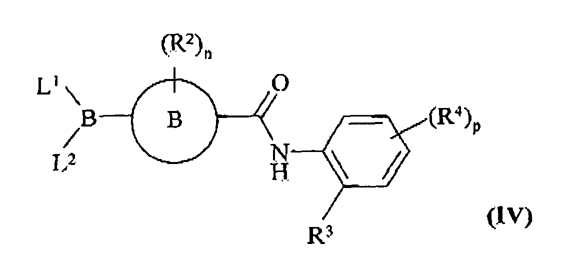 wherein L¹ and L² are ligands, with a compound of the formula **(V)** 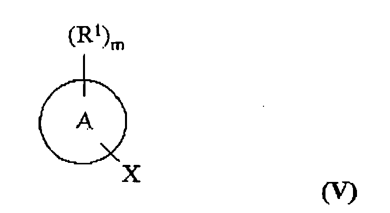 wherein X is a reactive group; or
(c) The reaction, in the presence of 4-(4,6-dimethoxy-1,3,5-triazinyl-2-yl)-4-methylmorpholinium chloride, of a compound of the formula **(VI)** 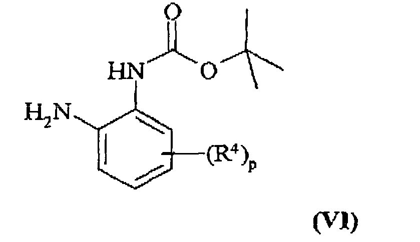 with a compound of the formula **(VII)** 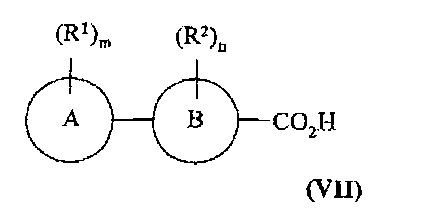
and thereafter if necessary:
i) converting a compound of the formula **(I)** into another compound of the formula **(I);** and/or
ii) removing any protecting groups.

10. A pharmaceutical composition which comprises a compound of the formula (I), or a pharmaceutically acceptable salt or *in vivo* hydrolysable ester or amide thereof, according to claims 1 to 8 in association with a pharmaceutically-acceptable diluent or carrier.

11. A compound of the formula **(I),** or a pharmaceutically acceptable salt or *in vivo* hydrolysable ester or amide thereof, according to claims 1 to 8 for use as a medicament.

12. The use of a compound of the formula **(1),** or a pharmaceutically acceptable salt or *in vivo* hydrolysable ester or amide thereof, according to claims 1 to 8 in the manufacture of a medicament for use in the production of a HDAC inhibitory effect in a warm-blooded animal such as man.

13. The use of a compound of the formula **(I)**, or a pharmaceutically acceptable salt or *in vivo* hydrolysable ester or amide thereof, according to claims 1 to 8 in the manufacture of a medicament for use in the treatment of cancer.

14. A compound of the formula **(I),** or a pharmaceutically acceptable salt or in *vivo* hydrolysable ester or amide thereof, according to claims 1 to 8 for use in the treatment of cancer.

## Patentansprüche

1. Verbindungen der Formel (I): 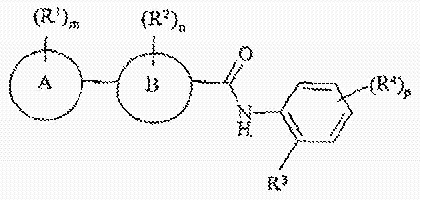 worin:
Ring A für Pyridyl, Chinolyl, Indolyl, Pyrimidinyl, Morpholinyl, Piperidinyl, Piperazinyl, Pyridazinyl, Pyrazinyl, Thiazolyl, Thienyl, Thienopyrimidinyl, Thienopyridinyl, Purinyl, 1',2',3',6'-Tetrahydropyridinyl, Triazinyl, Oxazolyl, Pyrazolyl oder Furanyl steht; wobei, wenn Ring A eine -NH-Einheit enthält, der Stickstoff gegebenenfalls durch eine Gruppe ausgewählt aus G substituiert sein kann;
Ring B für Thienyl, Thiadiazolyl, Thiazolyl, Pyrimidyl, Pyrazinyl, Pyridazinyl oder Pyridyl steht;
R¹ für einen Substituenten an Kohlenstoff steht und ausgewählt ist aus Halogen, Nitro, Cyano, Hydroxy, Trifluormethyl, Trifluormethoxy, Amino, Carboxy, Carbamoyl, Mercapto, Sulfamoyl, C₁₋₆-Alkyl, C₂₋₆-Alkenyl C₂₋₆-Alkynyl, C₁₋₆-Alkoxy, C₁₋₆-Alkanoyl, C₁₋₆-Alkanoyloxy, *N*-(C₁₋₆-Alkyl) amino, *N, N*-(C₁₋₆-Alkyl)₂-amino, C₁₋₆-Alkanoylamino, *N*-(C₁₋₆-Alkyl) carbamoyl, *N, N*-(C₁₋₆-Alkyl)₂carbamoyl, C₁₋₆-Alkyl-S(O)ₐ, wobei a für 0 bis 2 steht, C₁₋₆-Alkoxycarbonyl, *N*-(C₁₋₆-Alkyl) sulfamoyl, *N,N*-(C₁₋₆Alkyl)₂sulfamoyl, Aryl, Aryloxy, Aryl-C₁₋₆-Alkyl, einer heterozyklischen Gruppe, (heterozyklische Gruppe)-C₁₋₆-Alkyl oder einer Gruppe (D-E-), wobei R¹ einschließlich der Gruppe (D-E-) gegebenenfalls an Kohlenstoff durch ein oder mehrere V substituiert sein kann; und wobei, wenn die heterozyklische Gruppe eine -NH-Einheit enthält, der Stickstoff gegebenenfalls durch eine Gruppe ausgewählt aus J substituiert sein kann;
wobei V für Halogen, Nitro, Cyano, Hydroxy, Oxo, Trifluormethyl, Trifluormethoxy, Amino, Carboxy, Carbamoyl, Mercapto, Sulfamoyl, C₁₋₆-Alkyl, C₂₋₆-Alkenyl, C₂₋₆-Alkynyl, C₁₋₆-Alkoxy, C₁₋₆-Alkanoyl, C₁₋₆-Alkanoyloxy, *N-*(C₁₋₆-Alkyl) amino, *N, N-*(C₁₋₆-Alkyl)₂-amino, C₁₋₆-Alkanoylamino, *N-* (C₁₋₆-Alkyl)-carbamoyl, *N,N-*(C₁₋₆-Alkyl)₂-carbamoyl, C₁₋₆-AlkylS(O)ₐ, wobei a für 0 bis 2 steht, C₁₋₆-Alcoxycarbonyl; *N-*(C₁₋₆-Alkyl) sulfamoyl, N, *N-*(C₁₋₆-Alkyl)₂ sulfamoyl oder eine Gruppe (D'-E'-) steht, wobei V einschließlich der Gruppe (D'-E'-) gegebenenfalls an Kohlenstoff durch ein oder mehrere W substituiert sein kann;
W und Z unabhängig voneinander ausgewählt sind aus Halogen, Nitro, Cyano, Hydroxy, Oxo, Trifluormethyl, Trifluormethoxy, Amino, Carboxy, Carbamoyl, Mercapto, Sulfamoyl, C₁₋₆-Alkyl, C₂₋₆-Alkenyl, C₂₋₆-Alkynyl, C₁₋₆-Alkoxy, C₁₋₆-Alkanoyl, C₁₋₆-Alkanoyloxy, *N*-(C₁₋₆-Alkyl) amino, *N, N*-(C₁₋₆-Alkyl)₂-amino, C₁₋₆-Alkanoylamino, *N*-(C₁₋₆-Alkyl) carbamoyl, *N,N*-(C₁₋₆-Alkyl)₂-carbamoyl, C₁₋₆Alkyl-S(O)ₐ, wobei a für 0 bis 2 steht, C₁₋₆Alcoxycarbonyl, N-(C₁₋₆Alkyl)sulfamoyl, oder *N*,*N*-(C₁₋₆Alkyl)₂-sulfamoyl;
G, J und K unabhängig voneinander ausgewählt sind aus C₁₋₈-Alkyl, C₂₋₈-Alkenyl, C₂₋₈-Alkynyl, C₁₋₈-Alkanoyl, C₁₋₈-Alkylsulfonyl, C₁₋₈-Alkoxycarbonyl, Carbamoyl, *N*-(C₁₋₈-Alkyl)carbamoyl, *N,N-* (C₁₋₈-Alkyl)carbamoyl, Benzyloxycarbonyl, Benzoyl und Phenylsulfonyl, Aryl, Aryl-C₁₋₆-Alkyl oder (heterozyklische Gruppe)-C₁₋₆Alkyl; wobei G, J und K gegebenenfalls an Kohlenstoff durch ein oder mehrere Q substituiert sein können; und wobei, wenn die heterozyklische Gruppe eine -NH-Einheit enthält, der Stickstoff gegebenenfalls durch eine Gruppe ausgewählt aus Wasserstoff oder C₁₋₆-Alkyl substituiert sein kann;
Q für Halogen, Nitro, Cyano, Hydroxy, Oxo, Trifluormethyl, Trifluormethoxy, Amino, Carboxy, Carbamoyl, Mercapto, Sulfamoyl, C₁₋₆-Alkyl, C₂₋₆-Alkenyl, C₂₋₆-Alkynyl, C₁₋₆-Alkoxy, C₁₋₆-Alkanoyl, C₁₋₆-Alkanoyloxy, *N-*(C₁₋₆-Alkyl) amino, *N*, *N-*(C₁₋₆-Alkyl)₂-amino, C₁₋₆-Alkanoylamino, *N*-(C₁₋₆-Alkyl) carbamoyl, *N,N-*(C₁₋₆-Alkyl)₂carbamoyl, C₁₋₆Alkyl-S(O)ₐ, wobei a für 0 bis 2 steht, C₁₋₆Alkoxycarbonyl, C₁₋₆-Alkoxycarbonylamino, *N-*(C₁₋₆-Alkyl) sulfamoyl, *N*, *N-*(C₁₋₆-Alkyl)₂sulfamoyl, Aryl, Aryloxy, Aryl-C₁₋₆-Alkyl, Aryl-C₁₋₆-Alkoxy, eine heterozyklische Gruppe, (heterozyklische Gruppe-)-C₁₋₆-Alkyl, (heterozyklische Gruppe-)-C₁₋₆Alkoxy, oder eine Gruppe (D"-E"-) steht; wobei Q einschließlich der Gruppe (D"-E"-) gegebenenfalls an Kohlenstoff durch ein oder mehrere Z substituiert sein kann;
D, D' und D" unabhängig voneinander ausgewählt sind aus C₁₋₆-Alkyl, C₂₋₆-Alkenyl, C₂₋₆-Alkynyl, C₃₋₈-Zykloalkyl, C₃₋₈-Zykloalkyl-C₁₋₆-Alkyl, Aryl, Aryl-C₁₋₆Alkyl, einer heterozyklischen Gruppe, (heterozyklische Gruppe-)-C₁₋₆-Alkyl; wobei D, D' und D''gegebenenfalls an Kohlenstoff durch ein oder mehrere F' substituiert sein können; und wobei, wenn die heterozyklische Gruppe eine -NH-Einheit enthält, der Stickstoff gegebenenfalls durch eine Gruppe ausgewählt aus K substituiert sein kann;
E, E' und E" unabhängig voneinander ausgewählt sind aus -N(R^{a})-, -O-, -C(O)O-, -OC(O)-, -C(O)-, -N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})-, -N(R^{a})SO₂-; wobei R^{a} und R^{b} unabhängig voneinander ausgewählt sind aus Wasserstoff oder C₁₋₆-Alkyl, gegebenenfalls substituiert durch ein oder mehrere F, und r für 0-2 steht;
F und F' unabhängig voneinander ausgewählt sind aus Halogen, Nitro, Cyano, Hydroxy, Trifluormethyl, Trifluormethoxy, Amino, Carboxy, Carbamoyl, Mercapto, Sulfamoyl, C₁₋₆-Alkyl, C₂₋₆-Alkenyl, C₂₋₆-Alkynyl, C₁₋₆-Alkoxy, C₁₋₆-Alkanoyl, C₁₋₆-Alkanoyloxy, *N*-(C₁₋₆Alkyl)amino, *N, N*-(C₁₋₆-Alkyl)₂-amino, C₁₋₆-Alkanoylamino, *N*-(C₁₋₆-Alkyl) carbamoyl, *N, N*-(C₁₋₆-Alkyl)₂-carbamoyl, C₁₋₆-Alkyl-S(O)ₐ, wobei a für 0 bis 2 steht, C₁₋₆Alcoxycarbonyl, N-(C₁₋₆Alkyl)sulfamoyl und *N, N*-(C₁₋₆₋Alkyl)₂-sulfamoyl;
m für 0, 1, 2, 3 oder 4 steht; wobei die Werte von R¹ gleich oder verschieden sein können;
R² für Fluor oder Chlor steht;
n für 0, 1 oder 2 steht, wobei die Werte von R² gleich oder verschieden sein können;
R³ für Amino oder Hydroxy steht;
R⁴ für Halogen, Nitro, Cyano, Hydroxy, Trifluormethyl, Trifluormethoxy, Amino, Carboxy oder Carbamoyl steht; und
p für 0,1 oder 2 steht, wobei die Werte von R⁴ gleich oder verschieden sein können; und wobei:
es sich bei einer Arylgruppe um eine Gruppe ausgewählt aus Phenyl, Indenyl, Indanyl, Naphthyl, Tetrahydronaphthyl und Fluorenyl handelt,
und es sich bei einer heterozyklischen Gruppe um einen gesättigten, teilweise gesättigten oder ungesättigten, mono- oder bizyklischen Ring handelt, der 3-12 Atome enthält, von denen wenigstens ein Atom aus Stickstoff, Schwefel oder Sauerstoff ausgewählt ist und der, wenn nicht anders angegeben, über Kohlenstoff oder Stickstoff gebunden sein kann, und worin CH₂-Gruppen gegebenenfalls durch C(O) ersetzt sein können, und worin Ring-Schwefelatome gegebenenfalls zum S-Oxid oxidiert sein können;
oder deren pharmazeutisch annehmbare Salze oder in vivo hydrolisierbare Ester oder Amide.

2. Verbindungen der Formel (I) nach Anspruch 1, worin:
Ring A für Pyridin-4-yl, Pyridin-3-yl, Pyridin-2-yl, Morpholin-4-yl, Piperidin-4-yl, Piperidin-3-yl, Piperidin-2-yl, Piperazin-4-yl, Thiazol-2-yl, Thien-2-yl, Furan-3-yl, Pyrrolidin-1-yl, Piperidin-1-yl, Triazol-1-yl oder 1',2',3',6'-Tetrahydropyridin-4-yl steht, wobei, wenn Ring A eine -NH-Einheit enthält, der Stickstoff gegebenenfalls durch eine Gruppe ausgewählt aus G substituiert sein kann; oder deren pharmazeutisch annehmbare Salze oder in vivo hydrolisierbare Ester oder Amide.

3. Verbindungen der Formel (I) nach Anspruch 1, worin:
R¹ für einen Substituenten an Kohlenstoff steht und ausgewählt ist aus Cyano, Hydroxy, C₁₋₆-Alkyl oder einer Gruppe (D-E-); wobei R¹ einschließlich der Gruppe (D-E-) gegebenenfalls an Kohlenstoff durch ein oder mehrere V substituiert sein kann;
V für Cyano, Hydroxy oder eine Gruppe (D'-E'-) steht; wobei V einschließlich der Gruppe (D'-E'-) gegebenenfalls an Kohlenstoff durch ein oder mehrere W substituiert sein kann;
W und Z unabhängig voneinander ausgewählt sind aus Cyano, C₁₋₆-Alkyl oder C₁₋₆-Alkoxy;
G und K unabhängig voneinander ausgewählt sind aus C₁₋₈-Alkyl, C₂₋₈-Alkenyl, C₂₋₈-Alkynyl, Aryl-C₁₋₆-alkyl oder (heterozyklische Gruppe)- C₁₋₆-Alkyl; wobei G und K gegebenenfalls an Kohlenstoff durch ein oder mehrere Q substituiert sein können;
Q für Cyano, Hydroxy, Oxo, C₁₋₆-Alkyl, C₂₋₆-Alkenyl, C₁₋₆-Alkoxy, C₁₋₆-Alkanoyl, C₁₋₆-Alkanoyloxy, *N*-(C₁₋₆-Alkyl) carbamoyl, *N*,*N-*(C₁₋₆Alkyl)₂carbamoyl, , C₁₋₆-Alkoxycarbonyl, C₁₋₆-Alkoxycarbonylamino, Aryl, Aryloxy oder eine Gruppe (D"-E"-) steht; wobei Q einschließlich der Gruppe (D"-E"-) gegebenenfalls an Kohlenstoff durch ein oder mehrere Z substituiert sein kann;
D, D' und D" unabhängig voneinander ausgewählt sind aus Aryl, Aryl-C₁₋₆-Alkyl oder einer heterozyklischen Gruppe, wobei D, D' und D" gegebenenfalls an Kohlenstoff durch ein oder mehrere F' substituiert sein können; und wobei, wenn die heterozyklische Gruppe eine -NH-Einheit enthält, der Stickstoff gegebenenfalls durch eine Gruppe ausgewählt aus K substituiert sein kann;
E, E' und E" unabhängig voneinander ausgewählt sind aus -O-, -C(O)O-, -OC(O)-, -C(O)-, -N(R^{a})C(O)-, -C(O)N(R^{a})-, -S(O)ᵣ- ; wobei R^{a} ausgewählt ist aus Wasserstoff oder C₁₋₆-Alkyl, gegebenenfalls substituiert durch ein oder mehrere F, und r für 0-2 steht; und
F und F' unabhängig voneinander ausgewählt sind aus Nitro, Hydroxy, C₁₋₆-Alkyl, C₁₋₆-Alkoxy, C₁₋₆-Alkanoyl, *N-*(C₁₋₆-Alkyl) amino, *N,N-*(C₁₋₆-Alkyl)₂amino, C₁₋₆-Alkanoylamino oder C₁₋₆-Alkoxycarbonyl; oder deren pharmazeutisch annehmbare Salze oder in vivo hydrolisierbare Ester oder Amide.

4. Verbindungen der Formel (I) nach Anspruch 1, worin m für 1 steht.

5. Verbindungen der Formel (I) nach Anspruch 1, worin R² für Fluor steht und n für 0 oder 1 steht.

6. Verbindungen der Formel (I) nach Anspruch 1, worin R³ für Amino steht.

7. Verbindungen der Formel (I) nach Anspruch 1, worin p für 0 steht.

8. Verbindungen der Formel (I) nach Anspruch 1, worin :
Ring A für Pyridin-4-yl, Pyridin-3-yl, Pyridin-2-yl, Morpholin-4-yl, Piperidin-4-yl, Piperidin-3-yl, Piperidin-2-yl, Piperazin-4-yl, Thiazol-2-yl, Thien-2-yl, Furan-3-yl, Pyrrolidin-1-yl, Piperidin-1-yl, Triazol-1-yl oder 1',2',3',6'-Tetrahydropyridin-4-yl steht, wobei, wenn Ring A eine -NH-Einheit enthält, der Stickstoff gegebenenfalls durch eine Gruppe ausgewählt aus G substituiert sein kann ;
Ring B für Thienyl, Thiazolyl, Pyrimidyl, Pyrazinyl, Pyridazinyl oder Pyridyl steht;
R¹ für einen Substituenten an Kohlenstoff steht und ausgewählt ist aus Cyano, Hydroxy, C₁₋₆-Alkyl oder einer Gruppe (D-E-); wobei R¹ einschließlich der Gruppe (D-E-) gegebenenfalls an Kohlenstoff durch ein oder mehrere V substituiert sein kann ;
V für Cyano, Hydroxy oder eine Gruppe (D'-E'-) steht; wobei V einschließlich der Gruppe (D'-E'-) gegebenenfalls an Kohlenstoff durch ein oder mehrere W substituiert sein kann ;
W und Z unabhängig voneinander ausgewählt sind aus Cyano, C₁₋₆-Alkyl oder C₁₋₆-Alkoxy;
G und K unabhängig voneinander ausgewählt sind aus C₁₋₈-Alkyl, C₂₋₈-Alkenyl, C₂₋₈-Alkynyl, Aryl-C₁₋₆-alkyl oder (heterozyklische Gruppe)- C₁₋₆-Alkyl; wobei G und K gegebenenfalls an Kohlenstoff durch ein oder mehrere Q substituiert sein können;
Q für Cyano, Hydroxy, Oxo, C₁₋₆-Alkyl, C₂₋₆-Alkenyl, C₁₋₆-Alkoxy, C₁₋₆-Alkanoyl, C₁₋₆-Alkanoyloxy, *N*-(C₁₋₆-Alkyl) carbamoyl, *N,N*-(C₁₋₆Alkyl)₂carbamoyl, , C₁₋₆-Alkoxycarbonyl, C₁₋₆- Alkoxycarbonylamino, Aryl, Aryloxy oder eine Gruppe (D"-E"-) steht; wobei Q einschließlich der Gruppe (D"-E"-) gegebenenfalls an Kohlenstoff durch ein oder mehrere Z substituiert sein kann;
D, D' und D" unabhängig voneinander ausgewählt sind aus Aryl, Aryl-C₁₋₆-Alkyl oder einer heterozyklischen Gruppe, wobei D, D' und D" gegebenenfalls an Kohlenstoff durch ein oder mehrere F' substituiert sein können ; und wobei, wenn die heterozyklische Gruppe eine -NH-Einheit enthält, der Stickstoff gegebenenfalls durch eine Gruppe ausgewählt aus K substituiert sein kann;
E, E' und E" unabhängig voneinander ausgewählt sind aus -O-, -C(O)O-, -OC(O)-, -C(O)-, -N(R^{a})C(O)-, -C(O)N(R^{a})-, -S(O)ᵣ- ; wobei R^{a} ausgewählt ist aus Wasserstoff oder C₁₋₆-Alkyl, gegebenenfalls substituiert durch ein oder mehrere F, und r für 0-2 steht ; und
F und F' unabhängig voneinander ausgewählt sind aus Nitro, Hydroxy, C₁₋₆-Alkyl, C₁₋₆-Alkoxy, C₁₋₆-Alkanoyl, *N-*(C₁₋₆-Alkyl) amino, *N,N-*(C₁₋₆-Alkyl)₂amino, C₁₋₆-Alkanoylamino oder C₁₋₆-Alkoxycarbonyl; und
m für 0, 1 oder 2 steht ; wobei die Werte von R¹ gleich oder verschieden sein können;
R² für Fluor steht;
n für 0 oder 1 steht;
R³ für Amino steht;
R⁴ für Halogen steht; und
p für 0, 1 oder 2 steht, wobei die Werte von R⁴ gleich oder verschieden sein können;
oder deren pharmazeutisch annehmbare Salze oder in vivo hydrolisierbare Ester oder Amide davon.

9. Verfahrensherstellung einer Verbindung der Formel (I) eines pharmazeutisch annehmbaren Salzes oder eines in vivo hydrolisierbaren Esters davon nach Anspruch 1, bei dem man:
(a) eine Verbindung der Formel (II) 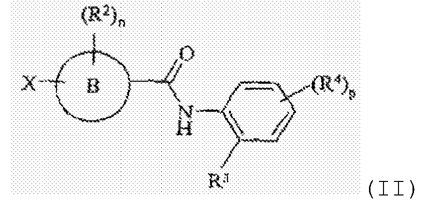 worin X für eine reaktive Gruppe steht, mit einer Verbindung der Formel (III) 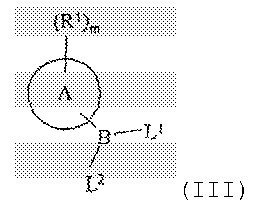 worin L¹ und L² für Liganden stehen, umsetzt ;
(b) eine Verbindung der Formel (IV) 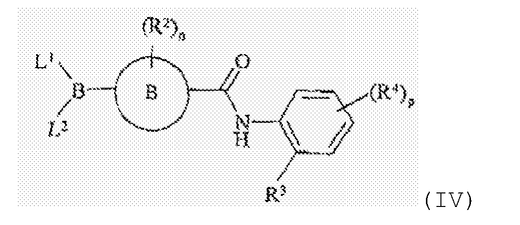 worin L¹ und L² für Liganden stehen, mit einer Verbindung der Formel (V) 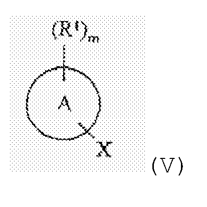 worin X für eine reaktive Gruppe steht, umsetzt; oder
(c) in Gegenwart von 4-(4,6-Dimethoxy-1,3,5-triazin-2-yl)-4-methylmorpholiniumchlorid eine Verbindung der Formel (VI) 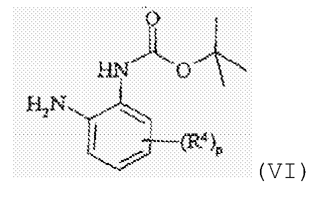 mit einer Verbindung der Formel (VII) 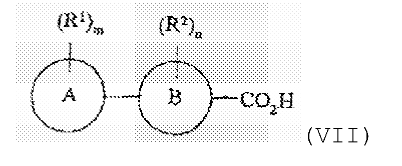
umsetzt und anschließend, falls erforderlich :
i) eine Verbindung der Formel (I) in eine andere Verbindung der Formel (I) umwandelt; und/oder
ii) gegebenenfalls vorhandene Schutzgruppen entfernt.

10. Pharmazeutische Zusammensetzung, enthaltend eine Verbindung nach Formel (I) oder ein pharmazeutisch annehmbares Salz oder einen in vivo hydrolisierbaren Ester oder ein in vivo hydrolisierbares Amid davon nach einem der Ansprüche 1 bis 8 zusammen mit einem pharmazeutisch annehmbaren Verdünnungsmittel oder Träger.

11. Verbindungen der Formel (I) nach Anspruch 1, oder deren pharmazeutisch annehmbare Salze oder in vivo hydrolisierbare Ester oder Amide davon nach einem der Ansprüche 1 bis 8 zur Verwendung als Medikament.

12. Verwendung einer Verbindung der Formel (I) oder eines pharmazeutisch annehmbaren Salzes oder eines in vivo hydrolisierbaren Esters oder Amids davon nach einem der Ansprüche 1 bis 8 bei der Herstellung eines Medikaments zur Verwendung beim Hervorrufen einer HDAC-hemmenden Wirkung in einem Warmblüter wie dem Menschen.

13. Verwendung einer Verbindung der Formel (I) oder eines pharmazeutisch annehmbaren Salzes oder eines in vivo hydrolisierbaren Esters oder Amids davon nach einem der Ansprüche 1 bis 8 bei der Herstellung eines Medikaments zur Verwendung bei der Behandlung von Krebs.

14. Verbindungen der Formel (I) und deren pharmazeutisch annehmbare Salze und in vivo hydrolisierbare Ester und Amide nach einem der Ansprüche 1 bis 8 zur Verwendung bei der Behandlung von Krebs.

## Revendications

1. Composé de formule (I) : 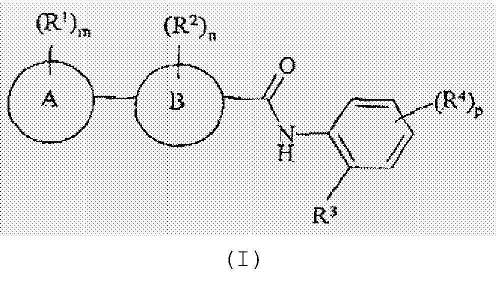 dans lequel :
le cycle A est un pyridyle, un quinolyle, un indolyle, un pyrimidinyle, un morpholinyle, un pipéridinyle, un pipérazinyle, un pyridazinyle, un pyrazinyle, un thiazolyle, un thiényle, un thiénopyrimidinyle, un thiénopyridinyle, un purinyle, un 1',2',3',6'-tétrahydropyridinyle, un triazinyle, un oxazolyle, un pyrazolyle ou un furanyle ; dans lequel si le cycle A contient un fragment -NH- cet azote peut être facultativement substitué par un groupe choisi parmi G ;
le cycle B est un thiényle, un thiadiazolyle, un thiazolyle, un pyrimidyle, un pyrazinyle, un pyridazinyle ou un pyridyle ;
R¹ est un substituant sur un carbone et est choisi parmi un halogène, un nitro, un cyano, un hydroxy, un trifluorométhyle, un trifluorométhoxy, un amino, un carboxy, un carbamoyle, un mercapto, un sulfamoyle, un alkyle en C₁₋₆, un alcényle en C₂₋₆, un alcynyle en C₂₋₆, un alcoxy en C₁₋₆, un alcanoyle en C₁₋₆, un alcanoyloxy en C₁₋₆, un *N*-(alkyle en C₁₋₆)amino, un *N,N*-(alkyle en C₁₋₆)₂amino, un alcanoylamino en C₁₋₆, un *N*-(alkyle en C₁₋₆)carbamoyle, un *N,N*-(alkyle en C₁₋₆)₂carbamoyle, un (alkyle en C₁₋₆)S(O)ₐ où a est 0 à 2, un (alcoxy en C₁₋₆)carbonyle, un *N*-(alkyle en C₁₋₆)sulfamoyle, un *N,N-*(alkyle en C₁₋₆)₂sulfamoyle, un aryle, un aryloxy, un aryl(alkyle en C₁₋₆), un groupe hétérocyclique, un (groupe hétérocyclique) (alkyle en C₁₋₆) ou un groupe (D-E-) ; dans lequel R¹, comprenant un groupe (D-E-), peut être facultativement substitué sur un carbone par un ou plusieurs V ; et dans lequel, si ledit groupe hétérocyclique contient un fragment -NH- cet azote peut être facultativement substitué par un groupe choisi parmi J ;
V est un halogène, un nitro, un cyano, un hydroxy, un oxo, un trifluorométhyle, un trifluorométhoxy, un amino, un carboxy, un carbamoyle, un mercapto, un sulfamoyle, un alkyle en C₁₋₆, un alcényle en C₂₋₆, un alcynyle en C₂₋₆, un alcoxy en C₁₋₆, un alcanoyle en C₁₋₆, un alcanoyloxy en C₁₋₆, un *N*-(alkyle en C₁₋₆)amino, un *N,N*-(alkyle en C₁₋₆)₂amino, un alcanoylamino en C₁₋₆, un *N*-(alkyle en C₁₋₆)carbamoyle, un *N,N*-(alkyle en C₁₋₆)acarbamoyle, un (alkyle en C₁₋₆)S(O)ₐ où a est 0 à 2, un (alcoxy en C₁₋₆)carbonyle, un *N*- (alkyle en C₁₋₆)sulfamoyle, un *N,N*-(alkyle en C₁₋₆)₂sulfamoyle ou un groupe (D'-E'-) ; dans lequel V, comprenant le groupe (D'-E'-), peut être facultativement substitué sur un carbone par un ou plusieurs W ;
W et Z sont indépendamment choisis parmi un halogène, un nitro, un cyano, un hydroxy, un oxo, un trifluorométhyle, un trifluorométhoxy, un amino, un carboxy, un carbamoyle, un mercapto, un sulfamoyle, un alkyle en C₁₋₆, un alcényle en C₂₋₆, un alcynyle en C₂₋₆, un alcoxy en C₁₋₆, un alcanoyle en C₁₋₆, un alcanoyloxy en C₁₋₆, un *N*-(alkyle en C₁₋₆)amino, un *N,N*-(alkyle en C₁₋₆)amino, un alcanoylamino en C₁₋₆, un *N*-(alkyle en C₁₋₆)carbamoyle, un *N*,*N*-(alkyle en C₁₋₆)₂carbamoyle, un (alkyle en C₁₋₆)S(O)ₐ où a est 0 à 2, un (alcoxy en C₁₋₆)carbonyle, un *N*-(alkyle en C₁₋₆)sulfamoyle ou un *N*,*N*-(alkyle en C₁₋₆)₂sulfamoyle ;
G, J et K sont indépendamment choisis parmi un alkyle en C₁₋₈, un alcényle en C₂₋₈, un alcynyle en C₂₋₈, un alcanoyle en C₁₋₈, un (alkyle en C₁₋₈)sulfonyle, un (alcoxy en C₁₋₈)carbonyle, un carbamoyle, un N-(alkyle en C₁₋₈)carbamoyle, un *N*,*N*-(alkyle en C₁₋₈)carbamoyle, un benzyloxycarbonyle, un benzoyle et un phénylsulfonyle, un aryle, un aryl (alkyle en C₁₋₆) ou un (groupe hétérocyclique) (alkyle en C₁₋₆) ; dans lequel G, J et K peuvent être facultativement substitués sur un carbone par un ou plusieurs Q ; et dans lequel si ledit groupe hétérocyclique contient un fragment -NH- cet azote peut être facultativement substitué par un groupe choisi parmi un hydrogène ou un alkyle en C₁₋₆ ;
Q est un halogène, un nitro, un cyano, un hydroxy, un oxo, un trifluorométhyle, un trifluorométhoxy, un amino, un carboxy, un carbamoyle, un mercapto, un sulfamoyle, un alkyle en C₁₋₆, un alcényle en C₂₋₆, un alcynyle en C₂₋₆, un alcoxy en C₁₋₆, un alcanoyle en C₁₋₆, un alcanoyloxy en C₁₋₆, un *N*-(alkyle en C₁₋₆)amino, un *N,N*-(alkyle en C₁₋₆)₂amino, un alcanoylamino en C₁₋₆, un *N*-(alkyle en C₁₋₆)carbamoyle, un *N,N*-(alkyle en C₁₋₆)₂carbamoyle, un (alkyle en C₁₋₆)S(O)ₐ où a est 0 à 2, un (alcoxy en C₁₋₆)carbonyle, un (alcoxy en C₁₋₆)carbonylamino, un *N*-(alkyle en C₁₋₆)sulfamoyle, un *N,N*-(alkyle en C₁₋₆)₂sulfamoyle, un aryle, un aryloxy, un aryl (alkyle en C₁₋₆), un aryl (alcoxy en C₁₋₆), un groupe hétérocyclique, un (groupe hétérocyclique)(alkyle en C₁₋₆), un (groupe hétérocyclique) (alcoxy en C₁₋₆), ou un groupe (D"-E"-) ; dans lequel Q comprenant le groupe (D"-E"-), peut être facultativement substitué sur un carbone par un ou plusieurs Z ;
D, D' et D" sont indépendamment choisis parmi un alkyle en C₁₋₆, un alcényle en C₂₋₆, un alcynyle en C₂₋₆, un cycloalkyle en C₃₋₈, un (cycloalkyle en C₃₋₈) (alkyle en C₁₋₆), un aryle, un aryl (alkyle en C₁₋₆), un groupe hétérocyclique, un (groupe hétérocyclique)(alkyle en C₁₋₆) ; dans lequel D, D' et D" peuvent être facultativement substitués sur un carbone par un ou plusieurs F' ; et dans lequel si ledit groupe hétérocyclique contient un fragment -NH- cet azote peut être facultativement substitué par un groupe choisi parmi K ;
E, E' et E" sont indépendamment choisis parmi -N(R^{a})-, -O-, -C(O)O-, -OC(O)-, -C(O)-, -N(R^{a})C(O)-, -N(R^{a})C(O)N(R^{b})-, -N(R^{a})C(O)O-, -OC(O)N(R^{a})-, -C(O)N(R^{a})-, -S(O)ᵣ-, -SO₂N(R^{a})_, -N(R^{a})SO₂-; dans lequel R^{a} et R^{b} sont indépendamment choisis parmi un hydrogène ou un alkyle en C₁₋₆ facultativement substitué par un ou plusieurs F et r est 0 à 2 ;
F et F' sont indépendamment choisis parmi un halogène, un nitro, un cyano, un hydroxy, un trifluorométhyle, un trifluorométhoxy, un amino, un carboxy, un carbamoyle, un mercapto, un sulfamoyle, un alkyle en C₁₋₆, un alcényle en C₂₋₆, un alcynyle en C₂₋₆, un alcoxy en C₁₋₆, un alcanoyle en C₁₋₆, un alcanoyloxy en C₁₋₆, un *N-*(alkyle en C₁₋₆)amino, un *N,N*-(alkyle en C₁₋₆)₂amino, un alcanoylamino en C₁₋₆, un *N*-(alkyle en C₁₋₆)carbamoyle, un *N,N*-(alkyle en C₁₋₆)₂carbamoyle, un (alkyle en C₁₋₆)S(O)ₐ où a est 0 à 2, un (alcoxy en C₁₋₆)carbonyle, un *N*-(alkyle en C₁₋₆)sulfamoyle et un *N,N*-(alkyle en C₁₋₆)₂sulfamoyle ;
m est 0, 1, 2, 3 or 4 ; dans lequel les valeurs de R¹ peuvent être identiques ou différentes ;
R² est un fluor ou un chlore ;
n est 0, 1 ou 2, dans lequel les valeurs de R² peuvent être identiques ou différentes ;
R³ est un amino ou un hydroxy ;
R⁴ est un halogène, un nitro, un cyano, un hydroxy, un trifluorométhyle, un trifluorométhoxy, un amino, un carboxy ou un carbamoyle ; et
p est 0, 1 ou 2, dans lequel les valeurs de R⁴ peuvent être identiques ou différentes ;
et dans lequel :
un groupe aryle est un groupe choisi parmi le phényle, l'indényle, l'indanyle, le naphtyle, le tétrahydronaphtyle et le fluorényle,
et un groupe hétérocyclique est un cycle mono ou bicyclique, saturé, partiellement saturé ou insaturé contenant de 3 à 12 atomes dont au moins un atome est choisi parmi l'azote, le soufre ou l'oxygène, et qui peut, sauf indication contraire, être lié à un carbone ou un azote, et dans lequel un groupe CH₂ peut facultativement être remplacé par un C(O), et dans lequel un atome de soufre de cycle peut être facultativement oxydé pour former les S-oxyde(s) ;
ou un sel pharmaceutiquement acceptable ou un ester ou amide hydrolysable *in vivo* de celui-ci.

2. Composé de formule (I) selon la revendication 1 dans lequel :
le cycle A est un pyridin-4-yle, un pyridin-3-yle, un pyridin-2-yle, un morpholin-4-yle, un pipéridin-4-yle, un pipéridin-3-yle, un pipéridin-2-yle, un pipérazin-4-yle, un thiazol-2-yle, un thién-2-yle, un furan-3-yle, un pyrrolidin-1-yle, un pipéridin-1-yle, un triazol-1-yle ou un 1',2',3',6'-tetrahydropyridin-4-yle dans lequel si le cycle A contient un fragment -NH- cet azote peut être facultativement substitué par un groupe choisi parmi G ; ou un sel pharmaceutiquement acceptable ou un ester ou amide hydrolysable *in vivo* de celui-ci.

3. Composé de formule (I) selon la revendication 1, dans lequel :
R¹ est un substituant sur un carbone et est choisi parmi un cyano, un hydroxy, un alkyle en C₁₋₆ ou un groupe (D-E-) ; dans lequel R¹, comprenant (D-E-), peut être facultativement substitué sur un carbone par un ou plusieurs V ;
V est un cyano, un hydroxy ou un groupe (D'-E'-) ; dans lequel V, comprenant le groupe (D'-E'-), peut être facultativement substitué sur un carbone par un ou plusieurs W ;
W et Z sont indépendamment choisis parmi un cyano, un alkyle en C₁₋₆ ou un alcoxy en C₁₋₆ ;
G et K sont indépendamment choisis parmi un alkyle en C₁₋₈, un alcényle en C₂₋₈, un alcynyle en C₂₋₈, un aryl (alkyle en C₁₋₆) ou un (groupe hétérocyclique) (alkyle en C₁₋₆) ; dans lequel G et K peuvent être facultativement substitués sur un carbone par un ou plusieurs Q ;
Q est un cyano, un hydroxy, un oxo, un alkyle en C₁₋₆, un alcényle en C₂₋₆, un alcoxy en C₁₋₆, un alcanoyle en C₁₋₆, un alcanoyloxy en C₁₋₆, un *N*-(alkyle en C₁₋₆)carbamoyle, un *N,N*-(alkyle en C₁₋₆)₂carbamoyle, un (alcoxy en C₁₋₆)carbonyle, un (alcoxy en C₁₋₆)carbonylamino, un aryle, un aryloxy ou un groupe (D"-E"-) ; dans lequel Q comprenant le groupe (D"-E"-), peut être facultativement substitué sur un carbone par un ou plusieurs Z ;
D, D' et D" sont indépendamment choisis parmi un aryle, un aryl(alkyle en C₁₋₆) ou un groupe hétérocyclique ; dans lequel D, D' et D" peuvent être facultativement substitués sur un carbone par un ou plusieurs F' ; et dans lequel si ledit groupe hétérocyclique contient un fragment -NH- cet azote peut être facultativement substitué par un groupe choisi parmi K ;
E, E' et E" sont indépendamment choisis parmi -O-, -C(O)O-, -OC(O)-, -C(O)-, -N(R^{a})C(O)-, -C(O)N(R^{a})-, -S(O)ᵣ- ; dans lequel R^{a} est choisi parmi un hydrogène ou un alkyle en C₁₋₆ facultativement substitué par un ou plusieurs F et r est 0 à 2 ; et
F et F' sont indépendamment choisis parmi un nitro, un hydroxy, un alkyle en C₁₋₆, un alcoxy en C₁₋₆, un alcanoyle en C₁₋₆, un *N*-(alkyle en C₁₋₆)amino, un *N*,*N-*(alkyle en C₁₋₆)₂amino, un alcanoylamino en C₁₋₆ ou un (alcoxy en C₁₋₆)carbonyle ; ou un sel pharmaceutiquement acceptable ou un ester ou amide hydrolysable *in vivo* de celui-ci.

4. Composé de formule (I) selon la revendication 1 dans lequel m est 1.

5. Composé de formule (I) selon la revendication 1 dans lequel R² est un fluor et n est 0 ou 1;

6. Composé de formule (I) selon la revendication 1 dans lequel R³ est un amino.

7. Composé de formule (I) selon la revendication 1 dans lequel p est 0.

8. Composé de formule (I) selon la revendication 1 dans lequel :
le cycle A est un pyridin-4-yle, un pyridin-3-yle, un pyridin-2-yle, un morpholin-4-yle, un pipéridin-4-yle, un pipéridin-3-yle, un pipéridin-2-yle, un pipérazin-4-yle, un thiazol-2-yle, un thién-2-yle, un furan-3-yle, un pyrrolidin-1-yle, un pipéridin-1-yle, un triazol-1-yle ou un 1',2',3',6'-tetrahydropyridin-4-yle dans lequel si le cycle A contient un fragment -NH- cet azote peut être facultativement substitué par un groupe choisi parmi G ;
le cycle B est un thiényle, un thiazolyle, un pyrimidyle, un pyrazinyle, un pyridazinyle ou un pyridyle ;
R¹ est un substituant sur un carbone et est choisi parmi un cyano, un hydroxy, un alkyle en C₁₋₆ ou un groupe (D-E-) ; dans lequel R¹, comprenant (D-E-), peut être facultativement substitué sur un carbone par un ou plusieurs V ;
V est un cyano, un hydroxy ou un groupe (D'-E'-) ; dans lequel V, comprenant le groupe (D'-E'-), peut être facultativement substitué sur un carbone par un ou plusieurs W ;
W et Z sont indépendamment choisis parmi un cyano, un alkyle en C₁₋₆ ou un alcoxy en C₁₋₆ ;
G et K sont indépendamment choisis parmi un alkyle en C₁₋₈, un alcényle en C₂₋₈, un alcynyle en C₂₋₈, un aryl (alkyle en C₁₋₆) ou un (groupe hétérocyclique) (alkyle en C₁₋₆) ; dans lequel G et K peuvent être facultativement substitués sur un carbone par un ou plusieurs Q ;
Q est un cyano, un hydroxy, un oxo, un alkyle en C₁₋₆, un alcényle en C₂₋₆, un alcoxy en C₁₋₆, un alcanoyle en C₁₋₆, un alcanoyloxy en C₁₋₆, un *N*-(alkyle en C₁₋₆)carbamoyle, un *N*,*N*-(alkyle en C₁₋₆)₂carbamoyle, un (alcoxy en C₁₋₆)carbonyle, un (alcoxy en C₁₋₆)carbonylamino, un aryle, un aryloxy ou un groupe (D"-E"-) ; dans lequel Q comprenant le groupe (D"-E"-), peut être facultativement substitué sur un carbone par un ou plusieurs Z ;
D, D' et D" sont indépendamment choisis parmi un aryle, un aryl(alkyle en C₁₋₆) ou un groupe hétérocyclique ; dans lequel D, D' et D" peuvent être facultativement substitués sur un carbone par un ou plusieurs F' ; et dans lequel si ledit groupe hétérocyclique contient un fragment -NH- cet azote peut être facultativement substitué par un groupe choisi parmi K ;
E, E' et E" sont indépendamment choisis parmi -O-, -C(O)O-, -OC(O)-, -C(O)-, -N(R^{a})C(O)-, -C(O)N(R^{a})-, -S(O)ᵣ- ; dans lequel R^{a} est choisi parmi un hydrogène ou un alkyle en C₁₋₆ facultativement substitué par un ou plusieurs F et r est 0 à 2 ;
F et F' sont indépendamment choisis parmi un nitro, un hydroxy, un alkyle en C₁₋₆, un alcoxy en C₁₋₆, un alcanoyle en C₁₋₆, un *N*-(alkyle en C₁₋₆)amino, un *N*,*N-*(alkyle en C₁₋₆)₂amino, un alcanoylamino en C₁₋₆ ou un (alcoxy en C₁₋₆)carbonyle ;
m est 0, 1, ou 2 ; dans lequel les valeurs de R¹ peuvent être identiques ou différentes ;
R² est un fluor ;
n est 0 ou 1 ;
R³ est un amino ;
R⁴ est un halogène ; et
p est 0, 1 ou 2, dans lequel les valeurs de R⁴ peuvent être identiques ou différentes ;
ou un sel pharmaceutiquement acceptable ou un ester ou amide hydrolysable *in vivo* de celui-ci.

9. Procédé pour préparer un composé de formule (I) ou un sel pharmaceutiquement acceptable ou un ester ou amide hydrolysable *in vivo* de celui-ci, selon la revendication 1, ledit procédé comprenant les étapes de :
(a) réaction d'un composé de formule (II) 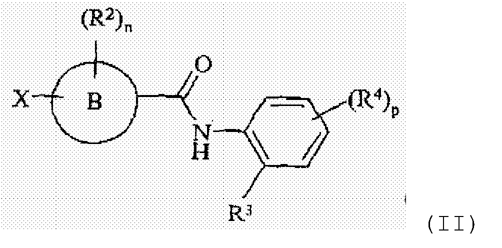 dans lequel X est un groupe réactif, avec un composé de formule (III) 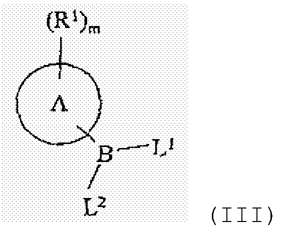 dans lequel L¹ et L² sont des ligands ;
(b) réaction d'un composé de formule (IV) 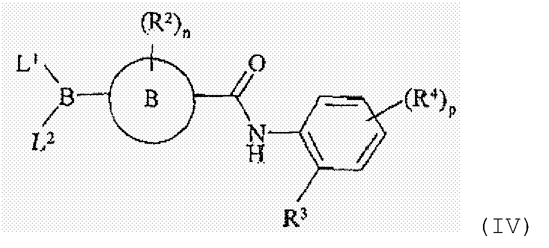 dans lequel L¹ et L² sont des ligands, avec un composé de formule (V) 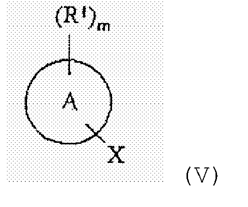 dans lequel X est un groupe réactif ; ou
(c) réaction, en présence de chlorure de 4-(4,6-diméthoxy-1,3,5-triazinyl-2-yl)-4-méthylmorpholinium, d'un composé de formule (VI) 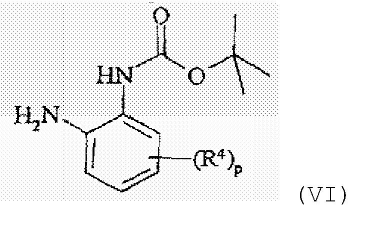 avec un composé de formule (VII) 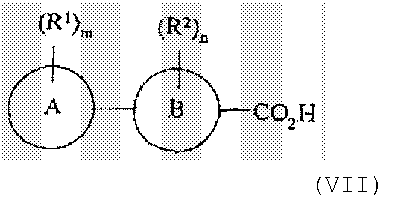
et ensuite si nécessaire :
i) convertir un composé de formule (I) en un autre composé de formule (I) ; et/ou
ii) l'élimination de groupes protecteurs éventuels.

10. Composition pharmaceutique qui comprend un composé de formule (I), ou un sel pharmaceutiquement acceptable ou un ester ou amide hydrolysable *in vivo* de celui-ci, selon les revendications 1 à 8 en association avec un diluant ou véhicule pharmaceutiquement acceptable.

11. Composé de formule (I), ou un sel pharmaceutiquement acceptable ou un ester ou amide hydrolysable *in vivo* de celui-ci, selon les revendications 1 à 8 pour utilisation en tant que médicament.

12. Composé de formule (I), ou un sel pharmaceutiquement acceptable ou un ester ou amide hydrolysable *in vivo* de celui-ci, selon les revendications 1 à 8 dans la fabrication d'un médicament pour utilisation dans la production d'un effet inhibiteur de HDAC dans un animal à sang chaud tel qu'un humain.

13. Composé de formule (I), ou un sel pharmaceutiquement acceptable ou un ester ou amide hydrolysable *in vivo* de celui-ci, selon les revendications 1 à 8 dans la fabrication d'un médicament pour utilisation dans le traitement du cancer.

14. Composé de formule (I), ou un sel pharmaceutiquement acceptable ou un ester ou amide hydrolysable *in vivo* de celui-ci, selon les revendications 1 à 8 pour utilisation dans le traitement du cancer.
